# EUROPEAN PATENT SPECIFICATION

(11) **EP 2 521 777 B1**
(45) Date of publication and mention of the grant of the patent: **28.12.2016**
(21) Application number: 11731735.4
(22) Date of filing: 05.01.2011
(51) Int. Cl.: C12N 15/00

(54) **COMPOSITIONS AND METHODS FOR TREATING GLIOBLASTOMA GBM**
ZUSAMMENSETZUNGEN UND VERFAHREN ZUR BEHANDLUNG VON GLIOBLASTOMEN (GBM)
COMPOSITIONS ET PROCÉDÉS POUR TRAITER LE GLIOBLASTOME GBM

(30) Priority: 05.01.2010 US 282228 P; 07.01.2010 US 282248 P
(43) Date of publication of application: 14.11.2012
(73) Proprietor: Vascular Biogenics Ltd., 60376 Or Yehuda (IL)
(72) Inventor: COHEN, Yael, 55602 Kiryat-Ono (IL); BANGIO, Livnat, 49776 Petach-Tikva (IL); BRENNER, Andrew J., Boerne Texas 78015 (US); BREITBART, Eyal, 73127 Hashmonaim (IL)
(74) Representative: Vossius & Partner Patentanwälte Rechtsanwälte mbB
(86) International application number: PCT/IL2011/000009
(87) International publication number: WO 2011/083466

(56) References cited:
- WO-A1-00/61150
- WO-A2-2007/096882
- US-A1- 2003 195 338
- US-A1- 2007 286 845
- US-A1- 2007 286 845
- US-B1- 6 204 055
- GREENBERGER S ET AL: "Transcription-control led gene therapy against tumor angiogenesis", JOURNAL OF CLINICAL INVESTIGATION, AMERICAN SOCIETY FOR CLINICAL INVESTIGATION, US, vol. 113, no. 7, 1 April 2004 (2004-04-01) , pages 1017-1024, XP002349281, ISSN: 0021-9738, DOI: 10.1172/JCI200420007
- CHI ET AL.: 'Angiogenesis as a therapeutic target in malignant gliomas.' ONCOLOGIST. vol. 14, no. 6, June 2009, pages 621 - 636, XP008163702
- BRENNER ET AL.: 'Antivascular activity of VB111 in glioblastoma xenografts.' J. CLIN. ONCOL. vol. 28, no. 15, May 2010, XP008163739

## Description

### FIELD AND BACKGROUND OF THE INVENTION

The present invention, in some embodiments thereof, relates to a viral vector for use in treating malignant gliomas and, more particularly, but not exclusively, for treating Glioblastoma multiforme (GBM).

Malignant gliomas, the most common adult-onset neurological neoplasms, encompass a family of primary central nervous system tumors including glioblastoma, astrocytoma, oligodendroglioma, and ependymoma, along with the juvenile onset neoplasms such as juvenile pilocystic astrocytoma.

Malignant gliomas are typically characterized by over-expression of growth factors/tumor associated antigens believed to significantly contribute to the unchecked growth of such tumors. Various malignant gliomas, such as glioblastomas, exhibit epidermal growth factor receptor (EGFR) overexpression leading to increased aggressiveness and poor prognosis. Malignant gliomas may also display over-expression of platelet-derived growth factor receptor, a phenomenon which has also been correlated with increased malignancy and poor prognosis.

Malignant gliomas, the most common type of primary brain tumors, are aggressive, highly invasive, and neurologically destructive tumors which are among the deadliest of all human cancers. Of the estimated 17,000 new brain tumors diagnosed each year in the United States, about half are malignant gliomas. Malignant glioma cells produce very invasive brain tumors with infiltration of both white and gray matter. At the time of diagnosis, microscopic extension through much of the neural axis by malignant glioma is the rule. Such extension by motile invading cells underlies the incurability by surgery of most gliomas, even when they appear small and restricted in nature.

Glioblastoma multiforme (GBM), the most serious form of malignant glioma, are extremely aggressive brain tumors which generally arise in the upper brain (cerebrum), but which may also occur elsewhere in the central nervous system, such as in the spinal cord, cerebellum, brain stem, or optic chiasm. Low-grade gliomas, which include astrocytomas, oligodendrogliomas, and pilocytic astrocytomas, account for 25 % of all primary brain tumors, and over time most of these low-grade tumors dedifferentiate into more malignant gliomas. Diffuse astrocytomas are predominantly located in the cerebral hemispheres of adults and have an inherent tendency to progress to anaplastic astrocytoma and (secondary) glioblastoma. The majority of glioblastomas develop de novo (primary glioblastomas), without an identifiable less-malignant precursor lesion.

Despite optimal therapy with surgery, radiotherapy, and temozolomide chemotherapy, the median survival of patients with glioblastomas is only 12-15 months. When these tumors recur, conventional salvage therapies produce minimal benefit, with only 8-15 % of patients alive and free from progression at 6 months (6M-PFS).

Neovascularization is a major feature of glioblastomas (Maher et al., 2001, Genes Dev. 15:1311-1333). Angiogenesis activators are extremely important in tumor growth, as reflected by the fact that neovascularization must occur for solid tumors to grow beyond a diameter of 2-3 mm (Goldbrunner et al., 2000, J. Neurooncol. 50:53-62). One of the molecules that regulates this process is the vascular endothelial growth factor (VEGF). VEGF mRNA is overexpressed in the highly vascularized glioblastoma multiform (Maher et al., 2001, Genes Dev. 15:1311-1333). It has been demonstrated that the transfection of antisense-VEGF-complementary-DNA as well VEGF antisense RNA encoding vectors result in down-regulation of the endogenous VEGF and inhibits growth of gliomas in mice (Sasaki et al., 1999, Int. J. Dev. Neurosci. 17:579-591; Zheng et al., 2000, Acta Pharmacol. Sin. 21:211-214). A similar effect was observed upon the local delivery of the angiogenesis inhibitor endostatin (Read et al., 2001, Nat. Biotechnol. 19:29-34). However, this strategy has a cytostatic effect. It is effective in inhibiting tumor growth but not in actually eliminating them.

Bevacizumab (Avastin®) is a humanized monoclonal antibody that binds VEGF, preventing it from activating its receptors, especially VEGFR2, abrogating subsequent biologic effects. This drug has shown benefit in colorectal, non-small cell lung, and breast cancers, and is approved by the Food and Drug Administration for these indications. Several studies have now evaluated the combination of bevacizumab and the chemotherapeutic agent irinotecan in recurrent malignant gliomas and the results have been more encouraging.

In one phase II study, the combination of bevacizumab and irinotecan produced a response rate of 67 % and 6M-PFS of 56 % in recurrent anaplastic gliomas, and a response rate of 57 % and a 6M-PFS of 46 % in recurrent glioblastomas.

These preliminary findings have been recently confirmed by a large multi-center randomized phase II study of 167 patients with recurrent GBM who were treated with bevacizumab alone or in combination with irinotecan [Cloughesy T, Prados M, Wen P, et al. Society for Neuro-Oncology 12th Annual Meeting, 2007].

Patients receiving bevacizumab alone had a response rate of 20 % and a 6M-PFS of 35.1 %, while patients receiving the combination of bevacizumab in combination with irinotecan had a response rate of 34 % and 6M-PFS of 51 %.The median survival was 9.7 months for bevacizumab (Avastin) alone, and 8.7 months for the combination. In addition, treatment with bevacizumab was also associated with a significant reduction in peritumoral edema and the need for corticosteroids. As a result of these studies, the combination of bevacizumab with irinotecan is increasingly used for the treatment of patients with recurrent malignant gliomas.

Another agent proposed for the treatment of malignant gliomas is Aflibercept (VEGF-Trap). This is a soluble hybrid receptor, composed of portions of VEGFR-1 and VEGFR-2 fused to an immunoglobulin G1 Fc domain. Like bevacizumab, it is designed to deplete circulating VEGF, but has significantly greater affinity for VEGF than bevacizumab itself.

In addition, inhibitors of VEGF receptors have been proposed for the treatment of malignant gliomas. In a phase II trial study of a potent pan-VEGFR inhibitor, cediranib (AZD2171; Recentin) in patients with recurrent glioblastomas, response rates in excess of 50 % were observed and the 6M-PFS was increased to approximately 25 %. Studies with other inhibitors of VEGFR such as sorafenib (Nexavar), sunitinib (Sutent), vandetanib (ZD6474; Zactima), pazopanib (GW786034), and vatalanib (PTK787) in glioblastomas are also in progress.

In comparison with drugs targeting VEGF or VEGFR, agents inhibiting other angiogenic pathways have produced less success. Drugs that inhibit PDGF receptors such as imatinib mesylate (Gleevec) were ineffective, due partly to its poor penetration across the blood-brain barrier. Cilengitide, a drug that inhibits αvβ3 and αvβ5 integrins has shown modest activity in glioblastomas and studies combining it with other agents are in progress.

The use of viral vectors as gene delivery agents has been proposed for the treatment of malignant gliomas. Such viruses may be engineered to produce anticancer activity by expressing transgenes whose products exert a tumoricidal effect.

Several of such approaches have shown anti-tumor efficiency in experimental studies, and the first clinical trials for the treatment of malignant glioma were conducted in the 1990s. HSV-tk gene therapy has been the pioneering and most commonly used approach, but oncolytic conditionally replicating adenoviruses and herpes simplex virus mutant vectors, p53, interleukins, interferons, and antisense oligonucleotides have also been used.

United States Patent 5,747,340 teaches use of a murine endothelial cell-specific promoter which shows selectivity towards angiogenic cells.

International Application WO/2008/132729 teaches viral vectors comprising endothelial cell specific promoters which directs expression of a transgene in angiogenic cells for the treatment of cancer.

### SUMMARY OF THE INVENTION

The present invention provides the following:
1. A therapeutically effective amount of a viral vector for use in the treatment of a malignant glioma in a subject in need thereof, comprising a nucleic acid construct which comprises:
   (i) a first polynucleotide sequence encoding a Fas-chimera (Fas-c), and
   (ii) a second polynucleotide sequence encoding an endothelial cell-specific promoter, which is operably linked to the first polynucleotide sequence.
2. The viral vector for use of item 1, wherein the malignant glioma is selected from the group consisting of glioblastoma, astrocytoma, oligodendroglioma, ependymoma, and juvenile pilocystic astrocytoma.
3. The viral vector for use of item 1 or 2, wherein the therapeutically effective amount is about 10³ to about 10¹⁶ virus particles, about 10⁵ to about 10¹³ virus particles, about 10⁷ to about 10¹² virus particles, about 1x0¹² to about 5 x10¹² virus particles, or about 1x10¹³ to about 5 x10¹³ virus particles.
4. The viral vector for use of any one of items 1 to 3, wherein the virus vector is administered in at least two doses or at least three doses.

The viral vector for use of any one of items 1 to 4, wherein the virus vector is administered via intravenous administration or local administration.

The viral vector for use of any one of items 1 to 5, wherein said viral vector is a non-replicating adenoviral vector.

The viral vector for use of any one of items 1 to 6, wherein the Fas-c comprises an extracellular domain of TNFR1 and a trans-membrane region and an intracellular region of Fas.

The viral vector for use of any one of items 1 to 7, wherein the Fas-c comprises (i) SEQ ID NO: 2 and SEQ ID NO: 3 or (ii) SEQ ID NO: 4.

The viral vector for use of any one of items 1 to 8, wherein the promoter comprises the sequence set forth in SEQ ID NO: 8 or the complementary sequence thereof.

The viral vector for use of item 9, wherein (i) the promoter further comprises at least one copy of the sequence set forth in SEQ ID NO: 6 or the complementary sequence thereof or (ii) the promoter comprises the sequence set forth in SEQ ID NO: 7 or a complementary sequence thereof.

The viral vector for use of any one of items 1 to 10, wherein said promoter comprises a hypoxia response element (HRE) comprising the sequence set forth in SEQ ID NO: 5.

The viral vector for use of any one of items 1 to 11, wherein the promoter comprises SEQ ID NO: 12.

The viral vector for use of any one of items 1 to 12, wherein the viral vector consists of the sequence set forth in SEQ ID NO: 9 or SEQ ID NO: 10.

The viral vector for use of any one of items 1 to 13, wherein the malignant glioma is glioblastoma.

The viral vector for use of any one of items 1 to 3, wherein the virus vector is administered as a single unit dose.

The viral vector for use of any one of items 1 to 15, wherein the virus vector is adenovirus serotype 5.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings and images. With specific reference now to the drawings and images in detail, it is stressed that the particular shown are by way of example and for purposes of illustrative discussion of embodiments of the invention. In this regard, the description taken with the drawings makes apparent to those skilled in the art how embodiments of the invention may be practiced.

In the drawings:
FIG. 1 is a time chart illustrating an exemplary treatment schedule to measure the in-vivo effects of VB-111 in nude rats pre-inoculated with U87 tumor cells.
FIG. 2 is a graph illustrating the effect of VB-111 on survival of rats pre-inoculated with U87 tumor cells.
FIG. 3 is a graph illustrating the effect of VB-111 on tumor size (as measured by luciferase activity) of rats pre-inoculated with U87 tumor cells.
FIG. 4 is a graph illustrating the effect of VB-111 on tumor size (as measured by MRI) of rats pre-inoculated with U87 tumor cells.
FIGs. 5A-F are photographs of brain slices illustrating the effect of VB-111 on tumor size (as measured by MRI) of rats pre-inoculated with U87 tumor cells.

### DESCRIPTION OF SPECIFIC EMBODIMENTS OF THE INVENTION

The present invention, in some embodiments thereof, relates to a viral vector for use in treating malignant gliomas and, more particularly, but not exclusively, for treating Glioblastoma multiforme (GBM).

Malignant gliomas, the most common subtype of primary brain tumors, are aggressive, highly invasive, and neurologically destructive tumors. These tumors are considered to be among the deadliest of all human cancers. In its most aggressive form, glioblastoma (GBM), media survival ranges from 9 to 12 months. Despite several decades of technological advances in neurosurgery and radiation therapy there has been no significant change in the overall statistics.

Gene therapy approaches for the treatment of malignant gliomas have been attempted. However, while these approaches have proved successful in vitro and in animal models, these strategies have met with limited success in clinical trials. It is believed that the low in vivo infection efficiency of the vectors is connected to the histological structure of the glioblastomas. These are very solid tumors, which are almost completely impermeable to diffusion of big particles such as viruses.

The present inventors surprisingly found that treatment of glioblastomas in animals could be carried out effectively using viral vectors encoding a toxic molecule (Fas-chimera (Fas-c)) under the control of a promoter which directs transcription in endothelial cells.

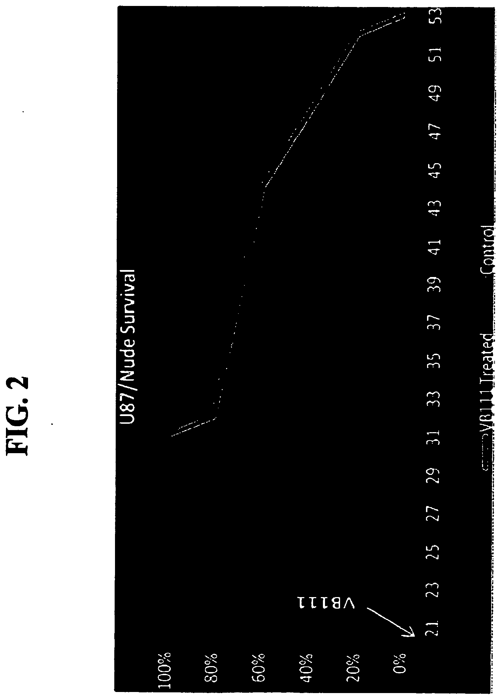

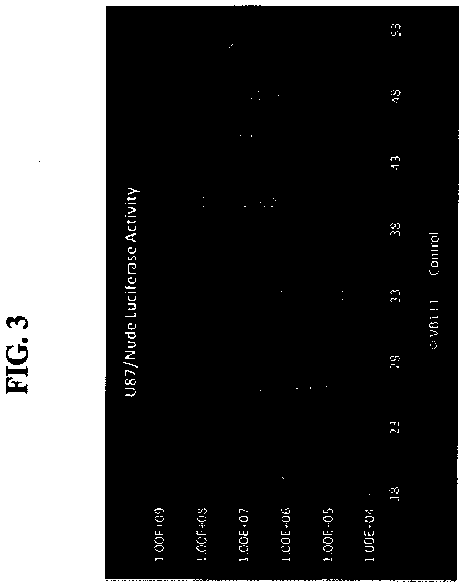

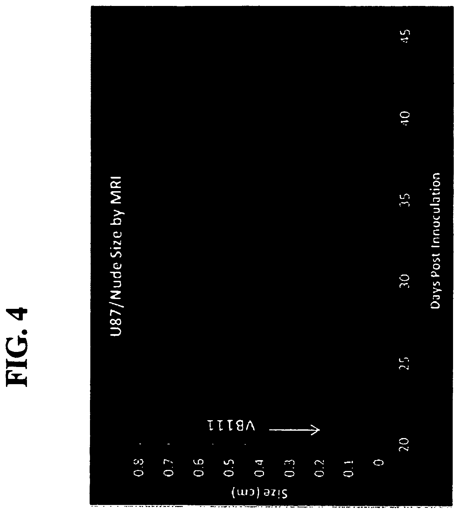

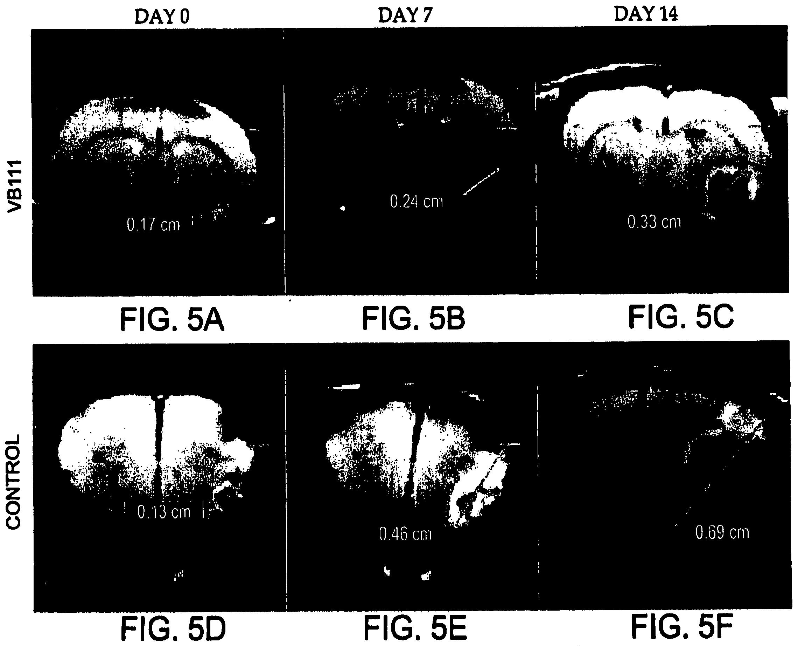

Using an animal model of glioblastoma whereby rats were pre-inoculated with U87 tumor cells, the present inventors showed that administration of such viral vectors decreased the size of the luciferase-tagged tumors as measured by luciferase activity (Figure 3) and MRI (Figures 4 and 5). The present inventors further showed that administration of such viral vectors increased survival of the rats (Figure 2).

Thus, according to one aspect of the present invention, there is provided a therapeutically effective amount of a viral vector comprising:
(i) a first polynucleotide sequence encoding a Fas-chimera (Fas-c); and
(ii) a second polynucleotide sequence encoding an endothelial cell-specific promoter for use in treating a malignant glioma.

As used herein, the phrase "malignant glioma" refers to a primary central nervous system tumor typically characterized by over-expression of growth factor receptors and/or tumor associated antigens.
The malignant glioma may exhibit epidermal growth factor receptor (EGFR) overexpression. The malignant glioma may exhibit platelet-derived growth factor receptor (PDFR) overexpression. The malignant glioma may exhibit both platelet-derived growth factor receptor (PDFR) overexpression and epidermal growth factor receptor (EGFR) overexpression.

Examples of malignant gliomas include, but are not limited to glioblastoma, astrocytoma, oligodendroglioma, ependymoma, and juvenile onset neoplasms such as juvenile pilocystic astrocytoma.
Subjects to be treated may include mammals - e.g. humans. The subject may have received a prior treatment for the malignant glioma
(e.g. radiotherapy and/or chemotherapy) and the malignant glioma may have relapsed. The subject may not have received a prior treatment for the malignant glioma.

The phrase "viral vector" refers to a replication competent or replication-deficient viral particle which are capable of transferring nucleic acid molecules into a host.
Replication Defective Vectors and Replication Defective Vector-Producing Packaging Cells may be used. Examples of such vectors are
adenoviral vectors, AAV vectors and retroviral vectors and others described in Shir et al, Cellular and Molecular Neurobiology, Vol. 21, No. 6, December 2001.

The term "virus" refers to any of the obligate intracellular parasites having no protein-synthesizing or energy-generating mechanism. The viral genome may be RNA or DNA contained with a coated structure of protein of a lipid membrane. Examples of viruses useful herein include baculoviridiae, parvoviridiae, picornoviridiae, herepesviridiae, poxviridiae, adenoviridiae, picotrnaviridiae. The term recombinant virus includes chimeric (or even multimeric) viruses, i.e. vectors constructed using complementary coding sequences from more than one viral subtype. (See, e.g. Feng, et al. Nature Biotechnology 15:866-870) The term "adenovirus" is synonymous with the term "adenoviral vector" and refers to viruses of the genus adenoviridiae. The term adenoviridiae refers collectively to animal adenoviruses of the genus mastadenovirus including but no limited to human, bovine, ovine, equine, canine, porcine, murine and simian adenovirus subgenera. In particular, human adenoviruses includes the A-F subgenera as well as the individual serotypes thereof the individual serotypes and A-F subgenera including but not limited to human adenovirus types 1, 2, 3, 4, 4a, 5, 6, 7, 8, 9, 10, 11 (Ad11A and Ad 11P), 12, 13, 14, 15, 16, 17, 18, 19, 19a, 20, 21, 22, 23, 24, 25, 26, 27, 28, 29, 30, 31, 32, 33, 34, 34a, 35, 35p, 36, 37, 38, 39, 40, 41, 42, 43, 44, 45, 46, 47, 48, and 91. The term bovine adenoviruses includes but is not limited to bovine adenovirus types 1, 2, 3, 4, 7, and 10. The term canine adenoviruses includes but is not limited to canine types 1 (strains CLL, Glaxo, RI261, Utrect, Toronto 26-61) and 2. The term equine adenoviruses includes but is not limited to equine types 1 and 2. The term porcine adenoviruses includes but is not limited to porcine types 3 and 4. The adenovirus may be derived from the human adenovirus serotypes 2 or 5.
Adenovirus vectors can be replication-competent or replication deficient in a target cell.

The adenovirus vectors are conditionally or selectively replicating adenoviruses, wherein a gene[s] required for viral replication is [are] operatively linked to a cell and/or context-specific promoter. Examples of selectively replicating or conditionally replicating viral vectors are known in the art (see, for example, US 7,691,370). The adenovirus vector may be a conditionally replicating adenovirus wherein the E1 gene is under transcriptional control of the pre-proendothelin promoter PPE-1 (PPE-1, SEQ ID NO: 13). The adenovirus
vector may be a conditionally replicating or selectively replicating adenovirus wherein the E1 gene is under transcriptional control of the modified pre-proendothelin promoter PPE-1-3X (PPE-1-3X, SEQ ID NO: 12). Adenovirus vectors which may be
suitable for use with the present disclosure include all adenovirus serotypes having hexon protein structure. Viral vectors suitable for therapeutic use include adenoviral vectors, retrovirusal vectors, AAV, herpesvirus vectors and the like. Engineering and production of viral vectors is well known in the art, as described in detail in, for example, US Patent No: 7,732,129 or 6,649,158.

The adenovirus may be a C-type adenovirus (Ad5, Ad2), a B-type adenovirus (Ad3, Ad16, Ad21, Ad35, Ad50), an E-type adenovirus (Ad4) or an F-type adenovirus (Ad41).

As used herein, the phrase adenoviral vector refers to a vector in which, among the nucleic acid molecules in the viral particle, sequences necessary to function as a viral vector are based on the adenoviral genome.

The adenoviral vector may be a non-replicating serotype 5 (Ad5) adenoviral vector.

The adenoviral vector may comprise a sequence as set forth in SEQ ID NO: 1 or SEQ ID NO: 11.
Oncolytic viruses which reproduce themselves in cancer cells and subsequently kill the initially infected cells by lysis may be used. Such viruses proceed to infect adjacent cells thus repeating the cycle. Examples of oncolytic viruses include, but are not limited to Herpes Simplex Virus, conditionally replicative Ads (CRAds) and reoviruses.

Two major strategies for development of CRAd vectors have been developed, mainly focusing on the genetic engineering of the early 1 (E1) genes to restrict virus replication to target cells and to spare normal tissue. Genetic complementation-type (type 1) CRAds, such as Ad524, have a mutation in the immediately early (E1A) or early (E1B) adenoviral region, which is complemented in tumor cells but not in normal cells. In trans complementation-type (type 2) CRAds, virus replication is controlled via a tumor/tissue-specific promoter.

Reovirus is a naturally occurring oncolytic virus that requires activated Ras signaling pathways of tumor cells for its replication. Ras pathways are activated in most malignant gliomas via upstream signaling by receptor tyrosine kinases.

As mentioned the viral vectors of this aspect of the present invention comprise:
(i) a first polynucleotide sequence encoding a Fas-chimera (Fas-c); and
(ii) a second polynucleotide sequence encoding an endothelial cell-specific promoter for use in treating a malignant glioma.

Typically, such viral vectors are constructed using genetic recombination technology - i.e. recombinant viral vectors.

The Fas-chimera (Fas-c), is a previously described fusion of two "death receptors", constructed from the extracellular region of TNFR1 (SEQ ID NO: 2) and the trans-membrane and intracellular regions of Fas (SEQ ID NO: 3) [Boldin MP et al. J Biol Chem (1995) 270(14):7795-8].

According to one embodiment the Fas-c is encoded by a polynucleotide as set forth in SEQ NO: 4.

A viral
construct (e.g. an adenoviral construct) comprising an endothelial/periendothelial cell-specific promoter operatively linked to other cytotoxic polypeptides for the treatment of malignant glioma may be used.

Such polypeptides, include but are not limited to suicide polypeptides such as p53 and egr-1-TNF-alpha, cytotoxic pro-drug/enzymes for drug susceptibility therapy such as ganciclovir/thymidine kinase and 5-fluorocytosine/cytosine deaminase, and antimetastatic polypeptides such as 5 E1A.

The term "promoter" as used herein refers to a DNA sequence which directs transcription of a polynucleotide sequence operatively linked thereto in the cell in a constitutive or inducible manner. The promoter may also comprise enhancer elements which stimulate transcription from the linked promoter.

As used herein, the phrase endothelial cell-specific promoter refers to a promoter which directs expression of a gene operatively linked thereto in endothelial cells, wherein the level of expression in endothelial cells is at least 2 times higher than in non-endothelial cells. The level of expression in endothelial cells may be at least 5 times higher than in non-endothelial cells.

As used herein, the phrase periendothelial cell-specific promoter refers to a promoter which directs expression of a gene operatively linked thereto in periendothelial cells (i.e., pericytes in small vessels or smooth muscle cells in larger vessels), wherein the level of expression in endothelial cells is at least 2 times higher than in non-periendothelial cells.
The level of expression in periendothelial cells may be at least 5 times higher than in non-periendothelial cells.

Exemplary endothelial cell-specific promoters or periendothelial cell-specific promoters include, but are not limited to the preproendothelin-1 (PPE-1) promoter, and modifications thereof such as described herein below, the TIE-1 promoter, the TIE-2 promoter, the Endoglin promoter, the von Willerband promoter, the KDR/flk-1 promoter, The FLT-1 promoter, the Egr-1 promoter, the ICAM-1 promoter, the VCAM-1 promoter, the PECAM-1 promoter and the aortic carboxypeptidase-like protein (ACLP) promoter.

The preproendothelial promoter refers to the preproendothelin-1 promoter, of mammalian origin. The preproendothelin 1 promoter may be a murine preproendothelin 1 promoter as set forth in SEQ ID NO: 13.

The promoter may comprise at least one copy of an enhancer element that confers endothelial cell specific transcriptional activity. According to one embodiment the enhancer element is naturally found positioned between the -364 bp and -320 bp of the murine PPE-1 promoter (as set forth in SEQ ID NO: 6).. The promoter may comprise at least two or three of the above described enhancer elements. According to a specific embodiment, the promoter comprises two of the above described enhancer elements on one strand of the promoter DNA and one of the above described enhancer element on the complementary strand of the promoter DNA (as set forth in SEQ ID NO:7).

According to another embodiment, the promoter further comprises at least one hypoxia response element - e.g. comprising a sequence as set forth in SEQ ID NO: 5.

An exemplary promoter which can be used in the context of the present invention comprises a sequence as set forth in SEQ ID NO: 12. This promoter is also referred to herein as the PPE-1-3x promoter. This sequence comprises SEQ ID NO: 5 and SEQ ID NO: 7 (which itself comprises two copies of SEQ ID NO: 6 either side of one copy of SEQ ID NO: 8).

According to a particular embodiment of this aspect of the present invention, the viral vector consists of a sequence as set forth in SEQ ID NOs: 9 or 10. This viral vector is also referred to herein as VB111 and AD5PPE-1-3X-fas-chimera.

This sequence comprises SEQ ID NO: 12 in the antisense orientation at position 460-1437.

This sequence also comprises SEQ ID NO: 7 in the antisense orientation at position 894-1036; a single copy of SEQ ID NO: 8 in the antisense orientation at position 951-997; a first copy of SEQ ID NO: 6 in the antisense orientation at position 907-950; a second copy of SEQ ID NO: 6 in the antisense orientation at position 993-1036; and a third copy of SEQ ID NO: 6 at position 823-866 in the sense orientation.

The viral vector may comprise additional polynucleotide sequences capable of enhancing or inhibiting transcriptional activity of an endothelial specific promoter.
The additional polynucleotide sequence may include an isolated polynucleotide comprising at least 6 nucleotides of element X of a pre-proendothelin (PPE-1) promoter, the element X having a wild type sequence as set forth by SEQ ID NO:6, wherein the at least 6 nucleotides comprise at least 2 consecutive sequences derived from SEQ ID NO:6, each of the at least 2 consecutive sequences comprises at least 3 nucleotides, at least one of the at least 3 nucleotide being positioned next to at least one nucleotide position in SEQ ID NO:6, the at least one nucleotide position in SEQ ID NO:6 is selected from the group consisting of:
(i) at least one nucleotide of wild type M4 sequence set forth by SEQ ID NO: 15 (CATTC);
(ii) at least one nucleotide of wild type M5 sequence set forth by SEQ ID NO: 16 (CAATG);
(iii) at least one nucleotide of wild type M8 sequence set forth by SEQ ID NO: 19 (GCTTC);
(iv) at least one nucleotide of wild type M6 sequence set forth by SEQ ID NO: 17 (GGGTG);
(v) at least one nucleotide of wild type M7 sequence set forth by SEQ ID NO: 18 (ACTTT);
(vi) at least one nucleotide of wild type M1 sequence set forth by SEQ ID NO: 20 (GTACT); and
(v) at least one nucleotide of wild type M3 sequence set forth by SEQ ID NO: 21 (CTTTT);
wherein the at least one nucleotide position is mutated as compared to SEQ ID NO:6 by at least one nucleotide substitution, at least one nucleotide deletion and/or at least one nucleotide insertion, with the proviso that a mutation of the at least one nucleotide position does not result in nucleotides GGTA at position 21-24 of SEQ ID NO:6 and/or in nucleotides CATG at position 29-32 of SEQ ID NO:6, such that when the isolated polynucleotide is integrated into the PPE-1 promoter and placed upstream of a reporter gene (e.g., luciferase coding sequence) the expression level of the reporter gene is upregulated or downregulated as compared to when SEQ ID NO:6 is similarly integrated into the PPE-1 promoter and placed upstream of the reporter gene coding sequence.

The isolated polynucleotide may not be naturally occurring in a genome or a whole chromosome sequence of an organism.

As used herein the phrase "naturally occurring" refers to as found in nature, without any man-made modifications.

As described above, the at least 6 nucleotides of element X comprise at least 2 consecutive sequences derived from SEQ ID NO:6.

As used herein the phrase "consecutive sequence derived from SEQ ID NO:6 " refers to a nucleic acid sequence (a polynucleotide) in which the nucleotides appear in the same order as in the nucleic acid sequence of SEQ ID NO:6 from which they are derived. It should be noted that the order of nucleotides is determined by the chemical bond (phosphodiester bond) formed between a 3'-OH of a preceding nucleotide and the 5'-phosphate of the following nucleotide.

Each of the at least 2 consecutive sequences may comprise at least 3 nucleotides, e.g., 3 nucleotides, 4 nucleotides, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, 30 nucleotide, 31 nucleotides, 32 nucleotides, 33 nucleotides, 34 nucleotides, 35 nucleotides, 36 nucleotides, 37 nucleotides, 38 nucleotides, 39 nucleotides, 40 nucleotides, 41 nucleotides of SEQ ID NO:6.

As described, the isolated polynucleotide comprises at least 2 consecutive sequences derived from SEQ ID NO:6.

The isolated polynucleotide may comprise 2, 3, 4, 5, 6, 7, 8, 9, 10, 11, 12, 13 or 14 consecutive sequences derived from SEQ ID NO:6.

As used herein the phrase "wild type" with respect to a nucleotide sequence refers to the nucleic acid sequence as appears in SEQ ID NO:6. Examples include, but are not limited to wild type M4 sequence (SEQ ID NO: 15), wild type M5 sequence (SEQ ID NO: 16), wild type M8 (SEQ ID NO:19), wild type M6 sequence (SEQ ID NO:17), wild type M7 sequence (SEQ ID NO:18), wild type M1 (SEQ ID NO:20) and wild type M3 sequence (SEQ ID NO:21).

The mutation may be an insertion of at least one nucleotide in a nucleotide position with respect to SEQ ID NO:6.

The insertion may include at least 1, 2, 3, 4, 5, 6, 7, 8, 9, 10 nucleotides, e.g., at least about 15, at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, at least about 100, at least about 200, at least about 300, or more nucleotides.

It should be noted that the sequence which is inserted by the mutation can be derived from any source (e.g., species, tissue or cell type), and is not limited to the source of the sequence of element X.

The mutation may be a combination of any of the mutation types described above, *i.e*., substitution, insertion and deletion. For example, while one nucleotide position in SEQ ID NO:6 can be subject to a substitution mutation, another nucleotide position in SEQ ID NO:6 can be subject to a deletion or insertion. Additionally or alternatively, while one nucleotide position in SEQ ID NO:6 can be subject to a deletion mutation, another nucleotide position in SEQ ID NO:6 can be subject to a substitution or insertion. Additionally or alternatively, while one nucleotide position in SEQ ID NO:6 can be subject to an insertion mutation, another nucleotide position in SEQ ID NO:6 can be subject to a substitution or deletion. It should be noted that various other combinations are possible.

The mutation in the isolated polynucleotide of the invention may not result in nucleotides GGTA at position 21-24 of SEQ ID NO:6 and/or in nucleotides CATG at position 29-32 of SEQ ID NO:6.

As used herein the phrase "integrated into the PPE-1 promoter" refers to a nucleotide sequence (the isolated polynucleotide) which is covalently conjugated within the PPE-1 promoter sequence.

The isolated polynucleotide may further comprise at least one copy of a nucleic acid sequence selected from the group consisting of:
(i) wild type M4 sequence set forth by SEQ ID NO: 15 (CATTC),
(ii) wild type M5 sequence set forth by SEQ ID NO: 16 (CAATG),
(iii) wild type M8 sequence set forth by SEQ ID NO: 19 (GCTTC),
(iv) wild type M6 sequence set forth by SEQ ID NO: 17 (GGGTG),
(v) wild type M7 sequence set forth by SEQ ID NO: 18 (ACTTT);
(vi) wild type M1 sequence set forth by SEQ ID NO: 20 (GTACT), and
(vii) wild type M3 sequence set forth by SEQ ID NO: 21 (CTTTT).

The isolated polynucleotide may be integrated into (within), downstream of, or upstream of any known (or unknown) promoter sequence to thereby regulate (e.g., increase, decrease, modulate tissue-specificity, modulate inductive or constitutive expression) the transcriptional promoting activity of the promoter.

The isolated polynucleotide may be for increasing expression of a heterologous polynucleotide operably linked thereto in endothelial cells. Such a polynucleotide can include wild type sequences of M4 and/or M5 in the presence or absence of additional sequences from element X, and/or in the presence of other mutated sequences from element X.

The isolated polynucleotide may comprise at least one copy of the wild type M4 sequence set forth by SEQ ID NO: 15 (CATTC).

The isolated polynucleotide may comprise at least one copy of the wild type M5 sequence set forth by SEQ ID NO: 16 (CAATG).

The isolated polynucleotide may comprise at least one copy of the wild type M4 sequence set forth by SEQ ID NO: 15 (CATTC) and at least one copy of the wild type M5 sequence set forth by SEQ ID NO: 16 (CAATG).
The at least one nucleotide position which is mutated as compared to SEQ ID NO: 6 may be at least one nucleotide of the
wild type M8 sequence set forth by SEQ ID NO: 19 (GCTTC). It should be noted that such an isolated polynucleotide may further include a wild type M6 sequence (SEQ ID. NO:17) and/or a wild type M7 sequence (SEQ ID NO:18)

Non-limiting examples of isolated polynucleotides which include at least one copy of the wild type M4 sequence set forth by SEQ ID NO: 15 (CATTC) and a mutation in at least one nucleotide of the wild type M8 sequence set forth by SEQ ID NO: 19 (GCTTC) are provided in SEQ ID NOs:55-62.

Non-limiting examples of isolated polynucleotides which include at least one copy of the wild type M5 sequence set forth by SEQ ID NO: 16 (CAATG) and a mutation in at least one nucleotide of the wild type M8 sequence set forth by SEQ ID NO: 19 (GCTTC) are provided in SEQ ID NOs: 63-66.

Non-limiting examples of isolated polynucleotides which include at least one copy of the wild type M4 sequence set forth by SEQ ID NO: 15 (CATTC), at least one copy of the wild type M5 sequence set forth by SEQ ID NO: 16 (CAATG) and a mutation in at least one nucleotide of the wild type M8 sequence set forth by SEQ ID NO: 19 (GCTTC) are provided in SEQ ID NOs: 67-70.

The isolated polynucleotide may further comprising at least one copy of wild type M1 sequence set forth by SEQ ID NO: 20 (GTACT).

Non-limiting examples of isolated polynucleotides which include at least one copy of the wild type M4 sequence set forth by SEQ ID NO: 15 (CATTC), at least one copy of the wild type M1 sequence set forth by SEQ ID NO: 20 (GTACT), and a mutation in at least one nucleotide of the wild type M8 sequence set forth by SEQ ID NO: 19 (GCTTC) are provided in SEQ ID NOs: 71-105.

Non-limiting examples of isolated polynucleotides which include at least one copy of the wild type M5 sequence set forth by SEQ ID NO: 16 (CAATG), at least one copy of the wild type M1 sequence set forth by SEQ ID NO: 20 (GTACT) and a mutation in at least one nucleotide of the wild type M8 sequence set forth by SEQ ID NO: 19 (GCTTC) are provided in SEQ ID NOs: 106-136.

Non-limiting examples of isolated polynucleotides which include at least one copy of the wild type M4 sequence set forth by SEQ ID NO: 15 (CATTC), at least one copy of the wild type M5 sequence set forth by SEQ ID NO: 16 (CAATG), at least one copy of the wild type M1 sequence set forth by SEQ ID NO: 20 (GTACT) and a mutation in at least one nucleotide of the wild type M8 sequence set forth by SEQ ID NO: 19 (GCTTC) are provided in SEQ ID NOs:137-152.

The isolated polynucleotide may reduce expression of a heterologous polynucleotide operably linked thereto in endothelial cells. Such a polynucleotide can include mutations in M4 and/or M5 in the presence or absence of additional sequences from element X, and/or in the presence of other mutated sequences from element X.

The at least one nucleotide position which may be mutated as compared to SEQ ID NO:6 is at least one nucleotide of the wild type M4 sequence set forth by SEQ ID NO: 15 (CATTC).

Non-limiting examples of isolated polynucleotides which includes a mutation in at least one nucleotide of the wild type M4 sequence set forth by SEQ ID NO:46 (CATTC) are provided in SEQ ID NOs:153-162.

The at least one nucleotide position which may be mutated as compared to SEQ ID NO:6 is at least one nucleotide of the wild type M5 sequence set forth by SEQ ID NO: 16 (CAATG).

Non-limiting examples of isolated polynucleotides which include a mutation in at least one nucleotide of the wild type M5 sequence set forth by SEQ ID NO: 16 (CAATG) are provided in SEQ ID NOs:163-171.

The at least one nucleotide position which may be mutated as compared to SEQ ID NO:6 is at least one nucleotide of the wild type M4 sequence set forth by SEQ ID NO: 15 (CATTC) and at least one nucleotide of the wild type M5 sequence set forth by SEQ ID NO: 16 (CAATG).

Non-limiting examples of isolated polynucleotides which include a mutation in at least one nucleotide of the wild type M4 sequence set forth by SEQ ID NO: 15 (CATTC) and a mutation in at least one nucleotide of the wild type M5 sequence set forth by SEQ ID NO: 16 (GAATG) are provided in SEQ ID NOs:172-180.

The isolated polynucleotide may be for increasing expression of a heterologous polynucleotide operably linked thereto in cells other than endothelial cells. Such a polynucleotide can include mutations in M4 and/or M5 and wild type sequences of M6 and/or M7, in the presence or absence of additional sequences from element X, and/or in the presence of other mutated sequences from element X.

The isolated polynucleotide may comprise a mutation in M4 (SEQ ID NO: 15) and/or in M5 (SEQ ID NO: 16) and at least one copy of the wild type M6 set forth by SEQ ID NO: 17 (GGGTG) and/or at least one copy of wild type M7 set forth by SEQ ID NO:18.

Non-limiting examples of isolated polynucleotides which include a mutation in at least one nucleotide of the wild type M4 sequence set forth by SEQ ID NO: 15 (CATTC) and at least one copy of the wild type M6 set forth by SEQ ID NO: 17 (GGGTG) are provided in SEQ ID NOs:181-182.

Non-limiting examples of isolated polynucleotides which include a mutation in at least one nucleotide of the wild type M5 sequence set forth by SEQ ID NO: 16 (CAATG) and at least one copy of the wild type M6 set forth by SEQ ID NO: 17 (GGGTG) are provided in SEQ ID NOs:183-189.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG) and at least one copy of the wild type M6 set forth by SEQ ID NO: 17 (GGGTG) are provided in SEQ ID NOs:190-191.

The isolated polynucleotide may further comprise at least one copy of the wild type M7 sequence set forth by SEQ ID NO: 18 (ACTTT).

Non-limiting examples of isolated polynucleotides which include a mutation in at least one nucleotide of the wild type M4 sequence set forth by SEQ ID NO: 15 (CATTC) and at least one copy of the wild type M7 sequence set forth by SEQ ID NO: 18 (ACTTT) are provided in SEQ ID NOs:192-195.

Non-limiting examples of isolated polynucleotides which include a mutation in at least one nucleotide of the wild type M5 sequence set forth by SEQ ID NO: 16 (CAATG) and at least one copy of the wild type M7 sequence set forth by SEQ ID NO: 18 (ACTTT) are provided in SEQ ID NOs:196-198.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG) and at least one copy of the wild type M7 sequence set forth by SEQ ID NO: 18 (ACTTT) are provided in SEQ ID NOs:199-202.

The isolated polynucleotide may further comprise: at least one copy of the wild type M6 set forth by SEQ ID NO: 17 (GGGTG) and at least one copy of the wild type M7 sequence set forth by SEQ ID NO: 18 (ACTTT).

Non-limiting examples of isolated polynucleotides which include a mutation in at least one nucleotide of the wild type M4 sequence set forth by SEQ ID NO: 15 (CATTC), at least one.copy of the wild type M6 set forth by SEQ ID NO: 17 (GGGTG) and at least one copy of the wild type M7 sequence set forth by SEQ ID NO: 18 (ACTTT) are provided in SEQ ID NOs:203-205.

Non-limiting examples of isolated polynucleotides which include a mutation in at least one nucleotide of the wild type M5 sequence set forth by SEQ ID NO: 16 (CAATG), at least one copy of the wild type M6 set forth by SEQ ID NO: 17 (GGGTG) and at least one copy of the wild type M7 sequence set forth by SEQ ID NO: 18 (ACTTT) are provided in SEQ ID NOs:206-207.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M6 set forth by SEQ ID NO: 17 (GGGTG) and at least one copy of the wild type M7 sequence set forth by SEQ ID NO: 18 (ACTTT) are provided in SEQ ID NOs:208-209.

The isolated polynucleotide may reduce expression in cells of a heterologous polynucleotide operably linked thereto. Such a polynucleotide can include mutations in M4, M5, M6 and/or M7, in the presence or absence of additional sequences from element X, and/or in the presence of other mutated sequences from element X.

The isolated polynucleotide may comprise at least one mutation in wild type M4 (SEQ ID NO: 15) and/or in wild type M5 (SEQ ID NO:47) and in wild type M6 set forth by SEQ ID NO: 17 (GGGTG).

Non-limiting examples of isolated polynucleotides which include a mutation in at least one nucleotide of the wild type M4 sequence set forth by SEQ ID NO: 15 (CATTC) and a mutation in at least one nucleotide position of the wild type M6 set forth by SEQ ID NO: 17 (GGGTG) are provided in SEQ ID NOs:210-213.

Non-limiting examples of isolated polynucleotides which include a mutation in at least one nucleotide of the wild type M5 sequence set forth by SEQ ID NO: 16 (CAATG) and a mutation in at least one nucleotide position of the wild type M6 set forth by SEQ ID NO: 17 (GGGTG) are provided in SEQ ID NOs:214-222.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), and a mutation in at least one nucleotide position of the wild type M6 set forth by SEQ ID NO: 17 (GGGTG) are provided in SEQ ID NOs:223-231.

The isolated polynucleotide may further comprise at least one mutation in wild type M7 set forth by SEQ ID NO: 18 (ACTTT).

Non-limiting examples of isolated polynucleotides which include a mutation in at least one nucleotide of the wild type M4 sequence set forth by SEQ ID NO: 15 (CATTC) and a mutation in at least one nucleotide position of the wild type M7 set forth by SEQ ID NO: 18 (ACTTT) are provided in SEQ ID NOs:232-236.

Non-limiting examples of isolated polynucleotides which include a mutation in at least one nucleotide of the wild type M5 sequence set forth by SEQ ID NO: 16 (CAATG) and a mutation in at least one nucleotide position of the wild type M7 set forth by SEQ ID NO: 18 (ACTTT) are provided in SEQ ID NOs:237-240.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), and a mutation in at least one nucleotide position of the wild type M7 set forth by SEQ ID NO: 18 (ACTTT) are provided in SEQ ID NOs:241-248.

The isolated polynucleotide may further comprise at least one mutation in wild type M6 set forth by SEQ ID NO: 17 (GGGTG) and at least one mutation in wild type M7 set forth by SEQ ID NO: 18 (ACTTT).

Non-limiting examples of isolated polynucleotides which include a mutation in at least one nucleotide of the wild type M4 sequence set forth by SEQ ID NO: 15 (CATTC), a mutation in at least one nucleotide position of the wild type M6 set forth by SEQ ID NO: 17 (GGGTG) and a mutation in at least one nucleotide position of the wild type M7 set forth by SEQ ID NO: 18 (ACTTT) are provided in SEQ ID NOs:249-258.

Non-limiting examples of isolated polynucleotides which include a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG) and a mutation in at least one nucleotide position of the wild type M7 set forth by SEQ ID NO: 18 (ACTTT) are provided in SEQ ID NOs:259-264.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG) and a mutation in at least one nucleotide position of the wild type M7 set forth by SEQ ID NO: 18 (ACTTT) are provided in SEQ ID NOs:265-270.

The isolated polynucleotide may comprise at least one copy of the wild type M8 sequence set forth by SEQ ID NO: 19 (GCTTC) with additional wild type or mutated sequences derived from element X (SEQ ID NO:6).

Non-limiting examples of isolated polynucleotides which includes a mutation in at least one nucleotide of the wild type M4 sequence set forth by SEQ ID NO: 15 (CATTC) and at least one copy of the wild type M8 sequence set forth by SEQ ID NO: 19 (GCTTC) are provided in SEQ ID NOs:271-279.

Non-limiting examples of isolated polynucleotides which include a mutation in at least one nucleotide of the wild type M5 sequence set forth by SEQ ID NO: 16 (CAATG) and at least one copy of the wild type M8 sequence set forth by SEQ ID NO: 19 (GCTTC) are provided in SEQ ID NOs:280-287.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG) and at least one copy of the wild type M8 sequence set forth by SEQ ID NO: 19 (GCTTC) are provided in SEQ ID NOs:288-291.

Non-limiting examples of isolated polynucleotides which include a mutation in at least one nucleotide of the wild type M4 sequence set forth by SEQ ID NO: 15 (CATTC), at least one copy of the wild type M6 set forth by SEQ ID NO: 17 (GGGTG) and at least one copy of the wild type M8 sequence set forth by SEQ ID NO: 19 (GCTTC) are provided in SEQ ID NOs:294-298.

Non-limiting examples of isolated polynucleotides which include a mutation in at least one nucleotide of the wild type M5 sequence set forth by SEQ ID NO: 16 (CAATG), at least one copy of the wild type M6 set forth by SEQ ID NO: 17 (GGGTG) and at least one copy of the wild type M8 sequence set forth by SEQ ID NO: 19 (GCTTC) are provided in SEQ ID NOs:299-301.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M6 set forth by SEQ ID NO: 17 (GGGTG) and at least one copy of the wild type M8 sequence set forth by SEQ ID NO: 19 (GCTTC) are provided in SEQ ID NOs:302-303.

Non-limiting examples of isolated polynucleotides which include a mutation in at least one nucleotide of the wild type M4 sequence set forth by SEQ ID NO: 15 (CATTC), at least one copy of the wild type M7 sequence set forth by SEQ ID NO: 18 (ACTTT) and at least one copy of the wild type M8 sequence set forth by SEQ ID NO: 19 (GCTTC) are provided in SEQ ID NOs:304-308.

Non-limiting examples of isolated polynucleotides which include a mutation in at least one nucleotide of the wild type M5 sequence set forth by SEQ ID NO: 16 (CAATG), at least one copy of the wild type M7 sequence set forth by SEQ ID NO: 18 (ACTTT) and at least one copy of the wild type M8 sequence set forth by SEQ ID NO: 19 (GCTTC) are provided in SEQ ID NOs:309-311.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M7 sequence set forth by SEQ ID NO: 18 (ACTTT) and at least one copy of the wild type M8 sequence set forth by SEQ ID NO: 19 (GCTTC) are provided in SEQ ID NOs:312-315.

Non-limiting examples of isolated polynucleotides which include a mutation in at least one nucleotide of the wild type M4 sequence set forth by SEQ ID NO: 15 (CATTC), at least one copy of the wild type M6 set forth by SEQ ID NO: 17 (GGGTG), at least one copy of the wild type M7 sequence set forth by SEQ ID NO: 18 (ACTTT) and at least one copy of the wild type M8 sequence set forth by SEQ ID NO: 19 (GCTTC) are provided in SEQ ID NO:316.

Non-limiting examples of isolated polynucleotides which include a mutation in at least one nucleotide of the wild type M5 sequence set forth by SEQ ID NO: 16 (CAATG), at least one copy of the wild type M6 set forth by SEQ ID NO: 17 (GGGTG), at least one copy of the wild type M7 sequence set forth by SEQ ID NO: 18 (ACTTT) and at least one copy of the wild type M8 sequence set forth by SEQ ID NO: 19 (GCTTC) are provided in SEQ ID NO:317.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M6 set forth by SEQ ID NO: 17 (GGGTG), at least one copy of the wild type M7 sequence set forth by SEQ ID NO: 18 (ACTTT) and at least one copy of the wild type M8 sequence set forth by SEQ ID NO: 19 (GCTTC) are provided in SEQ ID NO:318.

Non-limiting examples of isolated polynucleotides which include a mutation in at least one nucleotide of the wild type M4 sequence set forth by SEQ ID NO: 15 (CATTC), a mutation in at least one nucleotide position of the wild type M6 set forth by SEQ ID NO: 17 (GGGTG) and at least one copy of the wild type M8 sequence set forth by SEQ ID NO: 19 (GCTTC) are provided in SEQ ID NOs:319-327.

Non-limiting examples of isolated polynucleotides which include a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG) and at least one copy of the wild type M8 sequence set forth by SEQ ID NO: 19 (GCTTC) are provided in SEQ ID NOs:328-333.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG) and at least one copy of the wild type M8 sequence set forth by SEQ ID NO: 19 (GCTTC) are provided in SEQ ID NOs:334-337.

Non-limiting examples of isolated polynucleotides which include a mutation in at least one nucleotide of the wild type M4 sequence set forth by SEQ ID NO: 15 (CATTC), a mutation in at least one nucleotide position of the wild type M7 set forth by SEQ ID NO: 18 (ACTTT) and at least one copy of the wild type M8 sequence set forth by SEQ ID NO: 19 (GCTTC) are provided in SEQ ID NOs:338-344.

Non-limiting examples of isolated polynucleotides which include a mutation in at least one nucleotide of the wild type M5 sequence set forth by SEQ ID NO: 16 (CAATG), a mutation in at least one nucleotide position of the wild type M7 set forth by SEQ ID NO: 18 (ACTTT) and at least one copy of the wild type M8 sequence set forth by SEQ ID NO: 19 (GCTTC) are provided in SEQ ID NOs:345-348.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), a mutation in at least one nucleotide position of the wild type M7 set forth by SEQ ID NO: 18 (ACTTT) and at least one copy of the wild type M8 sequence set forth by SEQ ID NO: 19 (GCTTC) are provided in SEQ ID NOs:349-354.

Non-limiting examples of isolated polynucleotides which include a mutation in at least one nucleotide of the wild type M4 sequence set forth by SEQ ID NO: 15 (CATTC), a mutation in at least one nucleotide position of the wild type M6 set forth by SEQ ID NO: 17 (GGGTG), a mutation in at least one nucleotide position of the wild type M7 set forth by SEQ ID NO: 18 (ACTTT) and at least one copy of the wild type M8 sequence set forth by SEQ ID NO: 19 (GCTTC) are provided in SEQ ID NOs:355-361.

Non-limiting examples of isolated polynucleotides which include a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG), a mutation in at least one nucleotide position of the wild type M7 set forth by SEQ ID NO: 18 (ACTTT) and at least one copy of the wild type M8 sequence set forth by SEQ ID NO: 19 (GCTTC) are provided in SEQ ID NOs:362-365.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG), a mutation in at least one nucleotide position of the wild type M7 set forth by SEQ ID NO: 18 (ACTTT) and at least one copy of the wild type M8 sequence set forth by SEQ ID NO: 19 (GCTTC) are provided in SEQ ID NOs:366-369.

The isolated polynucleotide may comprise at least one copy of the wild type M3 sequence set forth by SEQ ID NO: 21 (CTTTT) with additional wild type or mutated sequences derived from element. X (SEQ ID NO:6).

Non-limiting examples of isolated polynucleotides which includes a mutation in at least one nucleotide of the wild type M4 sequence set forth by SEQ ID NO: 15 (CATTC) and at least one copy of the wild type M3 sequence set forth by SEQ ID NO: 21 (CTTTT) are provided in SEQ ID NOs:378-384.

Non-limiting examples of isolated polynucleotides which include a mutation in at least one nucleotide of the wild type M5 sequence set forth by SEQ ID NO: 16 (CAATG) and at least one copy of the wild type M3 sequence set forth by SEQ ID NO: 21 (CTTTT) are provided in SEQ ID NOs:628-634.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG) and at least one copy of the wild type M3 sequence set forth by SEQ ID NO: 21 (CTTTT) are provided in SEQ ID NOs:370-377.

Non-limiting examples of isolated polynucleotides which include a mutation in at least one nucleotide of the wild type M4 sequence set forth by SEQ ID NO: 15 (CATTC), at least one copy of the wild type M6 set forth by SEQ ID NO: 17 (GGGTG) and at least one copy of the wild type M3 sequence set forth by SEQ ID NO: 21 (CTTTT) are provided in SEQ ID NOs:385-390.

Non-limiting examples of isolated polynucleotides which include a mutation in at least one nucleotide of the wild type M5 sequence set forth by SEQ ID NO: 16 (CAATG), at least one copy of the wild type M6 set forth by SEQ ID NO: 17 (GGGTG) and at least one copy of the wild type M3 sequence set forth by SEQ ID NO: 21 (CTTTT) are provided in SEQ ID NOs:391-396.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M6 set forth by SEQ ID NO: 17 (GGGTG) and at least one copy of the wild type M3 sequence set forth by SEQ ID NO: 21 (CTTTT) are provided in SEQ ID NOs:397-401.

Non-limiting examples of isolated polynucleotides which include a mutation in at least one nucleotide of the wild type M4 sequence set forth by SEQ ID NO: 15 (CATTC), at least one copy of the wild type M7 sequence set forth by SEQ ID NO: 18 (ACTTT) and at least one copy of the wild type M3 sequence set forth by SEQ ID NO: 21 (CTTTT) are provided in SEQ ID NOs:402-409.

Non-limiting examples of isolated polynucleotides which include a mutation in at least one nucleotide of the wild type M5 sequence set forth by SEQ ID NO: 16 (CAATG), at least one copy of the wild type M7 sequence set forth by SEQ ID NO: 18 (ACTTT) and at least one copy of the wild type M3 sequence set forth by SEQ ID NO: 21 (CTTTT) are provided in SEQ ID NOs:410-417.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M7 sequence set forth by SEQ ID NO: 18 (ACTTT) and at least one copy of the wild type M3 sequence set forth by SEQ ID NO: 21 (CTTTT) are provided in SEQ ID NOs:418-423.

Non-limiting examples of isolated polynucleotides which include a mutation in at least one nucleotide of the wild type M4 sequence set forth by SEQ ID NO: 15 (CATTC), at least one copy of the wild type M6 set forth by SEQ ID NO: 17 (GGGTG), at least one copy of the wild type M7 sequence set forth by SEQ ID NO: 18 (ACTTT) and at least one copy of the wild type M3 sequence set forth by SEQ ID NO: 21 (CTTTT) are provided in SEQ ID NOs:424-425.

Non-limiting examples of isolated polynucleotides which include a mutation in at least one nucleotide of the wild type M5 sequence set forth by SEQ ID NO: 16 (CAATG), at least one copy of the wild type M6 set forth by SEQ ID NO: 17 (GGGTG), at least one copy of the wild type M7 sequence set forth by SEQ ID NO: 18 (ACTTT) and at least one copy of the wild type M3 sequence set forth by SEQ ID NO: 21 (CTTTT) are provided in SEQ ID NOs:538-540.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M6 set forth by SEQ ID NO: 17 (GGGTG), at least one copy of the wild type M7 sequence set forth by SEQ ID NO: 18 (ACTTT) and at least one copy of the wild type M3 sequence set forth by SEQ ID NO: 21 (CTTTT) are provided in SEQ ID NO:426.

Non-limiting examples of isolated polynucleotides which include a mutation in at least one nucleotide of the wild type M4 sequence set forth by SEQ ID NO: 15 (CATTC), a mutation in at least one nucleotide position of the wild type M6 set forth by SEQ ID NO: 17 (GGGTG) and at least one copy of the wild type M3 sequence set forth by SEQ ID NO: 21 (CTTTT) are provided in SEQ ID NOs:427-435.

Non-limiting examples of isolated polynucleotides which include a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG) and at least one copy of the wild type M3 sequence set forth by SEQ ID NO: 21 (CTTTT) are provided in SEQ ID NOs:436-444.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG) and at least one copy of the wild type M3 sequence set forth by SEQ ID NO: 21 (CTTTT) are provided in SEQ ID NOs:445-451.

Non-limiting examples of isolated polynucleotides which include a mutation in at least one nucleotide of the wild type M4 sequence set forth by SEQ ID NO: 15 (CATTC), a mutation in at least one nucleotide position of the wild type M7 set forth by SEQ ID NO: 18 (ACTTT) and at least one copy of the wild type M3 sequence set forth by SEQ ID NO: 21 (CTTTT) are provided in SEQ ID NOs:452-458.

Non-limiting examples of isolated polynucleotides which include a mutation in at least one nucleotide of the wild type M5 sequence set forth by SEQ ID NO: 16 (CAATG), a mutation in at least one nucleotide position of the wild type M7 set forth by SEQ ID NO: 18 (ACTTT) and at least one copy of the wild type M3 sequence set forth by SEQ ID NO: 21 (CTTTT) are provided in SEQ ID NOs:459-465.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), a mutation in at least one nucleotide position of the wild type M7 set forth by SEQ ID NO: 18 (ACTTT) and at least one copy of the wild type M3 sequence set forth by SEQ ID NO: 21 (CTTTT) are provided in SEQ ID NO:466.

Non-limiting examples of isolated polynucleotides which include a mutation in at least one nucleotide of the wild type M4 sequence set forth by SEQ ID NO: 15 (CATTC), a mutation in at least one nucleotide position of the wild type M6 set forth by SEQ ID NO: 17 (GGGTG), a mutation in at least one nucleotide position of the wild type M7 set forth by SEQ ID NO: 18 (ACTTT) and at least one copy of the wild type M3 sequence set forth by SEQ ID NO: 21 (CTTTT) are provided in SEQ ID NOs:467-471.

Non-limiting examples of isolated polynucleotides which include a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG), a mutation in at least one nucleotide position of the wild type M7 set forth by SEQ ID NO: 18 (ACTTT) and at least one copy of the wild type M3 sequence set forth by SEQ ID NO: 21 (CTTTT) are provided in SEQ ID NOs:472-477.

Non-limiting examples of isolated polynucleotides which include a mutation in at least one nucleotide of the wild type M4 sequence set forth by SEQ ID NO: 15

(CATTC), a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG), a mutation in at least one nucleotide position of the wild type M7 set forth by SEQ ID NO: 18 (ACTTT) and at least one copy of the wild type M3 sequence set forth by SEQ ID NO: 21 (CTTTT) are provided in SEQ ID NOs:478-483.

The isolated polynucleotide may further comprise at least one copy of the wild type M8 sequence set forth by SEQ ID NO: 19 (GCTTC) and at least one copy of the wild type M3 sequence set forth by SEQ ID NO: 21 (CTTTT) with additional wild type or mutated sequences derived from element X (SEQ ID NO:6).

Non-limiting examples of isolated polynucleotides which includes a mutation in at least one nucleotide of the wild type M4 sequence set forth by SEQ ID NO: 15 (CATTC), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:484-495.

Non-limiting examples of isolated polynucleotides which include a mutation in at least one nucleotide of the wild type M5 sequence set forth by SEQ ID NO: 16 (CAATG), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:496-507.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:508-515.

Non-limiting examples of isolated polynucleotides which include a mutation in at least one nucleotide of the wild type M4 sequence set forth by SEQ ID NO: 15 (CATTC), at least one copy of the wild type M6 set forth by SEQ ID NO: 17 (GGGTG), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:516-519.

Non-limiting examples of isolated polynucleotides which include a mutation in at least one nucleotide of the wild type M5 sequence set forth by SEQ ID NO: 16 (CAATG), at least one copy of the wild type M6 set forth by SEQ ID NO: 17 (GGGTG), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:520-523.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M6 set forth by SEQ ID NO: 17 (GGGTG), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:524-525.

Non-limiting examples of isolated polynucleotides which include a mutation in at least one nucleotide of the wild type M4 sequence set forth by SEQ ID NO: 15 (CATTC), at least one copy of the wild type M7 sequence set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:526-529.

Non-limiting examples of isolated polynucleotides which include a mutation in at least one nucleotide of the wild type M5 sequence set forth by SEQ ID NO: 16 (CAATG), at least one copy of the wild type M7 sequence set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:530-533.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M7 sequence set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:534-535.

Non-limiting examples of isolated polynucleotides which include a mutation in at least one nucleotide of the wild type M4 sequence set forth by SEQ ID NO: 15 (CATTC), at least one copy of the wild type M6 set forth by SEQ ID NO: 17 (GGGTG), at least one copy of the wild type M7 sequence set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT)are provided in SEQ ID NOs:536-537.

Non-limiting examples of isolated polynucleotides which include a mutation in at least one nucleotide of the wild type M5 sequence set forth by SEQ ID NO: 16 (CAATG), at least one copy of the wild type M6 set forth by SEQ ID NO: 17 (GGGTG), at least one copy of the wild type M7 sequence set forth by SEQ ID NO: 18 (ACTTT) at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:538-539.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), at least one copy of the wild type M6 set forth by SEQ ID NO: 17 (GGGTG), at least one copy of the wild type M7 sequence set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NO:540.

Non-limiting examples of isolated polynucleotides which include a mutation in at least one nucleotide of the wild type M4 sequence set forth by SEQ ID NO: 15 (CATTC), a mutation in at least one nucleotide position of the wild type M6 set forth by SEQ ID NO: 17 (GGGTG), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:541-547.

Non-limiting examples of isolated polynucleotides which include a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:548-554.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:555-559.

Non-limiting examples of isolated polynucleotides which include a mutation in at least one nucleotide of the wild type M4 sequence set forth by SEQ ID NO: 15 (CATTC), a mutation in at least one nucleotide position of the wild type M7 set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:560-566.

Non-limiting examples of isolated polynucleotides which include a mutation in at least one nucleotide of the wild type M5 sequence set forth by SEQ ID NO: 16 (CAATG), a mutation in at least one nucleotide position of the wild type M7 set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:567-573.

Non-limiting examples of isolated polynucleotides which include a mutation in at least one nucleotide of the wild type M4 sequence set forth by SEQ ID NO: 15 (CATTC), a mutation in at least one nucleotide of the wild type M5 sequence set forth by SEQ ID NO: 16 (CAATG), a mutation in at least one nucleotide position of the wild type M7 set forth by SEQ ID NO: 18 (ACITT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:574-578.

Non-limiting examples of isolated polynucleotides which include a mutation in at least one nucleotide of the wild type M4 sequence set forth by SEQ ID NO: 15 (CATTC), a mutation in at least one nucleotide position of the wild type M6 set forth by SEQ ID NO: 17 (GGGTG), a mutation in at least one nucleotide position of the wild type M7 set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:579-583.

Non-limiting examples of isolated polynucleotides which include a mutation in at least one nucleotide of the wild type M5 sequence set forth by SEQ ID NO: 16 (CAATG), a mutation in at least one nucleotide position of the wild type M6 set forth by SEQ ID NO: 17 (GGGTG), a mutation in at least one nucleotide position of the wild type M7 set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:584-588.

Non-limiting examples of isolated polynucleotides which include a mutation in at least one nucleotide of the wild type M4 sequence set forth by SEQ ID NO: 15 (CATTC), mutation in at least one nucleotide of the wild type M5 sequence set forth by SEQ ID NO: 16 (CAATG), a mutation in at least one nucleotide position of the wild type M6 set forth by SEQ ID NO: 17 (GGGTG), a mutation in at least one nucleotide position of the wild type M7 set forth by SEQ ID NO: 18 (ACTTT), at least one copy of the wild type M8 sequence set forth by SEQ ID NO: 19 (GCTTC) and at least one copy of the wild type M3 sequence set forth by SEQ ID NO: 21 (CTTTT) are provided in SEQ ID NOs:589-592.

The isolated polynucleotide may comprise at least one copy of wild type M3 sequence (SEQ ID NO: 21) and at least one copy of wild type M8 sequence (SEQ ID NO: 19), with at least one mutation in wild type M6 (SEQ ID NO: 17) and/or in wild type M7 (SEQ ID NO:50).

Non-limiting examples of isolated polynucleotides which include at least one copy of the wild type M8 sequence set forth by SEQ ID NO: 19 (GCTTC) and at least one copy of the wild type M3 sequence set forth by SEQ ID NO: 21 (CTTTT), with a mutation in at least one nucleotide of the wild type M6 sequence (SEQ ID NO: 17), and/or a mutation in at least one nucleotide of the wild type M7 (SEQ ID NO: 18) are provided in SEQ ID NOs:593-600.

The present inventors have envisaged that an isolated polynucleotide which includes the wild type M8 sequence (SEQ ID NO: 19) and/or the wild type M3 (SEQ ID NO: 21) sequence in addition to tissue specific enhancers (e.g., wild type M4 and/or wild type M5), and/or induced enhancers (e.g., developmentally related- or stress related-enhancers) is expected to exert a more specific regulatory effect by suppressing expression in non-target cells or under non-induced conditions.

The isolated polynucleotide may comprise at least one copy of the wild type M8 sequence set forth by SEQ ID NO: 19 (GCTTC) and an endothelial specific enhancer sequence.

The isolated polynucleotide may comprise at least one copy of the wild type M8 sequence set forth by SEQ ID NO: 19 (GCTTC) and at least one copy of wild type M4 sequence set forth by SEQ ID NO: 15.

The isolated polynucleotide may comprise at least one copy of the wild type M8 sequence set forth by SEQ ID NO: 19 (GCTTC) and at least one copy of wild type M5 sequence set forth by SEQ ID NO:16.

The isolated polynucleotide may comprise at least one copy of the wild type M8 sequence set forth by SEQ ID NO: 19 (GCTTC), at least one copy of wild type M4 sequence set forth by SEQ ID NO: 15 and at least one copy of wild type M5 sequence set forth by SEQ ID NO:16.

The isolated polynucleotide may comprise at least one copy of the wild type M3 sequence set forth by SEQ ID NO: 21 (CTTTT) and an endothelial specific enhancer sequence.

The isolated polynucleotide may comprise at least one copy of the wild type M3 sequence set forth by SEQ ID NO: 21 (CTTTT) and at least one copy of wild type M4 sequence set forth by SEQ ID NO: 15.

The isolated polynucleotide may comprise at least one copy of the wild type M3 sequence set forth by SEQ ID NO: 21 (CTTTT) and at least one copy of wild type M5 sequence set forth by SEQ ID NO: 16.

The isolated polynucleotide may comprise at least one copy of the wild type M3 sequence set forth by SEQ ID NO: 21 (CTTTT), at least one copy of wild type M4 sequence set forth by SEQ ID NO: 15 and at least one copy of wild type M5 sequence set forth by SEQ ID NO:16.

The isolated polynucleotide may comprise at least one copy of the wild type M3 sequence set forth by SEQ ID NO: 21 (CTTTT), at least one copy of wild type M8 sequence set forth by SEQ ID NO: 19 (GCTTC) and an endothelial specific enhancer sequence.

The isolated polynucleotide may comprise at least one copy of the wild type M3 sequence set forth by SEQ ID NO: 21 (CTTTT), at least one copy of wild type M8 sequence set forth by SEQ ID NO: 19 (GCTTC) and at least one copy of wild type M4 sequence set forth by SEQ ID NO: 15.

The isolated polynucleotide may comprise at least one copy of the wild type M3 sequence set forth by SEQ ID NO: 21 (CTTTT), at least one copy of wild type M8 sequence set forth by SEQ ID NO: 19 (GCTTC) and at least one copy of wild type M5 sequence set forth by SEQ ID NO: 16.

The isolated polynucleotide may comprise at least one copy of the wild type M3 sequence set forth by SEQ ID NO: 21 (CTTTT), at least one copy of wild type M8 sequence set forth by SEQ ID NO: 19 (GCTTC), at least one copy of wild type M4 sequence set forth by SEQ ID NO: 15 and at least one copy of wild type M5 sequence set forth by SEQ ID NO: 16.

The isolated polynucleotide may comprise at least one copy of the wild type M3 sequence set forth by SEQ ID NO: 21 (CTTTT), at least one copy of wild type M8 sequence set forth by SEQ ID NO: 19 (GCTTC) and at least one enhancer element such as wild type M6 (SEQ ID NO: 17) and/or wild type M7 sequence (SEQ ID NO:18).

The isolated polynucleotide may include at least one copy of wild type M8 with additional flanking sequences such as at least one copy of a wild type M8 sequence (SEQ ID NO:19), at least one copy of wild type M7 (SEQ ID NO: 18) and/or wild type M9 sequence (SEQ ID NO: 14, CTGGA); and/or the isolated polynucleotide includes at least one copy of wild type M8 and at least one mutation in M7, with or without M9 (SEQ ID NO: 22). Such polynucleotides can be used as a non-specific repressor.

The isolated polynucleotide may be for increasing expression of a heterologous polynucleotide operably linked thereto in cells/tissues.

The isolated polynucleotide may comprise at least one copy of wild type M6 sequence set forth by SEQ ID NO: 17 (GGGTG) and/or at least one copy of wild type M7 sequence set forth by SEQ ID NO: 18 (ACTTT).

The isolated polynucleotide may include: at least one copy of wild type M6 (SEQ ID NO: 17) and a mutation in at least one nucleotide of wild type M8 (SEQ ID NO: 19).

Non-limiting examples of isolated polynucleotide which include at least one copy of wild type M6 (SEQ ID NO: 17) and a mutation in at least one nucleotide of the wild type M8 (SEQ ID NO: 19) are provided in SEQ ID NOs:23-26.

The isolated polynucleotide may include at least one copy of wild type M7 (SEQ ID NO: 18) and a mutation in at least one nucleotide of wild type M8 (SEQ ID NO: 19).

Non-limiting examples of isolated polynucleotide which include at least one copy of wild type M7 (SEQ ID NO: 18) and a mutation in at least one nucleotide of the wild type M8 (SEQ ID NO: 19) are provided in SEQ ID NOs:27-28.

The isolated polynucleotide may include at least one copy of wild type M6 (SEQ ID NO: 17), at least one copy of wild type M7 (SEQ ID NO: 18) and a mutation in at least one nucleotide of wild type M8 (SEQ ID NO: 19).

The isolated polynucleotide may include at least one copy of wild type M1 (SEQ ID NO: 20) and a mutation in at least one nucleotide of wild type M8 (SEQ ID NO: 19).

Non-limiting examples of isolated polynucleotide which include at least one copy of wild type M1 (SEQ ID NO: 20) and a mutation in at least one nucleotide of the wild type M8 (SEQ ID NO: 19) are provided in SEQ ID NOs:43-54 and 601-632.

The isolated polynucleotide may include at least one copy of wild type M1 (SEQ ID NO: 20), at least one copy of wild type M6 (SEQ ID NO: 17) and/or at least one copy of wild type M7 (SEQ ID NO: 18) and a mutation in at least one nucleotide of wild type M8 (SEQ ID NO: 19).

Non-limiting examples of isolated polynucleotides which include a mutation in at least one nucleotide of wild type M8 (SEQ ID NO: 19) and at least one copy of wild type M1 (SEQ ID NO: 20), wild type M6 (SEQ ID NO: 17) and/or wild type M7 (SEQ ID NO: 18) are provided in SEQ ID NOs:29-42.

Additional examples of regulatory isolated polynucleotides which can be used are disclosed (SEQ ID NOs: 633-644) in the Examples.section which follows. in the Examples section which follows.

There is disclosed an isolated polynucleotide comprising a nucleic acid sequence which comprises a first polynucleotide comprising the pre-proendothelin (PPE-1) promoter set forth by SEQ ID NO:13 and a second polynucleotide comprising at least one copy of a nucleic acid sequence selected from the group consisting of:
(i) wild type M4 sequence set forth by SEQ ID NO: 15 (CATTC),
(ii) wild type M5 sequence set forth by SEQ ID NO: 16 (CAATG),
(iii) wild type M8 sequence set forth by SEQ ID NO: 19 (GCTTC),
(iv) wild type M6 sequence set forth by SEQ ID NO: 17 (GGGTG),
(v) wild type M7 sequence set forth by SEQ ID NO: 18 (ACTTT);
(vi) wild type M1 sequence set forth by SEQ ID NO: 20 (GTACT), and
(vii) wild type M3 sequence set forth by SEQ ID NO: 21 (CTTTT);
with the proviso that the second polynucleotide is not SEQ ID NO:6 (element X), and wherein the isolated polynucleotide is not SEQ ID NO:12 (PPE-1-3X).

Each of the wild type M4, M5, M8, M6, M7 and/or M1 sequences may be placed in a head to tail (5'→3') orientation with respect to the PPE-1 promoter set forth by SEQ ID NO:13.

Each of the wild type M4, M5, M8, M6, M7 and/or M1 sequences may be placed in a tail to head (3'→5') orientation with respect to the PPE-1 promoter set forth by SEQ ID NO:13.

The wild type M4, M5, M8, M6, M7 and/or M1 sequences may be place in various orientations (head to tail or tail to head) and/or sequential order with respect the other wild type M4, M5, M8, M6, M7 and/or M1 sequences, and/or with respect to the orientation of SEQ ID NO:13.

Construction of such viral vectors may be effected using known molecular biology techniques such as those described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986].

Construction of the viral vector of SEQ ID NO: 9 is described in International Application WO/2008/132729.

The viral vector described herein may be for use in administration per se or as part of a pharmaceutical composition which also includes a physiologically acceptable carrier. The purpose of a pharmaceutical composition is to facilitate administration of the active ingredient to an organism.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the viral vector of the present invention accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, inrtaperitoneal, intranasal, or intraocular injections. Injection of the viral vectors into a spinal fluid can also be used as a mode of administration.

In order to enhance delivery of the virus to the central nervous system (CNS) various approaches may be taken. These include: neurosurgical strategies (e.g., intracerebral injection or intracerebroventricular infusion) and molecular manipulation of the virus. Thus for example, Tang et al., Gene Therapy (2007) 14, 523-532 teaches re-directing Ad5 vectors to the MTf transcytosis pathway in order to cross the BBB by manipulating the virus to express a full-length melanotransferrin (sCAR-MTf) polypeptide.

Other approaches for enhancing the delivery of the virus to the CNS include pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide).

The viral vectors may be engineered in order to avoid, suppress or manipulate the immune response, ideally resulting in sustained expression and immune tolerance to the transgene product - such methods are described for example in Nayak et al., Gene Therapy (12 November 2009).

Alternately, one may administer the pharmaceutical composition in a local rather than systemic manner, for example, via injection of the pharmaceutical composition directly into the brain of a patient and even more directly into the tumor cells themselves.

Pharmaceutical compositions may be manufactured by processes well known in the art, e.g., by means of conventional mixing, dissolving, granulating, dragee-making, levigating, emulsifying, encapsulating, entrapping or lyophilizing processes.

Pharmaceutical compositions for use thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, e.g. in physiologically compatible buffers such as Hank's solution, Ringer's solution, or physiological salt buffer. For transmucosal administration, penetrants appropriate to the barrier to be permeated are used in the formulation. Such penetrants are generally known in the art.

For buccal administration, the compositions may take the form of tablets or lozenges formulated in conventional manner.

For administration by nasal inhalation, the active ingredients for use are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition may also be formulated in rectal compositions such as suppositories or retention enemas, using, e.g., conventional suppository bases such as cocoa butter or other glycerides.

Pharmaceutical compositions suitable for use include compositions wherein the active ingredients are contained in an amount effective to achieve the intended purpose. More specifically, a therapeutically effective amount means an amount of active ingredients (i.e. viral particles) effective to prevent, alleviate or ameliorate symptom of a disorder (e.g., glioblastoma) or prolong the survival of the subject being treated.

Determination of a therapeutically effective amount is well within the capability of those skilled in the art, especially in light of the detailed disclosure provided herein.

For any preparation used herein the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide brain levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

The therapeutically effective amount of the active ingredient can be formulated in a unit dose. As used herein "unit dose" refers to a physically discrete unit containing a predetermined quantity of an active material calculated to individually or collectively produce a desired effect such as an anti-cancer effect. A single unit dose or a plurality of unit doses can be used to provide the desired effect, such as an anti-cancer therapeutic effect.

According to one embodiment, about 10³ to about 10¹⁶ virus particles may be used for administration to the subject.

According to another embodiment, about 10⁵ to about 10¹³ virus particles may be used for administration to the subject.

According to one embodiment, about 10⁷ to about 10¹² virus particles may be used for administration to the subject.

According to one embodiment, about 1x10¹² to about 5x10¹² virus particles may be used for administration to the subject.

According to one embodiment, about 1x10¹³ to about 5x10¹³ virus particles may be used for administration to the subject.

The subject may be administered intravenously with 1x10¹²- 1x10¹³ viral particles of SEQ ID NO: 9. or SEQ ID NO: 10.

The subject may be administered intravenously with at least two doses of 1xl0¹² - 1x10¹³ viral particles of SEQ ID NO: 9. or SEQ ID NO: 10. The subject may be administered intravenously with at least three or more doses of 1x10¹² - 1x10¹³ viral particles of SEQ ID NO: 9. or SEQ ID NO: 10. At least two doses may be administered at least about 1day, at least about 3 days, at least about 5 days, at least about 7 days, at least about 2 weeks, at least about 3 weeks, at least about 4 weeks, at least about 2 months, at least about 6 months, at least about 9 months, at least about 1 year, at least about 1.25 years, at least about 1.5 years, at least about 1.75 years, at least about 2 years, at least about 2.5 years, at least about 3 years or more apart.

Compositions may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation formulated in a compatible pharmaceutical carrier may also be prepared, placed in an appropriate container, and labeled for treatment of an indicated condition, as is further detailed above.

The vectors disclosed herein may be administered with additional ingredients which may improve the uptake of the nucleic acid construct by the cells, expression of the chimeric polypeptide by the nucleic acid construct in the cells, or the activity of the expressed chimeric polypeptide.

For example, the uptake of adenoviral vectors into EC cells can be enhanced by treating the vectors with engineered antibodies or small peptides. Such "adenobody" treatment, was shown effective in directing adenovirus constructs to EGF receptors on cells (Watkins et al 1997, Gene Therapy 4:1004-1012). In addition, Nicklin et al have shown that a small peptide, isolated via phage display, increased specificity and efficiency of vectors in endothelial cells and decreased the expression in liver cells in culture (Nicklin et al 2000, Circulation 102:231-237). In a recent study, an FGF retargeted adenoviral vector reduced the toxicity of tk in mice (Printz et al 2000, Human Gene Therapy 11:191-204).

Low dose radiation has been shown to cause breaks in DNA strands primarily in the G2/M phase, cell membrane damage enhancing the bystander effect, and thus may potentiate other cytotoxic and anti-neoplastic therapies, when administered in combination. Vascular endothelial cells may be particularly suitable to such combination, or adjunct, therapies, since it has been demonstrated that low dose radiation specifically targets the apoptotic system of the microvascular endothelial cells (Kolesnick et al., Oncogene 2003; 22:5897-906). Angiostatin has been shown to potentiate the therapeutic effects of low dose radiation (Gorski et al. Can Res 1998;58:5686-89). However, the effects of radiation are still poorly understood, since irradiation has also been shown to increase pro-angiogenic "tissue repair factors" (Itasaka et al., Am Assoc Canc Res, 2003; abstract 115). Similarly, certain chemotherapeutic agents have been shown to activate specific cytotoxic and apoptotic pathways [doxorubicin, cisplatin and mitomycin C induce accumulation of Fas receptor, FADD, and other proapoptotic signals in the FADD/MORT-1 pathway (Micheau et al., BBRC 1999 256:603-07)].

For example International Application WO/2008/132729 teaches combined doxorubicin and AdPPE-1 (3x)-Fas-c chimera construct administration in endothelial cells (BAEC). Thus, the viral vectors and the pharmaceutical compositions comprising same as described herein can be used to treat malignant gliomas alone or in combination with one or more other established or experimental therapeutic regimen for such disorders. Therapeutic regimen for treatment of malignant gliomas suitable for combination with the viral vectors described herein include, but are not limited to chemotherapy, radiotherapy, phototherapy and photodynamic therapy, surgery, nutritional therapy, ablative therapy, combined radiotherapy and chemotherapy, brachiotherapy, proton beam therapy, immunotherapy, cellular therapy and photon beam radiosurgical therapy.

Anti-cancer drugs that can be co-administered with the compounds of the disclosure include, but are not limited to Acivicin; Aclarubicin; Acodazole Hydrochloride; Acronine; Adriamycin; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta- I a; Interferon Gamma- I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Sulofenur; Talisomycin; Taxol; Tecogalan Sodium; Tegafur; Teloxantrone Hydrochloride; Temoporfin; Teniposide; Teroxirone; Testolactone; Thiamiprine; Thioguanine; Thiotepa; Tiazofuirin; Tirapazamine; Topotecan Hydrochloride; Toremifene Citrate; Trestolone Acetate; Triciribine Phosphate; Trimetrexate; Temozolomide (Temodar™); Bevacizumab, Dorafinib, Sorafenib (Nexavar™), Sunitinib (Sutent™), Vandetanib (ZD6474; Zactima™), Pazopanib (GW786034), and Vatalanib (PTK787), Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride. Additional antineoplastic agents include those disclosed in Chapter 52, Antineoplastic Agents (Paul Calabresi and Bruce A. Chabner), and the introduction thereto, 1202-1263, of Goodman and Gilman's "The Pharmacological Basis of Therapeutics", Eighth Edition, 1990, McGraw-Hill, Inc. (Health Professions Division).

The viral vectors described herein may also be administered with an agent that enhances expression of transgenes in adenoviral-mediated transient expression. For example International Application WO/2008/132729 teaches administration of a corticosteroid (e.g. dexamethasone and/or N-Acetyl Cysteine (NAC) prior to AdPPE-1 (3x)-Fas-c chimera construct administration.

In addition, the viral vectors described herein may also be administered with an agent that brings about transient immunosuppression, such as for example deoxyspergualin (DSG) or cyclophosphamide (see for example Smith et al., Gene Ther. 1996 Jun;3(6):496-502) in order to allow for repetitive dosing.

As used herein the term "about" refers to ± 10 %.

The terms "comprises", "comprising", "includes", "including"; "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format.

Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996).

Other general references are provided throughout this document. The procedures therein are believed to be well known in the art and are provided for the convenience of the reader.

### EXAMPLE 1

### Effect of VB-111 in an animals model of glioblastoma

### MATERIALS AND METHODS

*Construction and cloning of the viral vector:* The vector was constructed using a backbone containing most of the genome of adenovirus type 5, as well as partial homology to an adaptor plasmid, which enables recombination.

The E1 early transcriptional unit was deleted from the backbone plasmid, and further modified by deleting the pWE25 and the Amp resistance selection marker site.

The adaptor plasmid, containing sequences of the Ad5, CMV promoter, MCS, and SV40 polyA was modified to delete deleting the CMV promoter, and the PPE-1 promoter and Fas-c fragment were inserted by restriction digestion.

The modified PPE-1 promoter (PPE-1-3X, SEQ ID NO: 12) and the Faschimera transgene (Fas-c, SEQ ID NO: 4) were utilized for construction of the adenoviral vector. The PPE-1- (3X)-Fas-c element (2115bp) was constructed from the PPE-1- (3X)-luc element. This element contains the 1.4kb of the murine preproendothelin PPE-1-(3X) promoter, the Luciferase gene, the SV40 polyA site and the first intron of the murine ET-1 gene, originated from the pEL8 plasmid (8848bp) used by Harats et al (Harats D. et al., JCI, 1995). The PPE-3-Luc cassette was extracted from the pEL8 plasmid using the BamHI restriction enzyme. The Luciferase gene was substituted by the Fas-c gene [composed of the extra cellular and intra membranal domains of the human TNF-R1 (Tumor Necrosis Factor Receptor 1, SEQ ID NO: 2) and of the Fas (p55) intracellular domain (SEQ ID NO: 3) (Boldin et al, JBC, 1995)] to obtain the PPE-1-3x-Fas-c cassette.

PPE-1(3x)-Fas-c Plasmid - The cassette was further introduced into the backbone plasmid by restriction digestion, resulting with the PPE-1(3x)-Fas-c plasmid.

Adaptor-PPE-1(3x)-Fas-c Plasmid - The PPE-1-3x-Fas-c element was extracted from the first generation construct PPE-1-3x-Fas-c plasmid, and was amplified with designated PCR primers introducing SnaB1 and EcoR1 restriction sites at the 5'-and-3'-end respectively. These sites were used to clone the PPE-Fas-c fragment into the adaptor plasmid digested with SnaB1 and EcoR1, resulting in the adaptor-PPE-1-3x-Fas-c used for transfection of the host cells (for example, PER.C6 cells).

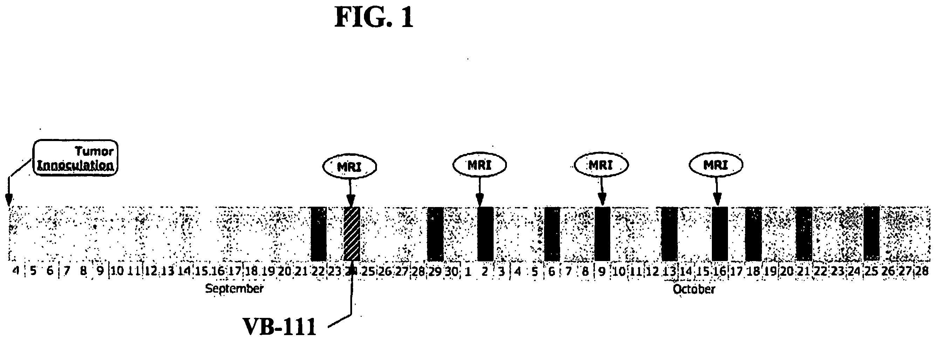

***Xenografts:*** 10⁶ U87 human glioma tumor cells expressing a biofluorescent/bioluminescent protein (luciferase) were implanted intracranially in the striatum of athymic nude rats (NxGen BioSciences). Animals were anesthesized with isoflurane prior to implantation. Briefly, glioma tumor cells expressing luciferase were implanted intracranially in the striatum of athymic nude rats (NxGen BioSciences). Animals were anesthesized with isoflurane prior to implantation and placed secured on a Just for Mice Stereotaxic (Stoelting) apparatus. A 1cm incision was made in the scalp and the bregma identified. A small burr hole was made in the cranium using a mounted Micromotor drill (Stoelting) at the identified position (1 mm forward and 4 mm lateral of the bregma). 1x10⁶ cells in a volume of 5ul were injected into the caudate over 5 minutes using a Quintessential Stereotaxic Injector (Stoelting) containing a 10ul Hamilton syringe mounted to the stereotaxic device to assure appropriate placement. Animals were imaged following isofluorane sedation using an IVIS chemiluminescence system. The fluorescence/bioluminescence of these tumors are typically detectable within 7-10 days due to rapid growth and high expression of the marker. An alternative imaging modality, i.e. MRI, was also utilized to assist with tumor visualization. Animals received chemical anesthesia for MRI imaging. Once tumor establishment and growth was detected (variable depending on the rate of growth for the respective line), rats were treated with VB-111. The total dose was 10¹¹ vp in a volume of 100 ul. Control groups received vehicle only. Animals were monitored for tumor growth or response through non-invasive imaging of fluorescence/luminescence - see Figure 1 for a typical treatment and monitoring regimen.

***Experimental protocol:*** Two types of tumor growth experiments were performed, tumor growth inhibition (TGI) and tumor growth delay (TGD). The TGI experiment was terminated when the animals showed clinical signs of tumor development i.e. become dull, listless, or moribund, usually prior to 4 weeks post-implantation, as median survival of 28-29 days has been typically observed in prior studies. Upon termination, all rats were weighed, sacrificed, and their tumors excised. For a TGD experiment, animals were sacrificed on an individual basis and tumor-related parameters (e.g. size) were measured. The average day of sacrifice was determined for all groups, and the tumor growth delay (TGD) for each treatment group compared to the control group was calculated.

***MRI:*** Magnetic resonance imaging has shown to be capable of demonstrating early changes within the tumor vasculature without any invasive measures. It is possible to generate maps of blood volume and blood flow, vascular permeability, white matter tracks, and apparent diffusion coefficient. These parameters offer clinically relevant physiological information that could help to characterize, stage tumor growth, and evaluate treatment efficacy. MRI was performed on a Bruker 7 Tesla scanner. Blood flow and blood volume was measured using dynamic contrast enhanced imaging technique following a bolus of gadopentetate-dimeglumine (GdDTPA). White matter tracks and apparent diffusion coefficient was measured using diffusion tensor imaging. Vascular permeability was measured using T1-weighted MRI obtained prior to and following contrast (Gd-DTPA) injection. For dynamic contrast enhanced MRI, single-shot gradient echo planar imaging (EPI) was used, resolution at 0.27x0.27x0.5 mm, 5 slices (no gap), matrix = 96x96, field of view = 25.6x25.6 mm, repetition time TR = 0.5 s, echo time TE = 20 ms. For diffusion tensor imaging, single-shot spin-echo echo-planar imaging was used, resolution be 0.27x0.27x0.5 mm, 15 slices (no gap), matrix = 96x96, field of view = 25.6x25.6 mm, repetition time TR = 2 s, echo time TE = 40 ms, b value = 0 s/mm2, and 6 diffusion direction of b = 1100 s/mm2. For T1-weighted MRI, conventional acquisition was used, resolution 0.27x0.27x0.5 mm, 15 slices (no gap), matrix = 96x96, field of view = 25.6x25.6 mm, repetition time TR = 0.5 s, echo time TE = 20 ms. The number of slices analyzed adequately covered the entire tumor region and roughly cover the entire cerebrum.

The maps described above were calculated using standard software. For permeability maps, the maps were processed using codes in Matlab to obtain maps of Ktrans (corresponding roughly to wash-in rates of the contrastagent. Ktrans can be influenced by flow, or by permeability, or both. In high-flow organs such as the brain, flow limitations are not usually a concern, but the blood-brain barrier severely limits permeability unless it is disrupted by disease. Even in such a state, Ktrans does not fully correspond to permeability, but it is related rather to the permeability*surface area product of the capillary bed (in nonflow-limited situations).

***Histopathology:*** To further characterize changes at the microscopic level, animals were sacrificed by cardiac puncture, followed by intracardiac saline and formalin irrigation. Necropsy was performed, and brains underwent standard H&E processing. The number of vessels per medium power field were counted.

### RESULTS

As illustrated in Figure 2, animal death began at approximately day 32. The median survival for the control group was 39.25 (+/-3.8) days and for the treatment group was 45.8 days.

***Luciferase activity:*** Luciferase activity was followed by ip injection of luciferin and optical imaging on a Xenogen system. The region of interest was generated automatically without manipulation and total photons recorded. As illustrated in Figure 3, a clear separation in activity was observed at day 33 with a mean (SD) in the control group of 9.7 (2.9) x 10⁶ versus 5.3 (6.2) x 10⁵ in the treated group.

***MRI:*** As illustrated in Figure 4, mean of the maximum diameters of tumors in the VB111 treated group was smaller than those for controls.

### EXAMPLE 2

### Effect of VB-111 in glioblastoma patients

***Treatment plan:*** VB-111 will be administered as a single intravenous infusion of 1x10¹² or 3x10¹² Dose.

Study consists of 2 cohorts.
Cohort 1a: 3-6 subjects, safety (1x10¹² VPs);
Cohort 1b: 3-6 subjects, safety (3x10¹² VPs);
Cohort 2: 23-26 subjects, efficacy & safety (3x10¹²VPs)
Cohort 1a & Cohort 1b: Study subjects will be enrolled sequentially. The first subject of each cohort will be treated and observed for 14 days; if no dose-limiting toxicities (DLT) are observed, then another two subjects will be recruited to that cohort. All six subjects of cohort 1 need to be observed for a minimum of 14 days and show no DLT for the start of the next cohort. If a DLT is observed in one patient in a specific dosing cohort, three additional subjects will be accrued for the same dosing cohort, and safety will be reassessed. If DLT is confirmed, i.e. two out of six subjects experience a DLT, then the study will be discontinued. All subjects in cohorts 1a and 1b must be observed for a minimum of 28 days prior to commencing cohort 2.

The study will be conducted according to the Simon's 2 step method. A total of 29 subjects are anticipated to enroll at the 3x1012 VP dose level (3-6 from cohort 2 and 23-26 in cohort 3). Step one will include the first 10 patients at this dose level. A subject will be considered to have a response if s/he has either 6 months progression free survival or at least a partial tumor response according to Rano criteria. An interim analysis will be performed after 10 patients from cohorts 2 and 3 have completed the study. If 2 or more responses occur in the step 1 subjects, step 2 will commence, enrolling an additional 19 subjects.

The following study stopping rule for halting the study will be applied:
A) If 3 out of 6 (or 5 out of 9 or 6 out of 12) subjects in the cohort 1a & 1b experience drug related DLT.
B) If 2 out of the subjects in cohort 1a experience a DLT.
C) If ANY death occurs within two weeks after the product is given, except death due to disease progression or clearly unrelated to study drug. Enrollment will be temporarily suspended for an ad hoc, emergency IDMC meeting to review the case and make a recommendation if enrolment can be reinstated.

When safety end point is achieved for cohorts 1a and 1b (day 28), eligible subjects will be enrolled into the study and commence cohort 2. It is expected that 26 GBM subjects will be enrolled into cohort 2 for additional safety and efficacy endpoints (or 23 patients if 6 were enrolled in cohort 2).

One dose of VB 111 (1x10¹², 3x10¹² or 1x10¹³ VPs) administered within 3 weeks after the screening visit. Subjects will return to the clinic for follow up visits at days 4, 7, 14 and 28 and on monthly schedule on days 56, 84, 112, 140 and 168 if no disease progression has occurred prior to the visit.

On days'7, 14, 28, 56, 112 and 168, subjects will be assessed for response using contrast and non-contrast brain magnetic resonance imaging (MRI) with assessment based on the RANO criteria.

The post study follow up period will include telephone contacts every two months after day 168 , early termination, or disease progression (whichever occurs earlier) to fellow up on survival. Follow up will continue until patient expires. The study duration is 7 months (6 months post dose), thereafter the subjects will be followed by telephone for survival data every two months. Surveillance MRIs will be performed every 2 months until 1 year, and then every 3 months until 2 years post dosing (or until progression).
***Population:*** Up to 35 eligible subjects (cohort 1 & cohort 2) with relapsed GBM.

### Main inclusion criteria:

1. Ability to understand and the willingness to sign a written informed consent document.
2. Subjects ≥18 years of age
3. Subjects must have histologically confirmed diagnosis of primary malignant glioma (glioblastoma multiforme, gliosarcoma or anaplastic astrocytoma, or anaplastic oligodendroglioma). Subjects with recurrent disease whose diagnostic pathology confirmed malignant glioma (glioblastoma multiforme, gliosarcoma or anaplastic astrocytoma, or anaplastic oligodendroglioma) will not need re-biopsy.
4. Evidence of measurable recurrent or residual primary CNS neoplasm on contrast-enhanced MRI, unless medically contraindicated (CT scan will then be used).
5. Measurable disease by RANO criteria.
6. Avastin and anti-angiogenic (TKIs such as sunitinib or sorafenib) naive subjects.
7. Disease progression or recurrence following standard of care treatment with radiotherapy and temozolomide.
8. An interval of at least 4 weeks between prior surgical resection and study enrolment.
9. Completed radiotherapy ≥90 days before study starts.
10. An interval of at least 12 weeks between prior radiotherapy or at least 4 weeks from prior chemotherapy, and enrolment on this protocol.
11. Recovered to Grade 1 or less from the toxic effects of any earlier intervention.
12. Karnofsky performance status ≥ 60%.
13. Adequate renal, liver, and bone marrow function according to the following criteria:
   - Absolute neutrophil count ≥1500/mcL
   - Platelets ≥125 000/mcL
   - Total bilirubin within upper limit of normal (ULN)
   - Aspartate aminotransferase (AST) ≤ 2.5 X institutional ULN
   - Creatinine within normal limits or creatinine clearance ≥ 50 mL/min for patients with creatinine levels above normal limits.
   - PT, PTT greater than 80% of the lower normal limits.
13. Subjects must be treated with corticosteroids on day 0. Subjects will be on a stable dose for 1 *week* prior to entry, and is not anticipated to require increase in steroid dose throughout the study.
14. No evidence of haemorrhage on the baseline MRI or CT scan.
15. Males and Females of childbearing potential must utilize, throughout the course of the trial a standard contraception method.

**Cohort 1a and 1b additional eligibility criteria:** Subjects without major mass effect of Tumor.

### Main exclusion criteria:

1. Pregnant or breastfeeding subjects
2. Co-medication that may interfere with study results; e.g. immunosuppressive agents other than corticosteroids
3. Active Infection.
4. Greater than 3 prior recurrences.
5. Evidence of CNS haemorrhage CTCAE on baseline MRI or CT scan.
6. Requires therapeutic anti-coagulation.
7. Prior anti-angiogenic therapy including VEGF-sequestering agents (e.g. bevicizumab, aflibercept) or VEGF inhibitors (e.g. cedirinib, pazopanib, sunitinib, sorafenib).
8. Prior stereotactic radiotherapy.
9. Known active secondary malignancy.
10. Expected to have surgery during study period.
11. Subjects, who suffered from an acute cardiac event within the last 12 months.
12. Subjects with active vascular disease, either myocardial or peripheral
13. Subjects with proliferative and/or vascular retinopathy
14. Subjects with known liver disease (alcoholic, drug/toxin induced, genetic, or autoimmune).
15. Subjects with known CNS metastatic disease (other than GBM).
16. Subjets with known active second malignancy.
17. Subjects testing positive to one of the following viruses: HIV, HBV and HCV
18. Subjects that have undergone major surgery within the last 4 weeks before enrolment.
19. Subjects may not have received any other investigational agent within 4 weeks before enrolment.
20. Uncontrolled intercurrent illness including, but not limited to ongoing or active infection, symptomatic congestive heart failure, unstable angina pectoris, cardiac arrhythmia, or psychiatric illness/social situations that would limit compliance with study requirements

***Test drug and formulation:*** VB-111 (SEQ ID NO: 9 or 10) is formulated as a sterile vector solution. The solution is supplied frozen (below -65°C), in single use, plastic screw cap vials. Each vial contains 1.1 mL of vector solution at a viral titer of 1x10¹² VP/ml. The vector solution should be thawed and maintained on ice during dilution and handling for a maximum of 3 hours.

***Dosage and administration:*** Prior to infusion, the solution for injection should be brought to room temperature Maximum time for drug in saline is 1 hour at room temperature. The vials should be opened in a biological safety cabinet and injected into 4 mL of normal saline for infusion for each 1 ml of drug. ie; for the 1x10¹² viral particle (VP) dose 1ml of drug+4ml of saline, for the 3x10¹² VP dose 3ml of drug+12ml of saline. A single infusion of approximately 5 mL/15ml of diluted VB-111 should be administered 1 mL/minute.

***Safety Evaluations:*** Adverse events will be recorded on an ongoing basis and up to 2 months following the administration of the test drug. Adverse events will be assessed for seriousness, relatedness to study drug, and severity (according to CTCAE 4.0). Vital signs will be recorded at screening, prior to dosing, 30, 60 minutes, 4 and 6 hours after dosing and at all patient visits. A physical examination will be conducted at screening, days 14, 28, 56, 84, 112, 140, 168 and at the end of the study. A 12 lead ECG will be obtained at screening, prior to dosing and on days 28 and 168 (or ET). Safety laboratory assessment (blood haematology and chemistry, urine analysis) will be conducted at screening, prior to dosing, and at all patient visits, starting from day 4±1 to 168 ±7.

***Distribution:*** Blood and urine samples will be collected prior to dosing, at the end of the infusion, days 4, 7, 14, 28 and 56, for evaluation of levels of virus DNA (in whole blood and urine) and its transgene (in whole blood).

***Tumor response:*** Tumor response will be assessed at screening, prior to dosing, days 14, 28, 56, 112, 140 and 168, and then every 2 months for 1 year and every 3 months for 2 years post dosing, using contrast and non-contrast brain magnetic resonance imaging (MRI) with assessment based on the RANO criteria, until progression of disease (local and central independent radiology review). For patients who do not progress or die, PFS will be censored at the time of initiation of alternative anticancer therapy, date of last radiologic assessment, or time of last contact.

## Claims

1. A therapeutically effective amount of a viral vector for use in the treatment of a malignant glioma in a subject in need thereof, comprising a nucleic acid construct which comprises:
(i) a first polynucleotide sequence encoding a Fas-chimera (Fas-c), and
(ii) a second polynucleotide sequence encoding an endothelial cell-specific promoter, which is operably linked to the first polynucleotide sequence.

2. The viral vector for use of claim 1, wherein the malignant glioma is selected from the group consisting of glioblastoma, astrocytoma, oligodendroglioma, ependymoma, and juvenile pilocystic astrocytoma.

3. The viral vector for use of claim 1 or 2, wherein the therapeutically effective amount is about 10³ to about 10¹⁶ virus particles, about 10⁵ to about 10¹³ virus particles, about 10⁷ to about 10¹² virus particles, about 1x10¹² to about 5x10¹² virus particles, or about 1x10¹³ to about 5x10¹³ virus particles.

4. The viral vector for use of any one of claims 1 to 3, wherein the virus vector is administered in at least two doses or at least three doses.

5. The viral vector for use of any one of claims 1 to 4, wherein the virus vector is administered via intravenous administration or local administration.

6. The viral vector for use of any one of claims 1 to 5, wherein said viral vector is a non-replicating adenoviral vector.

7. The viral vector for use of any one of claims 1 to 6, wherein the Fas-c comprises an extracellular domain of TNFR1 and a trans-membrane region and an intracellular region of Fas.

8. The viral vector for use of any one of claims 1 to 7, wherein the Fas-c comprises (i) SEQ ID NO: 2 and SEQ ID NO: 3 or (ii) SEQ ID NO: 4.

9. The viral vector for use of any one of claims 1 to 8, wherein the promoter comprises the sequence set forth in SEQ ID NO: 8 or the complementary sequence thereof.

10. The viral vector for use of claim 9, wherein (i) the promoter further comprises at least one copy of the sequence set forth in SEQ ID NO: 6 or the complementary sequence thereof or (ii) the promoter comprises the sequence set forth in SEQ ID NO: 7 or a complementary sequence thereof.

11. The viral vector for use of any one of claims 1 to 10, wherein said promoter comprises a hypoxia response element (HRE) comprising the sequence set forth in SEQ ID NO: 5.

12. The viral vector for use of any one of claims 1 to 11, wherein the promoter comprises SEQ ID NO: 12.

13. The viral vector for use of any one of claims 1 to 12, wherein the viral vector consists of the sequence set forth in SEQ ID NO: 9 or SEQ ID NO: 10.

14. The viral vector for use of any one of claims 1 to 13, wherein the malignant glioma is glioblastoma.

15. The viral vector for use of any one of claims 1 to 3, wherein the virus vector is administered as a single unit dose.

16. The viral vector for use of any one of claims 1 to 15, wherein the virus vector is adenovirus serotype 5.

## Patentansprüche

1. Therapeutisch wirksame Menge eines viralen Vektors zur Verwendung bei der Behandlung eines malignen Glioms bei einem Individuum, das einer solchen bedarf, umfassend ein Nucleinsäurekonstrukt, das umfasst:
(i) eine erste Polynucleotidsequenz, die eine Fas-Chimäre (Fas-c) codiert, und
(ii) eine zweite Polynucleotidsequenz, die einen Endothelzellenspezifischen Promotor codiert, der funktionell mit der ersten Polynucleotidsequenz verknüpft ist.

2. Viraler Vektor zur Verwendung nach Anspruch 1, wobei das maligne Gliom ausgewählt ist aus der Gruppe bestehend aus Glioblastom, Astrocytom, Oligodendrogliom, Ependymom und juvenilem pilocystischem Astrocytom.

3. Viraler Vektor zur Verwendung nach Anspruch 1 oder 2, wobei die therapeutisch wirksame Menge etwa 10³ bis etwa 10¹⁶ Viruspartikel, etwa 10⁵ bis etwa 10¹³ Viruspartikel, etwa 10⁷ bis etwa 10¹² Viruspartikel, etwa 1 x 10¹² bis etwa 5 x 10¹² Viruspartikel oder etwa 1 x 10¹³ bis etwa 5 x 10¹³ Viruspartikel ist.

4. Viraler Vektor zur Verwendung nach einem der Ansprüche 1 bis 3, wobei der virale Vektor in mindestens zwei Dosen oder mindestens drei Dosen verabreicht wird.

5. Viraler Vektor zur Verwendung nach einem der Ansprüche 1 bis 4, wobei der virale Vektor intravenös oder lokal verabreicht wird.

6. Viraler Vektor zur Verwendung nach einem der Ansprüche 1 bis 5, wobei der virale Vektor ein nicht-replizierender Adenovirusvektor ist.

7. Viraler Vektor zur Verwendung nach einem der Ansprüche 1 bis 6, wobei die Fas-c eine extrazelluläre Domäne von TNFR1 und eine transmembrane Region und eine intrazelluläre Region von Fas umfasst.

8. Viraler Vektor zur Verwendung nach einem der Ansprüche 1 bis 7, wobei die Fas-c (i) SEQ ID NO:2 und SEQ ID NO:3 oder (ii) SEQ ID NO:4 umfasst.

9. Viraler Vektor zur Verwendung nach einem der Ansprüche 1 bis 8, wobei der Promotor die in SEQ ID NO:8 gezeigte Sequenz oder die dazu komplementäre Sequenz umfasst.

10. Viraler Vektor zur Verwendung nach Anspruch 9, wobei (i) der Promotor des Weiteren mindestens eine Kopie der in SEQ ID NO:6 gezeigten Sequenz oder die dazu komplementäre Sequenz davon umfasst, oder (ii) der Promotor die in SEQ ID NO:7 gezeigte Sequenz oder eine dazu komplementäre Sequenz umfasst.

11. Viraler Vektor zur Verwendung nach einem der Ansprüche 1 bis 10, wobei der Promotor ein Hypoxie-Response-Element (HRE) umfasst, das die in SEQ ID NO:5 gezeigte Sequenz umfasst.

12. Viraler Vektor zur Verwendung nach einem der Ansprüche 1 bis 11, wobei der Promotor SEQ ID NO:12 umfasst.

13. Viraler Vektor zur Verwendung nach einem der Ansprüche 1 bis 12, wobei der virale Vektor aus der in SEQ ID NO:9 oder SEQ ID NO:10 gezeigten Sequenz besteht.

14. Viraler Vektor zur Verwendung nach einem der Ansprüche 1 bis 13, wobei das maligne Gliom ein Glioblastom ist.

15. Viraler Vektor zur Verwendung nach einem der Ansprüche 1 bis 3, wobei der virale Vektor als eine einzige Dosiseinheit verabreicht wird.

16. Viraler Vektor zur Verwendung nach einem der Ansprüche 1 bis 15, wobei der Virusvektor das Adenovirus vom Serotyp 5 ist.

## Revendications

1. Quantité thérapeutiquement efficace d'un vecteur viral pour son utilisation dans le traitement d'un gliome malin chez un sujet en ayant besoin, comprenant une construction d'acide nucléique qui comprend :
(i) une première séquence de polynucléotides codant pour une Fas-chimère (Fas-c), et
(ii) une seconde séquence de polynucléotides codant pour un promoteur spécifique des cellules endothéliales, qui est fonctionnellement lié à la première séquence de polynucléotides.

2. Vecteur viral pour son utilisation selon la revendication 1, dans lequel le gliome malin est choisi dans le groupe constitué par le glioblastome, l'astrocytome, l'oligodendrogliome, épendymome, et l'astrocytome pilocytique juvénile.

3. Vecteur viral pour son utilisation selon la revendication 1 ou 2, dans lequel la quantité thérapeutiquement efficace est d'environ 10³ à environ 10¹⁶ particules virales, d'environ 10⁵ à environ 10¹³ particules virales, d'environ 10⁷ à environ 10¹² particules virales, d'environ 1 x 10¹² à environ 5 x 10¹² particules virales, ou d'environ 1 x 10¹³ à environ 5 x 10¹³ particules virales.

4. Vecteur viral pour son utilisation selon l'une quelconque des revendications 1 à 3, dans lequel le vecteur viral est administré en au moins deux doses voire au moins trois doses.

5. Vecteur viral pour son utilisation selon l'une quelconque des revendications 1 à 4, dans lequel le vecteur viral est administré par administration par voie intraveineuse ou administration par voie locale.

6. Vecteur viral pour son utilisation selon l'une quelconque des revendications 1 à 5, dans lequel ledit vecteur viral est un vecteur adénoviral non réplicatif.

7. Vecteur viral pour son utilisation selon l'une quelconque des revendications 1 à 6, dans lequel la Fas-c comprend un domaine extracellulaire de TNFR1 et une région transmembranaire et une région intracellulaire de Fas.

8. Vecteur viral pour son utilisation selon l'une quelconque des revendications 1 à 7, dans lequel la Fas-c comprend (i) SEQ ID No : 2 et SEQ ID No : 3 ou (ii) SEQ ID No:4.

9. Vecteur viral pour son utilisation selon l'une quelconque des revendications 1 à 8, dans lequel le promoteur comprend la séquence représentée dans SEQ ID No : 8 ou la séquence complémentaire de celle-ci.

10. Vecteur viral pour son utilisation selon la revendication 9, dans lequel (i) le promoteur comprend en outre au moins une copie de la séquence représentée dans SEQ ID No : 6 ou la séquence complémentaire de celle-ci ou (ii) le promoteur comprend la séquence représentée dans SEQ ID No : 7 ou une séquence complémentaire de celle-ci.

11. Vecteur viral pour son utilisation selon l'une quelconque des revendications 1 à 10, dans lequel ledit promoteur comprend un élément de réponse à l'hypoxie (HRE) comprenant la séquence représentée dans SEQ ID No : 5.

12. Vecteur viral pour son utilisation selon l'une quelconque des revendications 1 à 11, dans lequel le promoteur comprend SEQ ID No:12.

13. Vecteur viral pour son utilisation selon l'une quelconque des revendications 1 à 12, dans lequel le vecteur viral est constitué de la séquence représentée dans SEQ ID No : 9 ou SEQ ID No : 10.

14. Vecteur viral pour son utilisation selon l'une quelconque des revendications 1 à 13, dans lequel le gliome malin est un glioblastome.

15. Vecteur viral pour son utilisation selon l'une quelconque des revendications 1 à 3, dans lequel le vecteur viral est administré sous la forme d'une monodose.

16. Vecteur viral pour son utilisation selon l'une quelconque des revendications 1 à 15, dans lequel le vecteur viral est un adénovirus sérotype 5.


### SEQUENCE LISTING

<110> vascular Biogenics Ltd. Cohen, Yael Bangio, Livnat Brenner, Andrew J. Breitbart, Eyal
<120> COMPOSITIONS AND METHODS FOR TREATING GLIOBLASTOMA GBM
<130> 50376
<150> US 61/262,228
   <151> 2010-01-05
<150> US 61/282,248
   <151> 2010-01-07
<160> 644
<170> PatentIn version 3.5
<210> 1
   <211> 34350
   <212> DNA
   <213> Artificial sequence
<220>
   <223> Empty Ad5 vector sequence without repeats
<400> 1
<210> 2
   <211> 590
   <212> DNA
   <213> Artificial sequence
<220>
   <223> TNFR portion of the TNFR-Fas chimera (Fas-c)
<400> 2
   atgggcctct ccaccgtgcc tgacctgctg ctgccgctgg tgctcctgga gctgttggtg    60
   ggaatatacc cctcaggggt tattggactg gtccctcacc taggggacag ggagaagaga   120
   gatagtgtgt gtccccaagg aaaatatatc caccctcaaa ataattcgat ttgctgtacc   180
   aagtgccaca aaggaaccta cttgtacaat gactgtccag gcccggggca ggatacggac   240
   tgcagggagt gtgagagcgg ctccttcacc gcttcagaaa accacctcag acactgcctc   300
   agctgctcca aatgccgaaa ggaaatgggt caggtggaga tctcttcttg cacagtggac   360
   cgggacaccg tgtgtggctg caggaagaac cagtaccggc attattggag tgaaaacctt   420
   ttccagtgct tcaattgcag cctctgcctc aatgggaccg tgcacctctc ctgccaggag   480
   aaacagaaca ccgtgtgcac ctgccatgca ggtttctttc taagagaaaa cgagtgtgtc   540
   tcctgtagta actgtaagaa aagcctggag tgcacgaagt tgtgcctacc              590
<210> 3
   <211> 511
   <212> DNA
   <213> Artificial sequence
<220>
   <223> Fas portion of the TNFR-Fas chimera (Fas-c)
<400> 3
   aagcttagga tccagatcta acttggggtg gctttgtctt cttcttttgc caattccact    60
   aattgtttgg gtgaagagaa aggaagtaca gaaaacatgc agaaagcaca gaaaggaaaa   120
   ccaaggttct catgaatctc caaccttaaa tcctgaaaca gtggcaataa atttatctga   180
   tgttgacttg agtaaatata tcaccactat tgctggagtc atgacactaa gtcaagttaa   240
   aggctttgtt cgaaagaatg gtgtcaatga agccaaaata gatgagatca agaatgacaa   300
   tgtccaagac acagcagaac agaaagttca actgcttcgt aattggcatc aacttcatgg   360
   aaagaaagaa gcgtatgaca cattgattaa agatctcaaa aaagccaatc tttgtactct   420
   tgcagagaaa attcagacta tcatcctcaa ggacattact agtgactcag aaaattcaaa   480
   cttcagaaat gaaatccaaa gcttggtcta g                                  511
<210> 4
   <211> 1101
   <212> DNA
   <213> Artificial sequence
<220>
   <223> TNFR1-Fas chimera (Fas-c) coding sequence
<400> 4
   atgggcctct ccaccgtgcc tgacctgctg ctgccgctgg tgctcctgga gctgttggtg     60
   ggaatatacc cctcaggggt tattggactg gtccctcacc taggggacag ggagaagaga    120
   gatagtgtgt gtccccaagg aaaatatatc caccctcaaa ataattcgat ttgctgtacc    180
   aagtgccaca aaggaaccta cttgtacaat gactgtccag gcccggggca ggatacggac    240
   tgcagggagt gtgagagcgg ctccttcacc gcttcagaaa accacctcag acactgcctc    300
   agctgctcca aatgccgaaa ggaaatgggt caggtggaga tctcttcttg cacagtggac    360
   cgggacaccg tgtgtggctg caggaagaac cagtaccggc attattggag tgaaaacctt    420
   ttccagtgct tcaattgcag cctctgcctc aatgggaccg tgcacctctc ctgccaggag    480
   aaacagaaca ccgtgtgcac ctgccatgca ggtttctttc taagagaaaa cgagtgtgtc    540
   tcctgtagta actgtaagaa aagcctggag tgcacgaagt tgtgcctacc aagcttagga    600
   tccagatcta acttggggtg gctttgtctt cttcttttgc caattccact aattgtttgg    660
   gtgaagagaa aggaagtaca gaaaacatgc agaaagcaca gaaaggaaaa ccaaggttct    720
   catgaatctc caaccttaaa tcctgaaaca gtggcaataa atttatctga tgttgacttg    780
   agtaaatata tcaccactat tgctggagtc atgacactaa gtcaagttaa aggctttgtt    840
   cgaaagaatg gtgtcaatga agccaaaata gatgagatca agaatgacaa tgtccaagac    900
   acagcagaac agaaagttca actgcttcgt aattggcatc aacttcatgg aaagaaagaa    960
   gcgtatgaca cattgattaa agatctcaaa aaagccaatc tttgtactct tgcagagaaa   1020
   attcagacta tcatcctcaa ggacattact agtgactcag aaaattcaaa cttcagaaat   1080
   gaaatccaaa gcttggtcta g                                             1101
<210> 5
   <211> 6
   <212> DNA
   <213> Artificial sequence
<220>
   <223> Hypoxia responsive element - E-box
<400> 5
   gcacgt 6
<210> 6
   <211> 44
   <212> DNA
   <213> Artificial sequence
<220>
   <223> Murine endothelial specific enhancer element
<400> 6
   gtacttcata cttttcattc caatggggtg actttgcttc tgga 44
<210> 7
   <211> 143
   <212> DNA
   <213> Artificial sequence
<220>
   <223> A triplicate copy of a murine enhancer sequence originated from the PPE-1 promoter
<400> 7
   gtacttcata cttttcattc caatggggtg actttgcttc tggagggtga ctttgcttct      60
   ggagccagta cttcatactt ttcattgtac ttcatacttt tcattccaat ggggtgactt     120
   tgcttctgga ggctagctgc cag                                             143
<210> 8
   <211> 47
   <212> DNA
   <213> Artificial sequence
<220>
   <223> EDC fragment
<400> 8
   ctggagggtg actttgcttc tggagccagt acttcatact tttcatt 47
<210> 9
   <211> 36460
   <212> DNA
   <213> Artificial sequence
<220>
   <223> PPE-1-3X-FasC virl construct including repeat sequences
<220>
   <221> misc_feature
   <222> (460)..(1437)
   <223> A modified murine pre-proendothelin-1 promoter (PPE-1-3X)
<220>
   <221> misc_feature
   <222> (1202)..(1207)
   <223> Hypoxia response element
<220>
   <221> misc_feature
   <222> (1438)..(1468)
   <223> Linker containing Restriction sites (NotI, PstI, BamHI)
<220>
   <221> misc_feature
   <222> (1469)..(2058)
   <223> TNFR portion of the Fas-TNFR-1 chimera (Fas-c)
<220>
   <221> misc_feature
   <222> (2059)..(2569)
   <223> FAS portion of the Fas-TNFR-1 chimera (Fas-c)
<220>
   <221> misc_feature
   <222> (2801)..(4062)
   <223> Duplication - copy 1
<220>
   <221> misc_feature
   <222> (4063)..(5315)
   <223> Duplication - copy 2
<400> 9
<210> 10
   <211> 35203
   <212> DNA
   <213> Artificial sequence
<220>
   <223> PPE-1-3X-FasC virl construct (lacking repeats)
<220>
   <221> misc_feature
   <222> (460)..(1437)
   <223> A modified murine pre-proendothelin-1 promoter (PPE-1-3X)
<220>
   <221> misc_feature
   <222> (1202)..(1207)
   <223> Hypoxia response element
<220>
   <221> misc_feature
   <222> (1438)..(1468)
   <223> Linker
<220>
   <221> misc_feature
   <222> (1469)..(2058)
   <223> TNFR portion of the Fas-TNFR-1 chimera (Fas-c)
<220>
   <221> misc_feature
   <222> (2059)..(2569)
   <223> FAS portion of the Fas-TNFR-1 chimera (Fas-c)
<400> 10
   catcatcaat aatatacctt attttggatt gaagccaata tgataatgag ggggtggagt     60
   ttgtgacgtg gcgcggggcg tgggaacggg gcgggtgacg tagtagtgtg gcggaagtgt    120
   gatgttgcaa gtgtggcgga acacatgtaa gcgacggatg tggcaaaagt gacgtttttg    180
   gtgtgcgccg gtgtacacag gaagtgacaa ttttcgcgcg gttttaggcg gatgttgtag    240
   taaatttggg cgtaaccgag taagatttgg ccattttcgc gggaaaactg aataagagga    300
   agtgaaatct gaataatttt gtgttactca tagcgcgtaa tatttgtcta gggccgcggg    360
   gactttgacc gtttacgtgg agactcgccc aggtgttttt ctcaggtgtt ttccgcgttc    420
   cgggtcaaag ttggcgtttt attattatag tcagtacgta cgtgtacttc tgatcggcga    480
   tactagggag ataaggatgt acctgacaaa accacattgt tgttgttatc attattattt    540
   agttttcctt ccttgctaac tcctgacgga atctttctca cctcaaatgc gaagtacttt    600
   agtttagaaa agacttggtg gaaggggtgg tggtggaaaa gtagggtgat cttccaaact    660
   aatctggttc cccgcccgcc ccagtagctg ggattcaaga gcgaagagtg gggatcgtcc    720
   ccttgtttga tcagaaagac ataaaaggaa aatcaagtga acaatgatca gccccacctc    780
   caccccaccc ccctgcgcgc gcacaataca atctatttaa ttgtacttca tacttttcat    840
   tccaatgggg tgactttgct tctggagaaa ctcttgattc ttgaactctg gggctggcag     900
   ctagcctcca gaagcaaagt caccccattg gaatgaaaag tatgaagtac aatgaaaagt 960
   atgaagtact ggctccagaa gcaaagtcac cctccagaag caaagtcacc ccattggaat 1020
   gaaaagtatg aagtacgcta gcaaaagggg aagcgggctg ctgctctctg caggttctgc 1080
   agcggtctct gtctagtggg tgttttcttt ttcttagccc tgcccctgga ttgtcagacg 1140
   gcgggcgtct gcctctgaag ttagccgtga tttcctctag agccgggtct tatctctggc 1200
   tgcacgttgc ctgtgggtga ctaatcacac aataacattg tttagggctg gaataaagtc 1260
   agagctgttt acccccactc tataggggtt caatataaaa aggcggcgga gaactgtccg 1320
   agtcagaagc gttcctgcac cggcgctgag agcctgaccc ggtctgctcc gctgtccttg 1380
   cgcgctgcct cccggctgcc cgcgacgctt tcgccccagt ggaagggcca cttgctgcgg 1440
   ccgctaattc tgcagatcgg gatccggcat gggcctctcc accgtgcctg acctgctgct 1500
   gccgctggtg ctcctggagc tgttggtggg aatatacccc tcaggggtta ttggactggt 1560
   ccctcaccta ggggacaggg agaagagaga tagtgtgtgt ccccaaggaa aatatatcca 1620
   ccctcaaaat aattcgattt gctgtaccaa gtgccacaaa ggaacctact tgtacaatga 1680
   ctgtccaggc ccggggcagg atacggactg cagggagtgt gagagcggct ccttcaccgc 1740
   ttcagaaaac cacctcagac actgcctcag ctgctccaaa tgccgaaagg aaatgggtca 1800
   ggtggagatc tcttcttgca cagtggaccg ggacaccgtg tgtggctgca ggaagaacca 1860
   gtaccggcat tattggagtg aaaacctttt ccagtgcttc aattgcagcc tctgcctcaa 1920
   tgggaccgtg cacctctcct gccaggagaa acagaacacc gtgtgcacct gccatgcagg 1980
   tttctttcta agagaaaacg agtgtgtctc ctgtagtaac tgtaagaaaa gcctggagtg 2040
   cacgaagttg tgcctaccaa gcttaggatc cagatctaac ttggggtggc tttgtcttct 2100
   tcttttgcca attccactaa ttgtttgggt gaagagaaag gaagtacaga aaacatgcag 2160
   aaagcacaga aaggaaaacc aaggttctca tgaatctcca accttaaatc ctgaaacagt 2220
   ggcaataaat ttatctgatg ttgacttgag taaatatatc accactattg ctggagtcat 2280
   gacactaagt caagttaaag gctttgttcg aaagaatggt gtcaatgaag ccaaaataga 2340
   tgagatcaag aatgacaatg tccaagacac agcagaacag aaagttcaac tgcttcgtaa 2400
   ttggcatcaa cttcatggaa agaaagaagc gtatgacaca ttgattaaag atctcaaaaa 2460
   agccaatctt tgtactcttg cagagaaaat tcagactatc atcctcaagg acattactag 2520
   tgactcagaa aattcaaact tcagaaatga aatccaaagc ttggtctagc tcgagcatgc 2580
   atctaggcgg ccgcatggca gaaattcgcg aattcgctag cgttaacgga tcctctagac 2640
   gagatccgaa cttgtttatt gcagcttata atggttacaa ataaagcaat agcatcacaa 2700
   atttcacaaa taaagcattt ttttcactgc attctagttg tggtttgtcc aaactcatca 2760
   atgtatctta tcatgtctag atctgtactg aaatgtgtgg gcgtggctta agggtgggaa 2820
   agaatatata aggtgggggt cttatgtagt tttgtatctg ttttgcagca gccgccgccg 2880
   ccatgagcac caactcgttt gatggaagca ttgtgagctc atatttgaca acgcgcatgc 2940
   ccccatgggc cggggtgcgt cagaatgtga tgggctccag cattgatggt cgccccgtcc 3000
   tgcccgcaaa ctctactacc ttgacctacg agaccgtgtc tggaacgccg ttggagactg 3060
   cagcctccgc cgccgcttca gccgctgcag ccaccgcccg cgggattgtg actgactttg 3120
   ctttcctgag cccgcttgca agcagtgcag cttcccgttc atccgcccgc gatgacaagt 3180
   tgacggctct tttggcacaa ttggattctt tgacccggga acttaatgtc gtttctcagc 3240
   agctgttgga tctgcgccag caggtttctg ccctgaaggc ttcctcccct cccaatgcgg 3300
   tttaaaacat aaataaaaaa ccagactctg tttggatttg gatcaagcaa gtgtcttgct 3360
   gtctttattt aggggttttg cgcgcgcggt aggcccggga ccagcggtct cggtcgttga 3420
   gggtcctgtg tattttttcc aggacgtggt aaaggtgact ctggatgttc agatacatgg 3480
   gcataagccc gtctctgggg tggaggtagc accactgcag agcttcatgc tgcggggtgg 3540
   tgttgtagat gatccagtcg tagcaggagc gctgggcgtg gtgcctaaaa atgtctttca 3600
   gtagcaagct gattgccagg ggcaggccct tggtgtaagt gtttacaaag cggttaagct 3660
   gggatgggtg catacgtggg gatatgagat gcatcttgga ctgtattttt aggttggcta 3720
   tgttcccagc catatccctc cggggattca tgttgtgcag aaccaccagc acagtgtatc 3780
   cggtgcactt gggaaatttg tcatgtagct tagaaggaaa tgcgtggaag aacttggaga 3840
   cgcccttgtg acctccaaga ttttccatgc attcgtccat aatgatggca atgggcccac 3900
   gggcggcggc ctgggcgaag atatttctgg gatcactaac gtcatagttg tgttccagga 3960
   tgagatcgtc ataggccatt tttacaaagc gcgggcggag ggtgccagac tgcggtataa 4020
   tggttccatc cggcccaggg gcgtagttac cctcacagat ttgcatttcc cacgctttga 4080
   gttcagatgg ggggatcatg tctacctgcg gggcgatgaa gaaaacggtt tccggggtag 4140
   gggagatcag ctgggaagaa agcaggttcc tgagcagctg cgacttaccg cagccggtgg 4200
   gcccgtaaat cacacctatt accggctgca actggtagtt aagagagctg cagctgccgt 4260
   catccctgag caggggggcc acttcgttaa gcatgtccct gactcgcatg ttttccctga 4320
   ccaaatccgc cagaaggcgc tcgccgccca gcgatagcag ttcttgcaag gaagcaaagt 4380
   ttttcaacgg tttgagaccg tccgccgtag gcatgctttt gagcgtttga ccaagcagtt 4440
   ccaggcggtc ccacagctcg gtcacctgct ctacggcatc tcgatccagc atatctcctc 4500
   gtttcgcggg ttggggcggc tttcgctgta cggcagtagt cggtgctcgt ccagacgggc 4560
   cagggtcatg tctttccacg ggcgcagggt cctcgtcagc gtagtctggg tcacggtgaa 4620
   ggggtgcgct ccgggctgcg cgctggccag ggtgcgcttg aggctggtcc tgctggtgct 4680
   gaagcgctgc cggtcttcgc cctgcgcgtc ggccaggtag catttgacca tggtgtcata 4740
   gtccagcccc tccgcggcgt ggcccttggc gcgcagcttg cccttggagg aggcgccgca 4800
   cgaggggcag tgcagacttt tgagggcgta gagcttgggc gcgagaaata ccgattccgg 4860
   ggagtaggca tccgcgccgc aggccccgca gacggtctcg cattccacga gccaggtgag 4920
   ctctggccgt tcggggtcaa aaaccaggtt tcccccatgc tttttgatgc gtttcttacc 4980
   tctggtttcc atgagccggt gtccacgctc ggtgacgaaa aggctgtccg tgtccccgta 5040
   tacagacttg agaggcctgt cctcgagcgg tgttccgcgg tcctcctcgt atagaaactc 5100
   ggaccactct gagacaaagg ctcgcgtcca ggccagcacg aaggaggcta agtgggaggg 5160
   gtagcggtcg ttgtccacta gggggtccac tcgctccagg gtgtgaagac acatgtcgcc 5220
   ctcttcggca tcaaggaagg tgattggttt gtaggtgtag gccacgtgac cgggtgttcc 5280
   tgaagggggg ctataaaagg gggtgggggc gcgttcgtcc tcactctctt ccgcatcgct 5340
   gtctgcgagg gccagctgtt ggggtgagta ctccctctga aaagcgggca tgacttctgc 5400
   gctaagattg tcagtttcca aaaacgagga ggatttgata ttcacctggc ccgcggtgat 5460
   gcctttgagg gtggccgcat ccatctggtc agaaaagaca atctttttgt tgtcaagctt 5520
   ggtggcaaac gacccgtaga gggcgttgga cagcaacttg gcgatggagc gcagggtttg 5580
   gtttttgtcg cgatcggcgc gctccttggc cgcgatgttt agctgcacgt attcgcgcgc 5640
   aacgcaccgc cattcgggaa agacggtggt gcgctcgtcg ggcaccaggt gcacgcgcca 5700
   accgcggttg tgcagggtga caaggtcaac gctggtggct acctctccgc gtaggcgctc 5760
   gttggtccag cagaggcggc cgcccttgcg cgagcagaat ggcggtaggg ggtctagctg 5820
   cgtctcgtcc ggggggtctg cgtccacggt aaagaccccg ggcagcaggc gcgcgtcgaa 5880
   gtagtctatc ttgcatcctt gcaagtctag cgcctgctgc catgcgcggg cggcaagcgc 5940
   gcgctcgtat gggttgagtg gggaccccca tggcatgggg tgggtgagcg cggaggcgta 6000
   catgccgcaa atgtcgtaaa cgtagagggg ctctctgagt attccaagat atgtagggta 6060
   gcatcttcca ccgcggatgc tggcgcgcac gtaatcgtat agttcgtgcg agggagcgag 6120
   gaggtcggga ccgaggttgc tacgggcggg ctgctctgct cggaagacta tctgcctgaa 6180
   gatggcatgt gagttggatg atatggttgg acgctggaag acgttgaagc tggcgtctgt 6240
   gagacctacc gcgtcacgca cgaaggaggc gtaggagtcg cgcagcttgt tgaccagctc 6300
   ggcggtgacc tgcacgtcta gggcgcagta gtccagggtt tccttgatga tgtcatactt 6360
   atcctgtccc ttttttttcc acagctcgcg gttgaggaca aactcttcgc ggtctttcca 6420
   gtactcttgg atcggaaacc cgtcggcctc cgaacggtaa gagcctagca tgtagaactg 6480
   gttgacggcc tggtaggcgc agcatccctt ttctacgggt agcgcgtatg cctgcgcggc 6540
   cttccggagc gaggtgtggg tgagcgcaaa ggtgtccctg accatgactt tgaggtactg 6600
   gtatttgaag tcagtgtcgt cgcatccgcc ctgctcccag agcaaaaagt ccgtgcgctt 6660
   tttggaacgc ggatttggca gggcgaaggt gacatcgttg aagagtatct ttcccgcgcg 6720
   aggcataaag ttgcgtgtga tgcggaaggg tcccggcacc tcggaacggt tgttaattac 6780
   ctgggcggcg agcacgatct cgtcaaagcc gttgatgttg tggcccacaa tgtaaagttc 6840
   caagaagcgc gggatgccct tgatggaagg caatttttta agttcctcgt aggtgagctc 6900
   ttcaggggag ctgagcccgt gctctgaaag ggcccagtct gcaagatgag ggttggaagc 6960
   gacgaatgag ctccacaggt cacgggccat tagcatttgc aggtggtcgc gaaaggtcct 7020
   aaactggcga cctatggcca ttttttctgg ggtgatgcag tagaaggtaa gcgggtcttg 7080
   ttcccagcgg tcccatccaa ggttcgcggc taggtctcgc gcggcagtca ctagaggctc 7140
   atctccgccg aacttcatga ccagcatgaa gggcacgagc tgcttcccaa aggcccccat 7200
   ccaagtatag gtctctacat cgtaggtgac aaagagacgc tcggtgcgag gatgcgagcc 7260
   gatcgggaag aactggatct cccgccacca attggaggag tggctattga tgtggtgaaa 7320
   gtagaagtcc ctgcgacggg ccgaacactc gtgctggctt ttgtaaaaac gtgcgcagta 7380
   ctggcagcgg tgcacgggct gtacatcctg cacgaggttg acctgacgac cgcgcacaag 7440
   gaagcagagt gggaatttga gcccctcgcc tggcgggttt ggctggtggt cttctacttc 7500
   ggctgcttgt ccttgaccgt ctggctgctc gaggggagtt acggtggatc ggaccaccac 7560
   gccgcgcgag cccaaagtcc agatgtccgc gcgcggcggt cggagcttga tgacaacatc 7620
   gcgcagatgg gagctgtcca tggtctggag ctcccgcggc gtcaggtcag gcgggagctc 7680
   ctgcaggttt acctcgcata gacgggtcag ggcgcgggct agatccaggt gatacctaat 7740
   ttccaggggc tggttggtgg cggcgtcgat ggcttgcaag aggccgcatc cccgcggcgc 7800
   gactacggta ccgcgcggcg ggcggtgggc cgcgggggtg tccttggatg atgcatctaa 7860
   aagcggtgac gcgggcgagc ccccggaggt agggggggct ccggacccgc cgggagaggg 7920
   ggcaggggca cgtcggcgcc gcgcgcgggc aggagctggt gctgcgcgcg taggttgctg 7980
   gcgaacgcga cgacgcggcg gttgatctcc tgaatctggc gcctctgcgt gaagacgacg 8040
   ggcccggtga gcttgaacct gaaagagagt tcgacagaat caatttcggt gtcgttgacg 8100
   gcggcctggc gcaaaatctc ctgcacgtct cctgagttgt cttgataggc gatctcggcc 8160
   atgaactgct cgatctcttc ctcctggaga tctccgcgtc cggctcgctc cacggtggcg 8220
   gcgaggtcgt tggaaatgcg ggccatgagc tgcgagaagg cgttgaggcc tccctcgttc 8280
   cagacgcggc tgtagaccac gcccccttcg gcatcgcggg cgcgcatgac cacctgcgcg 8340
   agattgagct ccacgtgccg ggcgaagacg gcgtagtttc gcaggcgctg aaagaggtag 8400
   ttgagggtgg tggcggtgtg ttctgccacg aagaagtaca taacccagcg tcgcaacgtg 8460
   gattcgttga tatcccccaa ggcctcaagg cgctccatgg cctcgtagaa gtccacggcg 8520
   aagttgaaaa actgggagtt gcgcgccgac acggttaact cctcctccag aagacggatg 8580
   agctcggcga cagtgtcgcg cacctcgcgc tcaaaggcta caggggcctc ttcttcttct 8640
   tcaatctcct cttccataag ggcctcccct tcttcttctt ctggcggcgg tgggggaggg 8700
   gggacacggc ggcgacgacg gcgcaccggg aggcggtcga caaagcgctc gatcatctcc 8760
   ccgcggcgac ggcgcatggt ctcggtgacg gcgcggccgt tctcgcgggg gcgcagttgg 8820
   aagacgccgc ccgtcatgtc ccggttatgg gttggcgggg ggctgccatg cggcagggat 8880
   acggcgctaa cgatgcatct caacaattgt tgtgtaggta ctccgccgcc gagggacctg 8940
   agcgagtccg catcgaccgg atcggaaaac ctctcgagaa aggcgtctaa ccagtcacag 9000
   tcgcaaggta ggctgagcac cgtggcgggc ggcagcgggc ggcggtcggg gttgtttctg 9060
   gcggaggtgc tgctgatgat gtaattaaag taggcggtct tgagacggcg gatggtcgac 9120
   agaagcacca tgtccttggg tccggcctgc tgaatgcgca ggcggtcggc catgccccag 9180
   gcttcgtttt gacatcggcg caggtctttg tagtagtctt gcatgagcct ttctaccggc 9240
   acttcttctt ctccttcctc ttgtcctgca tctcttgcat ctatcgctgc ggcggcggcg 9300
   gagtttggcc gtaggtggcg ccctcttcct cccatgcgtg tgaccccgaa gcccctcatc 9360
   ggctgaagca gggctaggtc ggcgacaacg cgctcggcta atatggcctg ctgcacctgc 9420
   gtgagggtag actggaagtc atccatgtcc acaaagcggt ggtatgcgcc cgtgttgatg 9480
   gtgtaagtgc agttggccat aacggaccag ttaacggtct ggtgacccgg ctgcgagagc 9540
   tcggtgtacc tgagacgcga gtaagccctc gagtcaaata cgtagtcgtt gcaagtccgc 9600
   accaggtact ggtatcccac caaaaagtgc ggcggcggct ggcggtagag gggccagcgt 9660
   agggtggccg gggctccggg ggcgagatct tccaacataa ggcgatgata tccgtagatg 9720
   tacctggaca tccaggtgat gccggcggcg gtggtggagg cgcgcggaaa gtcgcggacg 9780
   cggttccaga tgttgcgcag cggcaaaaag tgctccatgg tcgggacgct ctggccggtc 9840
   aggcgcgcgc aatcgttgac gctctagacc gtgcaaaagg agagcctgta agcgggcact 9900
   cttccgtggt ctggtggata aattcgcaag ggtatcatgg cggacgaccg gggttcgagc 9960
   cccgtatccg gccgtccgcc gtgatccatg cggttaccgc ccgcgtgtcg aacccaggtg 10020
   tgcgacgtca gacaacgggg gagtgctcct tttggcttcc ttccaggcgc ggcggctgct 10080
   gcgctagctt ttttggccac tggccgcgcg cagcgtaagc ggttaggctg gaaagcgaaa 10140
   gcattaagtg gctcgctccc tgtagccgga gggttatttt ccaagggttg agtcgcggga 10200
   cccccggttc gagtctcgga ccggccggac tgcggcgaac gggggtttgc ctccccgtca 10260
   tgcaagaccc cgcttgcaaa ttcctccgga aacagggacg agcccctttt ttgcttttcc 10320
   cagatgcatc cggtgctgcg gcagatgcgc ccccctcctc agcagcggca agagcaagag 10380
   cagcggcaga catgcagggc accctcccct cctcctaccg cgtcaggagg ggcgacatcc 10440
   gcggttgacg cggcagcaga tggtgattac gaacccccgc ggcgccgggc ccggcactac 10500
   ctggacttgg aggagggcga gggcctggcg cggctaggag cgccctctcc tgagcggcac 10560
   ccaagggtgc agctgaagcg tgatacgcgt gaggcgtacg tgccgcggca gaacctgttt 10620
   cgcgaccgcg agggagagga gcccgaggag atgcgggatc gaaagttcca cgcagggcgc 10680
   gagctgcggc atggcctgaa tcgcgagcgg ttgctgcgcg aggaggactt tgagcccgac 10740
   gcgcgaaccg ggattagtcc cgcgcgcgca cacgtggcgg ccgccgacct ggtaaccgca 10800
   tacgagcaga cggtgaacca ggagattaac tttcaaaaaa gctttaacaa ccacgtgcgt 10860
   acgcttgtgg cgcgcgagga ggtggctata ggactgatgc atctgtggga ctttgtaagc 10920
   gcgctggagc aaaacccaaa tagcaagccg ctcatggcgc agctgttcct tatagtgcag 10980
   cacagcaggg acaacgaggc attcagggat gcgctgctaa acatagtaga gcccgagggc 11040
   cgctggctgc tcgatttgat aaacatcctg cagagcatag tggtgcagga gcgcagcttg 11100
   agcctggctg acaaggtggc cgccatcaac tattccatgc ttagcctggg caagttttac 11160
   gcccgcaaga tataccatac cccttacgtt cccatagaca aggaggtaaa gatcgagggg 11220
   ttctacatgc gcatggcgct gaaggtgctt accttgagcg acgacctggg cgtttatcgc 11280
   aacgagcgca tccacaaggc cgtgagcgtg agccggcggc gcgagctcag cgaccgcgag 11340
   ctgatgcaca gcctgcaaag ggccctggct ggcacgggca gcggcgatag agaggccgag 11400
   tcctactttg acgcgggcgc tgacctgcgc tgggccccaa gccgacgcgc cctggaggca 11460
   gctggggccg gacctgggct ggcggtggca cccgcgcgcg ctggcaacgt cggcggcgtg 11520
   gaggaatatg acgaggacga tgagtacgag ccagaggacg gcgagtacta agcggtgatg 11580
   tttctgatca gatgatgcaa gacgcaacgg acccggcggt gcgggcggcg ctgcagagcc 11640
   agccgtccgg ccttaactcc acggacgact ggcgccaggt catggaccgc atcatgtcgc 11700
   tgactgcgcg caatcctgac gcgttccggc agcagccgca ggccaaccgg ctctccgcaa 11760
   ttctggaagc ggtggtcccg gcgcgcgcaa accccacgca cgagaaggtg ctggcgatcg 11820
   taaacgcgct ggccgaaaac agggccatcc ggcccgacga ggccggcctg gtctacgacg 11880
   cgctgcttca gcgcgtggct cgttacaaca gcggcaacgt gcagaccaac ctggaccggc 11940
   tggtggggga tgtgcgcgag gccgtggcgc agcgtgagcg cgcgcagcag cagggcaacc 12000
   tgggctccat ggttgcacta aacgccttcc tgagtacaca gcccgccaac gtgccgcggg 12060
   gacaggagga ctacaccaac tttgtgagcg cactgcggct aatggtgact gagacaccgc 12120
   aaagtgaggt gtaccagtct gggccagact attttttcca gaccagtaga caaggcctgc 12180
   agaccgtaaa cctgagccag gctttcaaaa acttgcaggg gctgtggggg gtgcgggctc 12240
   ccacaggcga ccgcgcgacc gtgtctagct tgctgacgcc caactcgcgc ctgttgctgc 12300
   tgctaatagc gcccttcacg gacagtggca gcgtgtcccg ggacacatac ctaggtcact 12360
   tgctgacact gtaccgcgag gccataggtc aggcgcatgt ggacgagcat actttccagg 12420
   agattacaag tgtcagccgc gcgctggggc aggaggacac gggcagcctg gaggcaaccc 12480
   taaactacct gctgaccaac cggcggcaga agatcccctc gttgcacagt ttaaacagcg 12540
   aggaggagcg cattttgcgc tacgtgcagc agagcgtgag ccttaacctg atgcgcgacg 12600
   gggtaacgcc cagcgtggcg ctggacatga ccgcgcgcaa catggaaccg ggcatgtatg 12660
   cctcaaaccg gccgtttatc aaccgcctaa tggactactt gcatcgcgcg gccgccgtga 12720
   accccgagta tttcaccaat gccatcttga acccgcactg gctaccgccc cctggtttct 12780
   acaccggggg attcgaggtg cccgagggta acgatggatt cctctgggac gacatagacg 12840
   acagcgtgtt ttccccgcaa ccgcagaccc tgctagagtt gcaacagcgc gagcaggcag 12900
   aggcggcgct gcgaaaggaa agcttccgca ggccaagcag cttgtccgat ctaggcgctg 12960
   cggccccgcg gtcagatgct agtagcccat ttccaagctt gatagggtct cttaccagca 13020
   ctcgcaccac ccgcccgcgc ctgctgggcg aggaggagta cctaaacaac tcgctgctgc 13080
   agccgcagcg cgaaaaaaac ctgcctccgg catttcccaa caacgggata gagagcctag 13140
   tggacaagat gagtagatgg aagacgtacg cgcaggagca cagggacgtg ccaggcccgc 13200
   gcccgcccac ccgtcgtcaa aggcacgacc gtcagcgggg tctggtgtgg gaggacgatg 13260
   actcggcaga cgacagcagc gtcctggatt tgggagggag tggcaacccg tttgcgcacc 13320
   ttcgccccag gctggggaga atgttttaaa aaaaaaaaaa gcatgatgca aaataaaaaa 13380
   ctcaccaagg ccatggcacc gagcgttggt ttcttgtatt tccccttagt atgcggcgcg 13440
   cggcgatgta tgaggaaggt cctcctccct cctacgagag tgtggtgagc gcggcgccag 13500
   tggcggcggc gctgggttct cccttcgatg ctcccctgga cccgccgttt gtgcctccgc 13560
   ggtacctgcg gcctaccggg gggagaaaca gcatccgtta ctctgagttg gcacccctat 13620
   tcgacaccac ccgtgtgtac ctggtggaca acaagtcaac ggatgtggca tccctgaact 13680
   accagaacga ccacagcaac tttctgacca cggtcattca aaacaatgac tacagcccgg 13740
   gggaggcaag cacacagacc atcaatcttg acgaccggtc gcactggggc ggcgacctga 13800
   aaaccatcct gcataccaac atgccaaatg tgaacgagtt catgtttacc aataagttta 13860
   aggcgcgggt gatggtgtcg cgcttgccta ctaaggacaa tcaggtggag ctgaaatacg 13920
   agtgggtgga gttcacgctg cccgagggca actactccga gaccatgacc atagacctta 13980
   tgaacaacgc gatcgtggag cactacttga aagtgggcag acagaacggg gttctggaaa 14040
   gcgacatcgg ggtaaagttt gacacccgca acttcagact ggggtttgac cccgtcactg 14100
   gtcttgtcat gcctggggta tatacaaacg aagccttcca tccagacatc attttgctgc 14160
   caggatgcgg ggtggacttc acccacagcc gcctgagcaa cttgttgggc atccgcaagc 14220
   ggcaaccctt ccaggagggc tttaggatca cctacgatga tctggagggt ggtaacattc 14280
   ccgcactgtt ggatgtggac gcctaccagg cgagcttgaa agatgacacc gaacagggcg 14340
   ggggtggcgc aggcggcagc aacagcagtg gcagcggcgc ggaagagaac tccaacgcgg 14400
   cagccgcggc aatgcagccg gtggaggaca tgaacgatca tgccattcgc ggcgacacct 14460
   ttgccacacg ggctgaggag aagcgcgctg aggccgaagc agcggccgaa gctgccgccc 14520
   ccgctgcgca acccgaggtc gagaagcctc agaagaaacc ggtgatcaaa cccctgacag 14580
   aggacagcaa gaaacgcagt tacaacctaa taagcaatga cagcaccttc acccagtacc 14640
   gcagctggta ccttgcatac aactacggcg accctcagac cggaatccgc tcatggaccc 14700
   tgctttgcac tcctgacgta acctgcggct cggagcaggt ctactggtcg ttgccagaca 14760
   tgatgcaaga ccccgtgacc ttccgctcca cgcgccagat cagcaacttt ccggtggtgg 14820
   gcgccgagct gttgcccgtg cactccaaga gcttctacaa cgaccaggcc gtctactccc 14880
   aactcatccg ccagtttacc tctctgaccc acgtgttcaa tcgctttccc gagaaccaga 14940
   ttttggcgcg cccgccagcc cccaccatca ccaccgtcag tgaaaacgtt cctgctctca 15000
   cagatcacgg gacgctaccg ctgcgcaaca gcatcggagg agtccagcga gtgaccatta 15060
   ctgacgccag acgccgcacc tgcccctacg tttacaaggc cctgggcata gtctcgccgc 15120
   gcgtcctatc gagccgcact ttttgagcaa gcatgtccat ccttatatcg cccagcaata 15180
   acacaggctg gggcctgcgc ttcccaagca agatgtttgg cggggccaag aagcgctccg 15240
   accaacaccc agtgcgcgtg cgcgggcact accgcgcgcc ctggggcgcg cacaaacgcg 15300
   gccgcactgg gcgcaccacc gtcgatgacg ccatcgacgc ggtggtggag gaggcgcgca 15360
   actacacgcc cacgccgcca ccagtgtcca cagtggacgc ggccattcag accgtggtgc 15420
   gcggagcccg gcgctatgct aaaatgaaga gacggcggag gcgcgtagca cgtcgccacc 15480
   gccgccgacc cggcactgcc gcccaacgcg cggcggcggc cctgcttaac cgcgcacgtc 15540
   gcaccggccg acgggcggcc atgcgggccg ctcgaaggct ggccgcgggt attgtcactg 15600
   tgccccccag gtccaggcga cgagcggccg ccgcagcagc cgcggccatt agtgctatga 15660
   ctcagggtcg caggggcaac gtgtattggg tgcgcgactc ggttagcggc ctgcgcgtgc 15720
   ccgtgcgcac ccgccccccg cgcaactaga ttgcaagaaa aaactactta gactcgtact 15780
   gttgtatgta tccagcggcg gcggcgcgca acgaagctat gtccaagcgc aaaatcaaag 15840
   aagagatgct ccaggtcatc gcgccggaga tctatggccc cccgaagaag gaagagcagg 15900
   attacaagcc ccgaaagcta aagcgggtca aaaagaaaaa gaaagatgat gatgatgaac 15960
   ttgacgacga ggtggaactg ctgcacgcta ccgcgcccag gcgacgggta cagtggaaag 16020
   gtcgacgcgt aaaacgtgtt ttgcgacccg gcaccaccgt agtctttacg cccggtgagc 16080
   gctccacccg cacctacaag cgcgtgtatg atgaggtgta cggcgacgag gacctgcttg 16140
   agcaggccaa cgagcgcctc ggggagtttg cctacggaaa gcggcataag gacatgctgg 16200
   cgttgccgct ggacgagggc aacccaacac ctagcctaaa gcccgtaaca ctgcagcagg 16260
   tgctgcccgc gcttgcaccg tccgaagaaa agcgcggcct aaagcgcgag tctggtgact 16320
   tggcacccac cgtgcagctg atggtaccca agcgccagcg actggaagat gtcttggaaa 16380
   aaatgaccgt ggaacctggg ctggagcccg aggtccgcgt gcggccaatc aagcaggtgg 16440
   cgccgggact gggcgtgcag accgtggacg ttcagatacc cactaccagt agcaccagta 16500
   ttgccaccgc cacagagggc atggagacac aaacgtcccc ggttgcctca gcggtggcgg 16560
   atgccgcggt gcaggcggtc gctgcggccg cgtccaagac ctctacggag gtgcaaacgg 16620
   acccgtggat gtttcgcgtt tcagcccccc ggcgcccgcg ccgttcgagg aagtacggcg 16680
   ccgccagcgc gctactgccc gaatatgccc tacatccttc cattgcgcct acccccggct 16740
   atcgtggcta cacctaccgc cccagaagac gagcaactac ccgacgccga accaccactg 16800
   gaacccgccg ccgccgtcgc cgtcgccagc ccgtgctggc cccgatttcc gtgcgcaggg 16860
   tggctcgcga aggaggcagg accctggtgc tgccaacagc gcgctaccac cccagcatcg 16920
   tttaaaagcc ggtctttgtg gttcttgcag atatggccct cacctgccgc ctccgtttcc 16980
   cggtgccggg attccgagga agaatgcacc gtaggagggg catggccggc cacggcctga 17040
   cgggcggcat gcgtcgtgcg caccaccggc ggcggcgcgc gtcgcaccgt cgcatgcgcg 17100
   gcggtatcct gcccctcctt attccactga tcgccgcggc gattggcgcc gtgcccggaa 17160
   ttgcatccgt ggccttgcag gcgcagagac actgattaaa aacaagttgc atgtggaaaa 17220
   atcaaaataa aaagtctgga ctctcacgct cgcttggtcc tgtaactatt ttgtagaatg 17280
   gaagacatca actttgcgtc tctggccccg cgacacggct cgcgcccgtt catgggaaac 17340
   tggcaagata tcggcaccag caatatgagc ggtggcgcct tcagctgggg ctcgctgtgg 17400
   agcggcatta aaaatttcgg ttccaccgtt aagaactatg gcagcaaggc ctggaacagc 17460
   agcacaggcc agatgctgag ggataagttg aaagagcaaa atttccaaca aaaggtggta 17520
   gatggcctgg cctctggcat tagcggggtg gtggacctgg ccaaccaggc agtgcaaaat 17580
   aagattaaca gtaagcttga tccccgccct cccgtagagg agcctccacc ggccgtggag 17640
   acagtgtctc cagaggggcg tggcgaaaag cgtccgcgcc ccgacaggga agaaactctg 17700
   gtgacgcaaa tagacgagcc tccctcgtac gaggaggcac taaagcaagg cctgcccacc 17760
   acccgtccca tcgcgcccat ggctaccgga gtgctgggcc agcacacacc cgtaacgctg 17820
   gacctgcctc cccccgccga cacccagcag aaacctgtgc tgccaggccc gaccgccgtt 17880
   gttgtaaccc gtcctagccg cgcgtccctg cgccgcgccg ccagcggtcc gcgatcgttg 17940
   cggcccgtag ccagtggcaa ctggcaaagc acactgaaca gcatcgtggg tctgggggtg 18000
   caatccctga agcgccgacg atgcttctga tagctaacgt gtcgtatgtg tgtcatgtat 18060
   gcgtccatgt cgccgccaga ggagctgctg agccgccgcg cgcccgcttt ccaagatggc 18120
   taccccttcg atgatgccgc agtggtctta catgcacatc tcgggccagg acgcctcgga 18180
   gtacctgagc cccgggctgg tgcagtttgc ccgcgccacc gagacgtact tcagcctgaa 18240
   taacaagttt agaaacccca cggtggcgcc tacgcacgac gtgaccacag accggtccca 18300
   gcgtttgacg ctgcggttca tccctgtgga ccgtgaggat actgcgtact cgtacaaggc 18360
   gcggttcacc ctagctgtgg gtgataaccg tgtgctggac atggcttcca cgtactttga 18420
   catccgcggc gtgctggaca ggggccctac ttttaagccc tactctggca ctgcctacaa 18480
   cgccctggct cccaagggtg ccccaaatcc ttgcgaatgg gatgaagctg ctactgctct 18540
   tgaaataaac ctagaagaag aggacgatga caacgaagac gaagtagacg agcaagctga 18600
   gcagcaaaaa actcacgtat ttgggcaggc gccttattct ggtataaata ttacaaagga 18660
   gggtattcaa ataggtgtcg aaggtcaaac acctaaatat gccgataaaa catttcaacc 18720
   tgaacctcaa ataggagaat ctcagtggta cgaaacagaa attaatcatg cagctgggag 18780
   agtcctaaaa aagactaccc caatgaaacc atgttacggt tcatatgcaa aacccacaaa 18840
   tgaaaatgga gggcaaggca ttcttgtaaa gcaacaaaat ggaaagctag aaagtcaagt 18900
   ggaaatgcaa tttttctcaa ctactgaggc agccgcaggc aatggtgata acttgactcc 18960
   taaagtggta ttgtacagtg aagatgtaga tatagaaacc ccagacactc atatttctta 19020
   catgcccact attaaggaag gtaactcacg agaactaatg ggccaacaat ctatgcccaa 19080
   caggcctaat tacattgctt ttagggacaa ttttattggt ctaatgtatt acaacagcac 19140
   gggtaatatg ggtgttctgg cgggccaagc atcgcagttg aatgctgttg tagatttgca 19200
   agacagaaac acagagcttt cataccagct tttgcttgat tccattggtg atagaaccag 19260
   gtacttttct atgtggaatc aggctgttga cagctatgat ccagatgtta gaattattga 19320
   aaatcatgga actgaagatg aacttccaaa ttactgcttt ccactgggag gtgtgattaa 19380
   tacagagact cttaccaagg taaaacctaa aacaggtcag gaaaatggat gggaaaaaga 19440
   tgctacagaa ttttcagata aaaatgaaat aagagttgga aataattttg ccatggaaat 19500
   caatctaaat gccaacctgt ggagaaattt cctgtactcc aacatagcgc tgtatttgcc 19560
   cgacaagcta aagtacagtc cttccaacgt aaaaatttct gataacccaa acacctacga 19620
   ctacatgaac aagcgagtgg tggctcccgg gctagtggac tgctacatta accttggagc 19680
   acgctggtcc cttgactata tggacaacgt caacccattt aaccaccacc gcaatgctgg 19740
   cctgcgctac cgctcaatgt tgctgggcaa tggtcgctat gtgcccttcc acatccaggt 19800
   gcctcagaag ttctttgcca ttaaaaacct ccttctcctg ccgggctcat acacctacga 19860
   gtggaacttc aggaaggatg ttaacatggt tctgcagagc tccctaggaa atgacctaag 19920
   ggttgacgga gccagcatta agtttgatag catttgcctt tacgccacct tcttccccat 19980
   ggcccacaac accgcctcca cgcttgaggc catgcttaga aacgacacca acgaccagtc 20040
   ctttaacgac tatctctccg ccgccaacat gctctaccct atacccgcca acgctaccaa 20100
   cgtgcccata tccatcccct cccgcaactg ggcggctttc cgcggctggg ccttcacgcg 20160
   ccttaagact aaggaaaccc catcactggg ctcgggctac gacccttatt acacctactc 20220
   tggctctata ccctacctag atggaacctt ttacctcaac cacaccttta agaaggtggc 20280
   cattaccttt gactcttctg tcagctggcc tggcaatgac cgcctgctta cccccaacga 20340
   gtttgaaatt aagcgctcag ttgacgggga gggttacaac gttgcccagt gtaacatgac 20400
   caaagactgg ttcctggtac aaatgctagc taactataac attggctacc agggcttcta 20460
   tatcccagag agctacaagg accgcatgta ctccttcttt agaaacttcc agcccatgag 20520
   ccgtcaggtg gtggatgata ctaaatacaa ggactaccaa caggtgggca tcctacacca 20580
   acacaacaac tctggatttg ttggctacct tgcccccacc atgcgcgaag gacaggccta 20640
   ccctgctaac ttcccctatc cgcttatagg caagaccgca gttgacagca ttacccagaa 20700
   aaagtttctt tgcgatcgca ccctttggcg catcccattc tccagtaact ttatgtccat 20760
   gggcgcactc acagacctgg gccaaaacct tctctacgcc aactccgccc acgcgctaga 20820
   catgactttt gaggtggatc ccatggacga gcccaccctt ctttatgttt tgtttgaagt 20880
   ctttgacgtg gtccgtgtgc accagccgca ccgcggcgtc atcgaaaccg tgtacctgcg 20940
   cacgcccttc tcggccggca acgccacaac ataaagaagc aagcaacatc aacaacagct 21000
   gccgccatgg gctccagtga gcaggaactg aaagccattg tcaaagatct tggttgtggg 21060
   ccatattttt tgggcaccta tgacaagcgc tttccaggct ttgtttctcc acacaagctc 21120
   gcctgcgcca tagtcaatac ggccggtcgc gagactgggg gcgtacactg gatggccttt 21180
   gcctggaacc cgcactcaaa aacatgctac ctctttgagc cctttggctt ttctgaccag 21240
   cgactcaagc aggtttacca gtttgagtac gagtcactcc tgcgccgtag cgccattgct 21300
   tcttcccccg accgctgtat aacgctggaa aagtccaccc aaagcgtaca ggggcccaac 21360
   tcggccgcct gtggactatt ctgctgcatg tttctccacg cctttgccaa ctggccccaa 21420
   actcccatgg atcacaaccc caccatgaac cttattaccg gggtacccaa ctccatgctc 21480
   aacagtcccc aggtacagcc caccctgcgt cgcaaccagg aacagctcta cagcttcctg 21540
   gagcgccact cgccctactt ccgcagccac agtgcgcaga ttaggagcgc cacttctttt 21600
   tgtcacttga aaaacatgta aaaataatgt actagagaca ctttcaataa aggcaaatgc 21660
   ttttatttgt acactctcgg gtgattattt acccccaccc ttgccgtctg cgccgtttaa 21720
   aaatcaaagg ggttctgccg cgcatcgcta tgcgccactg gcagggacac gttgcgatac 21780
   tggtgtttag tgctccactt aaactcaggc acaaccatcc gcggcagctc ggtgaagttt 21840
   tcactccaca ggctgcgcac catcaccaac gcgtttagca ggtcgggcgc cgatatcttg 21900
   aagtcgcagt tggggcctcc gccctgcgcg cgcgagttgc gatacacagg gttgcagcac 21960
   tggaacacta tcagcgccgg gtggtgcacg ctggccagca cgctcttgtc ggagatcaga 22020
   tccgcgtcca ggtcctccgc gttgctcagg gcgaacggag tcaactttgg tagctgcctt 22080
   cccaaaaagg gcgcgtgccc aggctttgag ttgcactcgc accgtagtgg catcaaaagg 22140
   tgaccgtgcc cggtctgggc gttaggatac agcgcctgca taaaagcctt gatctgctta 22200
   aaagccacct gagcctttgc gccttcagag aagaacatgc cgcaagactt gccggaaaac 22260
   tgattggccg gacaggccgc gtcgtgcacg cagcaccttg cgtcggtgtt ggagatctgc 22320
   accacatttc ggccccaccg gttcttcacg atcttggcct tgctagactg ctccttcagc 22380
   gcgcgctgcc cgttttcgct cgtcacatcc atttcaatca cgtgctcctt atttatcata 22440
   atgcttccgt gtagacactt aagctcgcct tcgatctcag cgcagcggtg cagccacaac 22500
   gcgcagcccg tgggctcgtg atgcttgtag gtcacctctg caaacgactg caggtacgcc 22560
   tgcaggaatc gccccatcat cgtcacaaag gtcttgttgc tggtgaaggt cagctgcaac 22620
   ccgcggtgct cctcgttcag ccaggtcttg catacggccg ccagagcttc cacttggtca 22680
   ggcagtagtt tgaagttcgc ctttagatcg ttatccacgt ggtacttgtc catcagcgcg 22740
   cgcgcagcct ccatgccctt ctcccacgca gacacgatcg gcacactcag cgggttcatc 22800
   accgtaattt cactttccgc ttcgctgggc tcttcctctt cctcttgcgt ccgcatacca 22860
   cgcgccactg ggtcgtcttc attcagccgc cgcactgtgc gcttacctcc tttgccatgc 22920
   ttgattagca ccggtgggtt gctgaaaccc accatttgta gcgccacatc ttctctttct 22980
   tcctcgctgt ccacgattac ctctggtgat ggcgggcgct cgggcttggg agaagggcgc 23040
   ttctttttct tcttgggcgc aatggccaaa tccgccgccg aggtcgatgg ccgcgggctg 23100
   ggtgtgcgcg gcaccagcgc gtcttgtgat gagtcttcct cgtcctcgga ctcgatacgc 23160
   cgcctcatcc gcttttttgg gggcgcccgg ggaggcggcg gcgacgggga cggggacgac 23220
   acgtcctcca tggttggggg acgtcgcgcc gcaccgcgtc cgcgctcggg ggtggtttcg 23280
   cgctgctcct cttcccgact ggccatttcc ttctcctata ggcagaaaaa gatcatggag 23340
   tcagtcgaga agaaggacag cctaaccgcc ccctctgagt tcgccaccac cgcctccacc 23400
   gatgccgcca acgcgcctac caccttcccc gtcgaggcac ccccgcttga ggaggaggaa 23460
   gtgattatcg agcaggaccc aggttttgta agcgaagacg acgaggaccg ctcagtacca 23520
   acagaggata aaaagcaaga ccaggacaac gcagaggcaa acgaggaaca agtcgggcgg    23580
   ggggacgaaa ggcatggcga ctacctagat gtgggagacg acgtgctgtt gaagcatctg    23640
   cagcgccagt gcgccattat ctgcgacgcg ttgcaagagc gcagcgatgt gcccctcgcc 23700
   atagcggatg tcagccttgc ctacgaacgc cacctattct caccgcgcgt accccccaaa 23760
   cgccaagaaa acggcacatg cgagcccaac ccgcgcctca acttctaccc cgtatttgcc 23820
   gtgccagagg tgcttgccac ctatcacatc tttttccaaa actgcaagat acccctatcc 23880
   tgccgtgcca accgcagccg agcggacaag cagctggcct tgcggcaggg cgctgtcata 23940
   cctgatatcg cctcgctcaa cgaagtgcca aaaatctttg agggtcttgg acgcgacgag 24000
   aagcgcgcgg caaacgctct gcaacaggaa aacagcgaaa atgaaagtca ctctggagtg 24060
   ttggtggaac tcgagggtga caacgcgcgc ctagccgtac taaaacgcag catcgaggtc 24120
   acccactttg cctacccggc acttaaccta ccccccaagg tcatgagcac agtcatgagt 24180
   gagctgatcg tgcgccgtgc gcagcccctg gagagggatg caaatttgca agaacaaaca 24240
   gaggagggcc tacccgcagt tggcgacgag cagctagcgc gctggcttca aacgcgcgag 24300
   cctgccgact tggaggagcg acgcaaacta atgatggccg cagtgctcgt taccgtggag 24360
   cttgagtgca tgcagcggtt ctttgctgac ccggagatgc agcgcaagct agaggaaaca 24420
   ttgcactaca cctttcgaca gggctacgta cgccaggcct gcaagatctc caacgtggag 24480
   ctctgcaacc tggtctccta ccttggaatt ttgcacgaaa accgccttgg gcaaaacgtg 24540
   cttcattcca cgctcaaggg cgaggcgcgc cgcgactacg tccgcgactg cgtttactta 24600
   tttctatgct acacctggca gacggccatg ggcgtttggc agcagtgctt ggaggagtgc 24660
   aacctcaagg agctgcagaa actgctaaag caaaacttga aggacctatg gacggccttc 24720
   aacgagcgct ccgtggccgc gcacctggcg gacatcattt tccccgaacg cctgcttaaa 24780
   accctgcaac agggtctgcc agacttcacc agtcaaagca tgttgcagaa ctttaggaac 24840
   tttatcctag agcgctcagg aatcttgccc gccacctgct gtgcacttcc tagcgacttt 24900
   gtgcccatta agtaccgcga atgccctccg ccgctttggg gccactgcta ccttctgcag 24960
   ctagccaact accttgccta ccactctgac ataatggaag acgtgagcgg tgacggtcta 25020
   ctggagtgtc actgtcgctg caacctatgc accccgcacc gctccctggt ttgcaattcg 25080
   cagctgctta acgaaagtca aattatcggt acctttgagc tgcagggtcc ctcgcctgac 25140
   gaaaagtccg cggctccggg gttgaaactc actccggggc tgtggacgtc ggcttacctt 25200
   cgcaaatttg tacctgagga ctaccacgcc cacgagatta ggttctacga agaccaatcc 25260
   cgcccgccta atgcggagct taccgcctgc gtcattaccc agggccacat tcttggccaa 25320
   ttgcaagcca tcaacaaagc ccgccaagag tttctgctac gaaagggacg gggggtttac 25380
   ttggaccccc agtccggcga ggagctcaac ccaatccccc cgccgccgca gccctatcag 25440
   cagcagccgc gggcccttgc ttcccaggat ggcacccaaa aagaagctgc agctgccgcc 25500
   gccacccacg gacgaggagg aatactggga cagtcaggca gaggaggttt tggacgagga 25560
   ggaggaggac atgatggaag actgggagag cctagacgag gaagcttccg aggtcgaaga 25620
   ggtgtcagac gaaacaccgt caccctcggt cgcattcccc tcgccggcgc cccagaaatc 25680
   ggcaaccggt tccagcatgg ctacaacctc cgctcctcag gcgccgccgg cactgcccgt 25740
   tcgccgaccc aaccgtagat gggacaccac tggaaccagg gccggtaagt ccaagcagcc 25800
   gccgccgtta gcccaagagc aacaacagcg ccaaggctac cgctcatggc gcgggcacaa 25860
   gaacgccata gttgcttgct tgcaagactg tgggggcaac atctccttcg cccgccgctt 25920
   tcttctctac catcacggcg tggccttccc ccgtaacatc ctgcattact accgtcatct 25980
   ctacagccca tactgcaccg gcggcagcgg cagcaacagc agcggccaca cagaagcaaa 26040
   ggcgaccgga tagcaagact ctgacaaagc ccaagaaatc cacagcggcg gcagcagcag 26100
   gaggaggagc gctgcgtctg gcgcccaacg aacccgtatc gacccgcgag cttagaaaca 26160
   ggatttttcc cactctgtat gctatatttc aacagagcag gggccaagaa caagagctga 26220
   aaataaaaaa caggtctctg cgatccctca cccgcagctg cctgtatcac aaaagcgaag 26280
   atcagcttcg gcgcacgctg gaagacgcgg aggctctctt cagtaaatac tgcgcgctga 26340
   ctcttaagga ctagtttcgc gccctttctc aaatttaagc gcgaaaacta cgtcatctcc 26400
   agcggccaca cccggcgcca gcacctgttg tcagcgccat tatgagcaag gaaattccca 26460
   cgccctacat gtggagttac cagccacaaa tgggacttgc ggctggagct gcccaagact 26520
   actcaacccg aataaactac atgagcgcgg gaccccacat gatatcccgg gtcaacggaa 26580
   tacgcgccca ccgaaaccga attctcctgg aacaggcggc tattaccacc acacctcgta 26640
   ataaccttaa tccccgtagt tggcccgctg ccctggtgta ccaggaaagt cccgctccca 26700
   ccactgtggt acttcccaga gacgcccagg ccgaagttca gatgactaac tcaggggcgc 26760
   agcttgcggg cggctttcgt cacagggtgc ggtcgcccgg gcagggtata actcacctga 26820
   caatcagagg gcgaggtatt cagctcaacg acgagtcggt gagctcctcg cttggtctcc 26880
   gtccggacgg gacatttcag atcggcggcg ccggccgctc ttcattcacg cctcgtcagg 26940
   caatcctaac tctgcagacc tcgtcctctg agccgcgctc tggaggcatt ggaactctgc 27000
   aatttattga ggagtttgtg ccatcggtct actttaaccc cttctcggga cctcccggcc 27060
   actatccgga tcaatttatt cctaactttg acgcggtaaa ggactcggcg gacggctacg 27120
   actgaatgtt aagtggagag gcagagcaac tgcgcctgaa acacctggtc cactgtcgcc 27180
   gccacaagtg ctttgcccgc gactccggtg agttttgcta ctttgaattg cccgaggatc 27240
   atatcgaggg cccggcgcac ggcgtccggc ttaccgccca gggagagctt gcccgtagcc 27300
   tgattcggga gtttacccag cgccccctgc tagttgagcg ggacagggga ccctgtgttc 27360
   tcactgtgat ttgcaactgt cctaaccctg gattacatca agatctttgt tgccatctct 27420
   gtgctgagta taataaatac agaaattaaa atatactggg gctcctatcg ccatcctgta 27480
   aacgccaccg tcttcacccg cccaagcaaa ccaaggcgaa ccttacctgg tacttttaac 27540
   atctctccct ctgtgattta caacagtttc aacccagacg gagtgagtct acgagagaac 27600
   ctctccgagc tcagctactc catcagaaaa aacaccaccc tccttacctg ccgggaacgt 27660
   acgagtgcgt caccggccgc tgcaccacac ctaccgcctg accgtaaacc agactttttc 27720
   cggacagacc tcaataactc tgtttaccag aacaggaggt gagcttagaa aacccttagg 27780
   gtattaggcc aaaggcgcag ctactgtggg gtttatgaac aattcaagca actctacggg 27840
   ctattctaat tcaggtttct ctagaatcgg ggttggggtt attctctgtc ttgtgattct 27900
   ctttattctt atactaacgc ttctctgcct aaggctcgcc gcctgctgtg tgcacatttg 27960
   catttattgt cagcttttta aacgctgggg tcgccaccca agatgattag gtacataatc 28020
   ctaggtttac tcacccttgc gtcagcccac ggtaccaccc aaaaggtgga ttttaaggag 28080
   ccagcctgta atgttacatt cgcagctgaa gctaatgagt gcaccactct tataaaatgc 28140
   accacagaac atgaaaagct gcttattcgc cacaaaaaca aaattggcaa gtatgctgtt 28200
   tatgctattt ggcagccagg tgacactaca gagtataatg ttacagtttt ccagggtaaa 28260
   agtcataaaa cttttatgta tacttttcca ttttatgaaa tgtgcgacat taccatgtac 28320
   atgagcaaac agtataagtt gtggccccca caaaattgtg tggaaaacac tggcactttc 28380
   tgctgcactg ctatgctaat tacagtgctc gctttggtct gtaccctact ctatattaaa 28440
   tacaaaagca gacgcagctt tattgaggaa aagaaaatgc cttaatttac taagttacaa 28500
   agctaatgtc accactaact gctttactcg ctgcttgcaa aacaaattca aaaagttagc 28560
   attataatta gaataggatt taaacccccc ggtcatttcc tgctcaatac cattcccctg 28620
   aacaattgac tctatgtggg atatgctcca gcgctacaac cttgaagtca ggcttcctgg 28680
   atgtcagcat ctgactttgg ccagcacctg tcccgcggat ttgttccagt ccaactacag 28740
   cgacccaccc taacagagat gaccaacaca accaacgcgg ccgccgctac cggacttaca 28800
   tctaccacaa atacacccca agtttctgcc tttgtcaata actgggataa cttgggcatg 28860
   tggtggttct ccatagcgct tatgtttgta tgccttatta ttatgtggct catctgctgc 28920
   ctaaagcgca aacgcgcccg accacccatc tatagtccca tcattgtgct acacccaaac 28980
   aatgatggaa tccatagatt ggacggactg aaacacatgt tcttttctct tacagtatga 29040
   ttaaatgaga catgattcct cgagttttta tattactgac ccttgttgcg ctttttttgt 29100
   gcgtgctcca cattggctgc ggtttctcac atcgaagtag actgcattcc agccttcaca 29160
   gtctatttgc tttacggatt tgtcaccctc acgctcatct gcagcctcat cactgtggtc 29220
   atcgccttta tccagtgcat tgactgggtc tgtgtgcgct ttgcatatct cagacaccat 29280
   ccccagtaca gggacaggac tatagctgag cttcttagaa ttctttaatt atgaaattta 29340
   ctgtgacttt tctgctgatt atttgcaccc tatctgcgtt ttgttccccg acctccaagc 29400
   ctcaaagaca tatatcatgc agattcactc gtatatggaa tattccaagt tgctacaatg 29460
   aaaaaagcga tctttccgaa gcctggttat atgcaatcat ctctgttatg gtgttctgca 29520
   gtaccatctt agccctagct atatatccct accttgacat tggctggaac gcaatagatg 29580
   ccatgaacca cccaactttc cccgcgcccg ctatgcttcc actgcaacaa gttgttgccg 29640
   gcggctttgt cccagccaat cagcctcgcc caccttctcc cacccccact gaaatcagct 29700
   actttaatct aacaggagga gatgactgac accctagatc tagaaatgga cggaattatt 29760
   acagagcagc gcctgctaga aagacgcagg gcagcggccg agcaacagcg catgaatcaa 29820
   gagctccaag acatggttaa cttgcaccag tgcaaaaggg gtatcttttg tctggtaaag 29880
   caggccaaag tcacctacga cagtaatacc accggacacc gccttagcta caagttgcca 29940
   accaagcgtc agaaattggt ggtcatggtg ggagaaaagc ccattaccat aactcagcac 30000
   tcggtagaaa ccgaaggctg cattcactca ccttgtcaag gacctgagga tctctgcacc 30060
   cttattaaga ccctgtgcgg tctcaaagat cttattccct ttaactaata aaaaaaaata 30120
   ataaagcatc acttacttaa aatcagttag caaatttctg tccagtttat tcagcagcac 30180
   ctccttgccc tcctcccagc tctggtattg cagcttcctc ctggctgcaa actttctcca 30240
   caatctaaat ggaatgtcag tttcctcctg ttcctgtcca tccgcaccca ctatcttcat 30300
   gttgttgcag atgaagcgcg caagaccgtc tgaagatacc ttcaaccccg tgtatccata 30360
   tgacacggaa accggtcctc caactgtgcc ttttcttact cctccctttg tatcccccaa 30420
   tgggtttcaa gagagtcccc ctggggtact ctctttgcgc ctatccgaac ctctagttac 30480
   ctccaatggc atgcttgcgc tcaaaatggg caacggcctc tctctggacg aggccggcaa 30540
   ccttacctcc caaaatgtaa ccactgtgag cccacctctc aaaaaaacca agtcaaacat 30600
   aaacctggaa atatctgcac ccctcacagt tacctcagaa gccctaactg tggctgccgc 30660
   cgcacctcta atggtcgcgg gcaacacact caccatgcaa tcacaggccc cgctaaccgt 30720
   gcacgactcc aaacttagca ttgccaccca aggacccctc acagtgtcag aaggaaagct 30780
   agccctgcaa acatcaggcc ccctcaccac caccgatagc agtaccctta ctatcactgc 30840
   ctcaccccct ctaactactg ccactggtag cttgggcatt gacttgaaag agcccattta 30900
   tacacaaaat ggaaaactag gactaaagta cggggctcct ttgcatgtaa cagacgacct 30960
   aaacactttg accgtagcaa ctggtccagg tgtgactatt aataatactt ccttgcaaac 31020
   taaagttact ggagccttgg gttttgattc acaaggcaat atgcaactta atgtagcagg 31080
   aggactaagg attgattctc aaaacagacg ccttatactt gatgttagtt atccgtttga 31140
   tgctcaaaac caactaaatc taagactagg acagggccct ctttttataa actcagccca 31200
   caacttggat attaactaca acaaaggcct ttacttgttt acagcttcaa acaattccaa 31260
   aaagcttgag gttaacctaa gcactgccaa ggggttgatg tttgacgcta cagccatagc 31320
   cattaatgca ggagatgggc ttgaatttgg ttcacctaat gcaccaaaca caaatcccct 31380
   caaaacaaaa attggccatg gcctagaatt tgattcaaac aaggctatgg ttcctaaact 31440
   aggaactggc cttagttttg acagcacagg tgccattaca gtaggaaaca aaaataatga 31500
   taagctaact ttgtggacca caccagctcc atctcctaac tgtagactaa atgcagagaa 31560
   agatgctaaa ctcactttgg tcttaacaaa atgtggcagt caaatacttg ctacagtttc 31620
   agttttggct gttaaaggca gtttggctcc aatatctgga acagttcaaa gtgctcatct 31680
   tattataaga tttgacgaaa atggagtgct actaaacaat tccttcctgg acccagaata 31740
   ttggaacttt agaaatggag atcttactga aggcacagcc tatacaaacg ctgttggatt 31800
   tatgcctaac ctatcagctt atccaaaatc tcacggtaaa actgccaaaa gtaacattgt 31860
   cagtcaagtt tacttaaacg gagacaaaac taaacctgta acactaacca ttacactaaa 31920
   cggtacacag gaaacaggag acacaactcc aagtgcatac tctatgtcat tttcatggga 31980
   ctggtctggc cacaactaca ttaatgaaat atttgccaca tcctcttaca ctttttcata 32040
   cattgcccaa gaataaagaa tcgtttgtgt tatgtttcaa cgtgtttatt tttcaattgc 32100
   agaaaatttc aagtcatttt tcattcagta gtatagcccc accaccacat agcttataca 32160
   gatcaccgta ccttaatcaa actcacagaa ccctagtatt caacctgcca cctccctccc 32220
   aacacacaga gtacacagtc ctttctcccc ggctggcctt aaaaagcatc atatcatggg 32280
   taacagacat attcttaggt gttatattcc acacggtttc ctgtcgagcc aaacgctcat 32340
   cagtgatatt aataaactcc ccgggcagct cacttaagtt catgtcgctg tccagctgct 32400
   gagccacagg ctgctgtcca acttgcggtt gcttaacggg cggcgaagga gaagtccacg 32460
   cctacatggg ggtagagtca taatcgtgca tcaggatagg gcggtggtgc tgcagcagcg 32520
   cgcgaataaa ctgctgccgc cgccgctccg tcctgcagga atacaacatg gcagtggtct 32580
   cctcagcgat gattcgcacc gcccgcagca taaggcgcct tgtcctccgg gcacagcagc 32640
   gcaccctgat ctcacttaaa tcagcacagt aactgcagca cagcaccaca atattgttca 32700
   aaatcccaca gtgcaaggcg ctgtatccaa agctcatggc ggggaccaca gaacccacgt 32760
   ggccatcata ccacaagcgc aggtagatta agtggcgacc cctcataaac acgctggaca 32820
   taaacattac ctcttttggc atgttgtaat tcaccacctc ccggtaccat ataaacctct 32880
   gattaaacat ggcgccatcc accaccatcc taaaccagct ggccaaaacc tgcccgccgg 32940
   ctatacactg cagggaaccg ggactggaac aatgacagtg gagagcccag gactcgtaac 33000
   catggatcat catgctcgtc atgatatcaa tgttggcaca acacaggcac acgtgcatac 33060
   acttcctcag gattacaagc tcctcccgcg ttagaaccat atcccaggga acaacccatt 33120
   cctgaatcag cgtaaatccc acactgcagg gaagacctcg cacgtaactc acgttgtgca 33180
   ttgtcaaagt gttacattcg ggcagcagcg gatgatcctc cagtatggta gcgcgggttt 33240
   ctgtctcaaa aggaggtaga cgatccctac tgtacggagt gcgccgagac aaccgagatc 33300
   gtgttggtcg tagtgtcatg ccaaatggaa cgccggacgt agtcatattt cctgaagcaa 33360
   aaccaggtgc gggcgtgaca aacagatctg cgtctccggt ctcgccgctt agatcgctct 33420
   gtgtagtagt tgtagtatat ccactctctc aaagcatcca ggcgccccct ggcttcgggt 33480
   tctatgtaaa ctccttcatg cgccgctgcc ctgataacat ccaccaccgc agaataagcc 33540
   acacccagcc aacctacaca ttcgttctgc gagtcacaca cgggaggagc gggaagagct 33600
   ggaagaacca tgtttttttt tttattccaa aagattatcc aaaacctcaa aatgaagatc 33660
   tattaagtga acgcgctccc ctccggtggc gtggtcaaac tctacagcca aagaacagat 33720
   aatggcattt gtaagatgtt gcacaatggc ttccaaaagg caaacggccc tcacgtccaa 33780
   gtggacgtaa aggctaaacc cttcagggtg aatctcctct ataaacattc cagcaccttc 33840
   aaccatgccc aaataattct catctcgcca ccttctcaat atatctctaa gcaaatcccg 33900
   aatattaagt ccggccattg taaaaatctg ctccagagcg ccctccacct tcagcctcaa 33960
   gcagcgaatc atgattgcaa aaattcaggt tcctcacaga cctgtataag attcaaaagc 34020
   ggaacattaa caaaaatacc gcgatcccgt aggtcccttc gcagggccag ctgaacataa 34080
   tcgtgcaggt ctgcacggac cagcgcggcc acttccccgc caggaaccat gacaaaagaa 34140
   cccacactga ttatgacacg catactcgga gctatgctaa ccagcgtagc cccgatgtaa 34200
   gcttgttgca tgggcggcga tataaaatgc aaggtgctgc tcaaaaaatc aggcaaagcc    34260
   tcgcgcaaaa aagaaagcac atcgtagtca tgctcatgca gataaaggca ggtaagctcc    34320
   ggaaccacca cagaaaaaga caccattttt ctctcaaaca tgtctgcggg tttctgcata    34380
   aacacaaaat aaaataacaa aaaaacattt aaacattaga agcctgtctt acaacaggaa    34440
   aaacaaccct tataagcata agacggacta cggccatgcc ggcgtgaccg taaaaaaact    34500
   ggtcaccgtg attaaaaagc accaccgaca gctcctcggt catgtccgga gtcataatgt    34560
   aagactcggt aaacacatca ggttgattca catcggtcag tgctaaaaag cgaccgaaat    34620
   agcccggggg aatacatacc cgcaggcgta gagacaacat tacagccccc ataggaggta    34680
   taacaaaatt aataggagag aaaaacacat aaacacctga aaaaccctcc tgcctaggca    34740
   aaatagcacc ctcccgctcc agaacaacat acagcgcttc cacagcggca gccataacag    34800
   tcagccttac cagtaaaaaa gaaaacctat taaaaaaaca ccactcgaca cggcaccagc    34860
   tcaatcagtc acagtgtaaa aaagggccaa gtgcagagcg agtatatata ggactaaaaa    34920
   atgacgtaac ggttaaagtc cacaaaaaac acccagaaaa ccgcacgcga acctacgccc    34980
   agaaacgaaa gccaaaaaac ccacaacttc ctcaaatcgt cacttccgtt ttcccacgtt    35040
   acgtcacttc ccattttaag aaaactacaa ttcccaacac atacaagtta ctccgcccta    35100
   aaacctacgt cacccgcccc gttcccacgc cccgcgccac gtcacaaact ccaccccctc    35160
   attatcatat tggcttcaat ccaaaataag gtatattatt gat                      35203
<210> 11
   <211> 33093
   <212> DNA
   <213> Artificial sequence
<220>
   <223> Empty Ad5 vector sequence (repeats included)
<400> 11
<210> 12
   <211> 978
   <212> DNA
   <213> Artificial sequence
<220>
   <223> PPE-1-3X promoter
<400> 12
   acgtgtactt ctgatcggcg atactaggga gataaggatg tacctgacaa aaccacattg 60
   ttgttgttat cattattatt tagttttcct tccttgctaa ctcctgacgg aatctttctc 120
   acctcaaatg cgaagtactt tagtttagaa aagacttggt ggaaggggtg gtggtggaaa 180
   agtagggtga tcttccaaac taatctggtt ccccgcccgc cccagtagct gggattcaag 240
   agcgaagagt ggggatcgtc cccttgtttg atcagaaaga cataaaagga aaatcaagtg 300
   aacaatgatc agccccacct ccaccccacc cccctgcgcg cgcacaatac aatctattta 360
   attgtacttc atacttttca ttccaatggg gtgactttgc ttctggagaa actcttgatt 420
   cttgaactct ggggctggca gctagcctcc agaagcaaag tcaccccatt ggaatgaaaa 480
   gtatgaagta caatgaaaag tatgaagtac tggctccaga agcaaagtca ccctccagaa 540
   gcaaagtcac cccattggaa tgaaaagtat gaagtacgct agcaaaaggg gaagcgggct 600
   gctgctctct gcaggttctg cagcggtctc tgtctagtgg gtgttttctt tttcttagcc 660
   ctgcccctgg attgtcagac ggcgggcgtc tgcctctgaa gttagccgtg atttcctcta 720
   gagccgggtc ttatctctgg ctgcacgttg cctgtgggtg actaatcaca caataacatt 780
   gtttagggct ggaataaagt cagagctgtt tacccccact ctataggggt tcaatataaa 840
   aaggcggcgg agaactgtcc gagtcagaag cgttcctgca ccggcgctga gagcctgacc 900
   cggtctgctc cgctgtcctt gcgcgctgcc tcccggctgc ccgcgacgct ttcgccccag 960
   tggaagggcc acttgctg 978
<210> 13
   <211> 1334
   <212> DNA
   <213> Mus musculus
<400> 13
   gtgtgtgtgt gtgtgtgtgt gtgtgtgtgt gtgtgtgtgt gtgtagtgta cttctgatcg      60
   gcgatactag ggagataagg atgtacctga caaaaccaca ttgttgttgt tatcattatt     120
   atttagtttt ccttccttgc taactcctga cggaatcttt ctcacctcaa atgcgaagta     180
   ctttagttta gaaaagactt ggtggaaggg gtggtggtgg aaaagtaggg tgatcttcca     240
   aactaatctg gttccccgcc cgccccagta gctgggattc aagagcgaag agtggggatc     300
   gtccccttgt ttgatcagaa agacataaaa ggaaaatcaa gtgaacaatg atcagcccca     360
   cctccacccc acccccctgc gcgcgcacaa tacaatctat ttaattgtac ttcatacttt      420
   tcattccaat ggggtgactt tgcttctgga gaaactcttg attcttgaac tctggggctg     480
   gcagctagca aaaggggaag cgggctgctg ctctctgcag gttctgcagc ggtctctgtc     540
   tagtgggtgt tttctttttc ttagccctgc ccctggattg tcagacggcg ggcgtctgcc      600
   tctgaagtta gccgtgattt cctctagagc cgggtcttat ctctggctgc acgttgcctg     660
   tgggtgacta atcacacaat aacattgttt agggctggaa taaagtcaga gctgtttacc     720
   cccactctat aggggttcaa tataaaaagg cggcggagaa ctgtccgagt cagacgcgtt     780
   cctgcaccgg cgctgagagc ctgacccggt ctgctccgct gtccttgcgc gctgcctccc     840
   ggctgcccgc gacgctttcg ccccagtgga agggccactt gctgaggacc gcgctgagat     900
   ctaaaaaaaa aacaaaaaac aaaaaacaaa aaaacccaga ggcgatcaga gcgaccagac     960
   accgtcctct tcgttttgca ttgagttcca tttgcaaccg agttttcttt ttttcctttt    1020
   tccccactct tctgacccct ttgcagaatg gattattttc ccgtgatctt ctctctgctg    1080
   ttcgtgactt tccaaggagc tccagaaaca ggtaggcgcc acttgcgaat ctttctactt    1140
   cagcgcagca gttatcgctt ctgttttcca cttttctttc tttcttttct ttcattcttt    1200
   cctttttatt tattttttta attactgaag ctccagcagc aagtgcctta caattaatta   1260
   acttctgtgt gaagcgaaag aaataaaacc cctgtttgaa tacagctgac tacaaccgag    1320
   tatcgcatag cttc                                                      1334
<210> 14
   <211> 5
   <212> DNA
   <213> Artificial sequence
<220>
   <223> wild type element M9
<400> 14
   ctgga 5
<210> 15
   <211> 5
   <212> DNA
   <213> Artificial sequence
<220>
   <223> wild type element H4
<400> 15
   cattc 5
<210> 16
   <211> 5
   <212> DNA
   <213> Artificial sequence
<220>
   <223> wild type element M5
<400> 16
   caatg 5
<210> 17
   <211> 5
   <212> DNA
   <213> Artificial sequence
<220>
   <223> wild type element M6
<400> 17
   gggtg 5
<210> 18
   <211> 5
   <212> DNA
   <213> Artificial sequence
<220>
   <223> wild type element M7
<400> 18
   acttt 5
<210> 19
   <211> 5
   <212> DNA
   <213> Artificial sequence
<220>
   <223> wild type element M8
<400> 19
   gcttc 5
<210> 20
   <211> 5
   <212> DNA
   <213> Artificial sequence
<220>
   <223> wild type element M1
<220>
   <221> misc_feature
   <222> (1)..(6)
   <223> n is a, c, g, or t
<400> 20
   gtact 5
<210> 21
   <211> 5
   <212> DNA
   <213> Artificial sequence
<220>
   <223> wild type element M3
<220>
   <221> misc_feature
   <222> (1)..(6)
   <223> n is a, c, g, or t
<400> 21
   ctttt 5
<210> 22
   <211> 19
   <212> DNA
   <213> Artificial sequence
<220>
   <223> Exemplary element X variant
<220>
   <221> misc_feature
   <222> (6)..(10)
   <223> At least one residue mutated or absent
<400> 22
   gggtgacttt gcttctgga 19
<210> 23
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 23
   gggtgacttt gcttctgga 19
<210> 24
   <211> 19
   <212> DNA
   <213> Artificial sequence
<220>
   <223> Exemplary element X variant
<220>
   <221> misc_feature
   <222> (12)..(16)
   <223> At least one residue mutated or absent
<400> 24
   ggggtgactt tgcttctgg 19
<210> 25
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 25
   caatggggtg gcttctgga 19
<210> 26
   <211> 19
   <212> DNA
   <213> Artificial sequence
<220>
   <223> Exemplary element X variant
<220>
   <221> misc_feature
   <222> (12)..(16)
   <223> At least one residue mutated or absent
<400> 26
   ccaatggggt ggcttctgg 19
<210> 27
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 27
   gggtgacttt gcttctgga 19
<210> 28
   <211> 19
   <212> DNA
   <213> Artificial sequence
<220>
   <223> Exemplary element X variant
<220>
   <221> misc_feature
   <222> (12)..(16)
   <223> At least one residue mutated or absent
<400> 28
   ggggtgactt tgcttctgg 19
<210> 29
   <211> 44
   <212> DNA
   <213> Artificial sequence
<220>
   <223> Exemplary element X variant
<220>
   <221> misc_feature
   <222> (36)..(40)
   <223> At least one residue mutated or absent
<400> 29
   gtacttcata cttttcattc caatggggtg actttgcttc tgga 44
<210> 30
   <211> 39
   <212> DNA
   <213> Artificial sequence
<220>
   <223> Exemplary element X variant
<220>
   <221> misc_feature
   <222> (31)..(35)
   <223> At least one residue mutated or absent
<400> 30
   gtactctttt cattccaatg gggtgacttt gcttctgga 39
<210> 31
   <211> 39
   <212> DNA
   <213> Artificial sequence
<220>
   <223> Exemplary element X variant
<220>
   <221> misc_feature
   <222> (31)..(35)
   <223> At least one residue mutated or absent
<400> 31
   gtacttcata cattccaatg gggtgacttt gcttctgga 39
<210> 32
   <211> 39
   <212> DNA
   <213> Artificial sequence
<220>
   <223> Exemplary element X variant
<220>
   <221> misc_feature
   <222> (31)..(35)
   <223> At least one residue mutated or absent
<400> 32
   gtacttcata cttttcaatg gggtgacttt gcttctgga 39
<210> 33
   <211> 39
   <212> DNA
   <213> Artificial sequence
<220>
   <223> Exemplary element X variant
<220>
   <221> misc_feature
   <222> (31)..(35)
   <223> At least one residue mutated or absent
<400> 33
   gtacttcata cttttcattc gggtgacttt gcttctgga 39
<210> 34
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 34
   gtactcattc caatggggtg actttgcttc tgga 34
<210> 35
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 35
   gtactcaatg gggtgacttt gcttctgga 29
<210> 36
   <211> 24
   <212> DNA
   <213> Artificial sequence
<220>
   <223> Exemplary element X variant
<220>
   <221> misc_feature
   <222> (16)..(20)
   <223> At least one residue mutated or absent
<400> 36
   gtactgggtg actttgcttc tgga 24
<210> 37
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 37
   gtacttcata caatggggtg actttgcttc tgga 34
<210> 38
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 38
   gtacttcata gggtgacttt gcttctgga 29
<210> 39
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 39
   gtacttcata cttttgggtg actttgcttc tgga 34
<210> 40
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 40
   gtactctttt caatggggtg actttgcttc tgga 34
<210> 41
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 41
   gtacttcata cattcgggtg actttgcttc tgga 34
<210> 42
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 42
   gtactctttt cattcgggtg actttgcttc tgga 34
<210> 43
   <211> 44
   <212> DNA
   <213> Artificial sequence
<220>
   <223> Exemplary element X variant
<220>
   <221> misc_feature
   <222> (36)..(40)
   <223> At least one residue mutated or absent
<400> 43
   gtacttcata cttttcattc caatggggtg actttgcttc tgga 44
<210> 44
   <211> 39
   <212> DNA
   <213> Artificial sequence
<220>
   <223> Exemplary element X variant
<220>
   <221> misc_feature
   <222> (31)..(35)
   <223> At least one residue mutated or absent
<400> 44
   gtactctttt cattccaatg gggtgacttt gcttctgga 39
<210> 45
   <211> 39
   <212> DNA
   <213> Artificial sequence
<220>
   <223> Exemplary element X variant
<220>
   <221> misc_feature
   <222> (31)..(35)
   <223> At least one residue mutated or absent
<400> 45
   gtacttcata cattccaatg gggtgacttt gcttctgga 39
<210> 46
   <211> 39
   <212> DNA
   <213> Artificial sequence
<220>
   <223> Exemplary element x variant
<220>
   <221> misc_feature
   <222> (31)..(35)
   <223> At least one residue mutated or absent
<400> 46
   gtacttcata cttttcaatg gggtgacttt gcttctgga 39
<210> 47
   <211> 39
   <212> DNA
   <213> Artificial sequence
<220>
   <223> Exemplary element X variant
<220>
   <221> misc_feature
   <222> (31)..(35)
   <223> At least one residue mutated or absent
<400> 47
   gtacttcata cttttcattc gggtgacttt gcttctgga 39
<210> 48
   <211> 39
   <212> DNA
   <213> Artificial sequence
<220>
   <223> Exemplary element X variant
<220>
   <221> misc_feature
   <222> (31)..(35)
   <223> At least one residue mutated or absent
<400> 48
   gtacttcata cttttcattc caatgacttt gcttctgga 39
<210> 49
   <211> 39
   <212> DNA
   <213> Artificial sequence
<220>
   <223> Exemplary element X variant
<220>
   <221> misc_feature
   <222> (31)..(35)
   <223> At least one residue mutated or absent
<400> 49
   gtacttcata cttttcattc caatggggtg gcttctgga 39
<210> 50
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 50
   gtactcattc caatggggtg actttgcttc tgga 34
<210> 51
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 51
   gtactcaatg gggtgacttt gcttctgga 29
<210> 52
   <211> 24
   <212> DNA
   <213> Artificial sequence
<220>
   <223> Exemplary element X variant
<220>
   <221> misc_feature
   <222> (16)..(20)
   <223> At least one residue mutated or absent
<400> 52
   gtactgggtg actttgcttc tgga 24
<210> 53
   <211> 19
   <212> DNA
   <213> Artificial sequence
<220>
   <223> Exemplary element X variant
<220>
   <221> misc feature
   <222> (11)..(15)
   <223> At least one residue mutated or absent
<400> 53
   gtactacttt gcttctgga 19
<210> 54
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 54
   gtacttcata caatggggtg actttgcttc tgga 34
<210> 55
   <211> 28
   <212> DNA
   <213> Artificial sequence
<220>
   <223> Exemplary element X variant
<220>
   <221> misc_feature
   <222> (21)..(25)
   <223> At least one residue mutated or absent
<400> 55
   cattccaatg gggtgacttt gcttctgg 28
<210> 56
   <211> 23
   <212> DNA
   <213> Artificial sequence
<220>
   <223> Exemplary element X variant
<220>
   <221> misc_feature
   <222> (16)..(20)
   <223> At least one residue mutated or absent
<400> 56
   cattcgggtg actttgcttc tgg 23
<210> 57
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 57
   cattcacttt gcttctgga 19
<210> 58
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 58
   cttttcattc gcttctgga 19
<210> 59
   <211> 23
   <212> DNA
   <213> Artificial sequence
<220>
   <223> Exemplary element X variant
<220>
   <221> misc_feature
   <222> (16)..(20)
   <223> At least one residue mutated or absent
<400> 59
   cattccaatg actttgcttc tgg 23
<210> 60
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 60
   cattccaatg gcttctgga 19
<210> 61
   <211> 23
   <212> DNA
   <213> Artificial sequence
<220>
   <223> Exemplary element X variant
<220>
   <221> misc_feature
   <222> (16)..(20)
   <223> At least one residue mutated or absent
<400> 61
   cattccaatg gggtggcttc tgg 23
<210> 62
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 62
   cattcgggtg gcttctgga 19
<210> 63
   <211> 23
   <212> DNA
   <213> Artificial sequence
<220>
   <223> Exemplary element X variant
<220>
   <221> misc_feature
   <222> (16)..(20)
   <223> At least one residue mutated or absent
<400> 63
   caatggggtg actttgcttc tgg 23
<210> 64
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 64
   caatggggtg gcttctgga 19
<210> 65
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 65
   caatgacttt gcttctgga 19
<210> 66
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 66
   cattccaatg gcttctgga 19
<210> 67
   <211> 28
   <212> DNA
   <213> Artificial sequence
<220>
   <223> Exemplary element X variant
<220>
   <221> misc_feature
   <222> (21)..(25)
   <223> At least one residue mutated or absent
<400> 67
   cattccaatg gggtgacttt gcttctgg 28
<210> 68
   <211> 23
   <212> DNA
   <213> Artificial sequence
<220>
   <223> Exemplary element X variant
<220>
   <221> misc_feature
   <222> (16)..(20)
   <223> At least one residue mutated or absent
<400> 68
   cattccaatg actttgcttc tgg 23
<210> 69
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 69
   cattccaatg gcttctgga 19
<210> 70
   <211> 23
   <212> DNA
   <213> Artificial sequence
<220>
   <223> Exemplary element X variant
<220>
   <221> misc_feature
   <222> (16)..(20)
   <223> At least one residue mutated or absent
<400> 70
   cattccaatg gggtggcttc tgg 23
<210> 71
   <211> 43
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (36)..(40)
   <223> At least one residue mutated or absent
<400> 71
   gtacttcata cttttcattc caatggggtg actttgcttc tgg 43
<210> 72
   <211> 43
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (36)..(40)
   <223> At least one residue mutated or absent
<400> 72
   gtacttcata cttttcattc caatggggtg actttgcttc tgg 43
<210> 73
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 73
   gtactctttt cattccaatg gggtgacttt gcttctgg 38
<210> 74
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 74
   gtacttcata cattccaatg gggtgacttt gcttctgg 38
<210> 75
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 75
   gtactcattc caatggggtg actttgcttc tgg 33
<210> 76
   <211> 43
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (36)..(40)
   <223> At least one residue mutated or absent
<400> 76
   gtacttcata cttttcattc caatggggtg actttgcttc tgg 43
<210> 77
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 77
   gtacttcata cttttcattc gggtgacttt gcttctgg 38
<210> 78
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 78
   gtacttcata cttttcattc caatgacttt gcttctgg 38
<210> 79
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 79
   gtacttcata cttttcattc caatggggtg gcttctgg 38
<210> 80
   <211> 33
   <212> DNA
   <213> Artificial sequence
<220>
   <223> Exemplary element x variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 80
   gtacttcata cttttcattc actttgcttc tgg 33
<210> 81
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 81
   gtacttcata cttttcattc gggtggcttc tgg 33
<210> 82
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 82
   gtacttcata cttttcattc gcttctgg 28
<210> 83
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 83
   gtacttcata cttttcattc caatggcttc tgg 33
<210> 84
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 84
   gtacttcata cattcgggtg actttgcttc tgg 33
<210> 85
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 85
   gtacttcata cattccaatg actttgcttc tgg 33
<210> 86
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 86
   gtacttcata cattccaatg gggtggcttc tgg 33
<210> 87
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 87
   gtacttcata cattcacttt gcttctgg 28
<210> 88
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 88
   gtacttcata cattcgggtg gcttctgg 28
<210> 89
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 89
   gtacttcata cattcgcttc tgg 23
<210> 90
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 90
   gtacttcata cattccaatg gcttctgg 28
<210> 91
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 91
   gtactctttt cattccaatg gggtgacttt gcttctgg 38
<210> 92
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 92
   gtactctttt cattcgggtg actttgcttc tgg 33
<210> 93
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 93
   gtactctttt cattccaatg actttgcttc tgg 33
<210> 94
   <211> 33
   <212> DNA
   <213> Artificial sequence
<220>
   <223> Exemplary element x variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 94
   gtactctttt cattccaatg gggtggcttc tgg 33
<210> 95
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 95
   gtactctttt cattcacttt gcttctgg 28
<210> 96
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 96
   gtactctttt cattcgggtg gcttctgg 28
<210> 97
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 97
   gtactctttt cattcgcttc tgg 23
<210> 98
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 98
   gtactctttt cattccaatg gcttctgg 28
<210> 99
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 99
   gtactcattc caatggggtg actttgcttc tgg 33
<210> 100
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 100
   gtactcattc gggtgacttt gcttctgg 28
<210> 101
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 101
   gtactcattc caatgacttt gcttctgg 28
<210> 102
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 102
   gtactcattc caatggggtg gcttctgg 28
<210> 103
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 103
   gtactcattc actttgcttc tgg 23
<210> 104
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 104
   gtactcattc gggtggcttc tgg 23
<210> 105
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 105
   gtactcattc caatggcttc tgg 23
<210> 106
   <211> 43
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (36)..(40)
   <223> At least one residue mutated or absent
<400> 106
   gtacttcata cttttcattc caatggggtg actttgcttc tgg 43
<210> 107
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 107
   gtactctttt cattccaatg gggtgacttt gcttctgg 38
<210> 108
   <211> 38
   <212> DNA
   <213> Artificial sequence
<220>
   <223> Exemplary element x variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 108
   gtacttcata cattccaatg gggtgacttt gcttctgg 38
<210> 109
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 109
   gtacttcata cttttcaatg gggtgacttt gcttctgg 38
<210> 110
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 110
   gtactcattc caatggggtg actttgcttc tgg 33
<210> 111
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 111
   gtacttcata caatggggtg actttgcttc tgg 33
<210> 112
   <211> 33
   <212> DNA
   <213> Artificial sequence
<220>
   <223> Exemplary element x variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 112
   gtactctttt caatggggtg actttgcttc tgg 33
<210> 113
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 113
   gtactcaatg gggtgacttt gcttctgg 28
<210> 114
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 114
   gtacttcata cttttcattc caatgacttt gcttctgg 38
<210> 115
   <211> 38
   <212> DNA
   <213> Artificial sequence
<220>
   <223> Exemplary element x variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 115
   gtacttcata cttttcattc caatggggtg gcttctgg 38
<210> 116
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 116
   gtacttcata cttttcattc caatggcttc tgg 33
<210> 117
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 117
   gtactctttt cattccaatg actttgcttc tgg 33
<210> 118
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 118
   gtacttcata cattccaatg actttgcttc tgg 33
<210> 119
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 119
   gtacttcata cttttcaatg actttgcttc tgg 33
<210> 120
   <211> 28
   <212> DNA
   <213> Artificial sequence
<220>
   <223> Exemplary element x variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 120
   gtactcattc caatgacttt gcttctgg 28
<210> 121
   <211> 28
   <212> DNA
   <213> Artificial sequence
<220>
   <223> Exemplary element x variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 121
   gtacttcata caatgacttt gcttctgg 28
<210> 122
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 122
   gtactctttt caatgacttt gcttctgg 28
<210> 123
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 123
   gtactcaatg actttgcttc tgg 23
<210> 124
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 124
   gtactctttt cattccaatg gggtggcttc tgg 33
<210> 125
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 125
   gtacttcata cattccaatg gggtggcttc tgg 33
<210> 126
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 126
   gtacttcata cttttcaatg gggtggcttc tgg 33
<210> 127
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 127
   gtactcattc caatggggtg gcttctgg 28
<210> 128
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 128
   gtacttcata caatggggtg gcttctgg 28
<210> 129
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 129
   gtactctttt caatggggtg gcttctgg 28
<210> 130
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 130
   gtactcaatg gggtggcttc tgg 23
<210> 131
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 131
   gtactctttt cattccaatg gcttctgg 28
<210> 132
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 132
   gtacttcata cattccaatg gcttctgg 28
<210> 133
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 133
   gtacttcata cttttcaatg gcttctgg 28
<210> 134
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 134
   gtactcattc caatggcttc tgg 23
<210> 135
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 135
   gtacttcata caatggcttc tgg 23
<210> 136
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 136
   gtactctttt caatggcttc tgg 23
<210> 137
   <211> 43
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (36)..(40)
   <223> At least one residue mutated or absent
<400> 137
   gtacttcata cttttcattc caatggggtg actttgcttc tgg 43
<210> 138
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 138
   gtactctttt cattccaatg gggtgacttt gcttctgg 38
<210> 139
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 139
   gtacttcata cattccaatg gggtgacttt gcttctgg 38
<210> 140
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 140
   gtactcattc caatggggtg actttgcttc tgg 33
<210> 141
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 141
   gtacttcata cttttcattc caatgacttt gcttctgg 38
<210> 142
   <211> 38
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (31)..(35)
   <223> At least one residue mutated or absent
<400> 142
   gtacttcata cttttcattc caatggggtg gcttctgg 38
<210> 143
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 143
   gtacttcata cttttcattc caatggcttc tgg 33
<210> 144
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 144
   gtactctttt cattccaatg actttgcttc tgg 33
<210> 145
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 145
   gtacttcata cattccaatg actttgcttc tgg 33
<210> 146
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 146
   gtactcattc caatgacttt gcttctgg 28
<210> 147
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 147
   gtactctttt cattccaatg gggtggcttc tgg 33
<210> 148
   <211> 33
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (26)..(30)
   <223> At least one residue mutated or absent
<400> 148
   gtacttcata cattccaatg gggtggcttc tgg 33
<210> 149
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 149
   gtactcattc caatggggtg gcttctgg 28
<210> 150
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 150
   gtactctttt cattccaatg gcttctgg 28
<210> 151
   <211> 28
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (21)..(25)
   <223> At least one residue mutated or absent
<400> 151
   gtacttcata cattccaatg gcttctgg 28
<210> 152
   <211> 23
   <212> DNA
   <213> Artificial sequence
<220>
   <223> Exemplary element X variant
<220>
   <221> misc_feature
   <222> (4)..(5)
   <223> At least one residue mutated or absent
<220>
   <221> misc_feature
   <222> (16)..(20)
   <223> At least one residue mutated or absent
<400> 152
   gtactcattc caatggcttc tgg 23
<210> 153
   <211> 43
   <212> DNA
   <213> Artificial sequence
<220>
   <223> Exemplary element X variant
<220>
   <221> misc_feature
   <222> (16)..(20)
   <223> At least one residue mutated or absent
<400> 153
   gtacttcata cttttcattc caatggggtg actttgcttc tgg 43
<210> 154
   <211> 19
   <212> DNA
   <213> Artificial sequence
<220>
   <223> Exemplary element X variant
<220>
   <221> misc_feature
   <222> (7)..(11)
   <223> At least one residue mutated or absent
<400> 154
   acttttcatt ccaatgggg 19
<210> 155
   <211> 19
   <212> DNA
   <213> Artificial sequence
<220>
   <223> Exemplary element X variant
<220>
   <221> misc_feature
   <222> (6)..(10)
   <223> At least one residue mutated or absent
<400> 155
   cttttcattc caatggggt 19
<210> 156
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 156
   ttttcattcc aatggggtg 19
<210> 157
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 157
   tttcattcca atggggtga 19
<210> 158
   <211> 19
   <212> DNA
   <213> Artificial sequence
<220>
   <223> Exemplary element X variant
<220>
   <221> misc_feature
   <222> (8)..(12)
   <223> At least one residue mutated or absent
<400> 158
   tacttttcat tccaatggg 19
<210> 159
   <211> 19
   <212> DNA
   <213> Artificial sequence
<220>
   <223> Exemplary element X variant
<220>
   <221> misc_feature
   <222> (9)..(13)
   <223> At least one residue mutated or absent
<400> 159
   atacttttca ttccaatgg 19
<210> 160
   <211> 19
   <212> DNA
   <213> Artificial sequence
<220>
   <223> Exemplary element X variant
<220>
   <221> misc_feature
   <222> (10)..(14)
   <223> At least one residue mutated or absent
<400> 160
   catacttttc attccaatg 19
<210> 161
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 161
   tcatactttt cattccaat 19
<210> 162
   <211> 19
   <212> DNA
   <213> Artificial sequence
<220>
   <223> Exemplary element X variant
<220>
   <221> misc_feature
   <222> (12)..(16)
   <223> At least one residue mutated or absent
<400> 162
   ttcatacttt tcattccaa 19
<210> 163
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 163
   ttccaatggg gtgactttg 19
<210> 164
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 164
   attccaatgg ggtgacttt 19
<210> 165
   <211> 19
   <212> DNA
   <213> Artificial sequence
<220>
   <223> Exemplary element X variant
<220>
   <221> misc_feature
   <222> (6)..(10)
   <223> At least one residue mutated or absent
<400> 165
   cattccaatg gggtgactt 19
<210> 166
   <211> 19
   <212> DNA
   <213> Artificial sequence
<220>
   <223> Exemplary element X variant
<220>
   <221> misc_feature
   <222> (7)..(11)
   <223> At least one residue mutated or absent
<400> 166
   tcattccaat ggggtgact 19
<210> 167
   <211> 19
   <212> DNA
   <213> Artificial sequence
<220>
   <223> Exemplary element X variant
<220>
   <221> misc_feature
   <222> (8)..(12)
   <223> At least one residue mutated or absent
<400> 167
   ttcattccaa tggggtgac 19
<210> 168
   <211> 19
   <212> DNA
   <213> Artificial sequence
<220>
   <223> Exemplary element X variant
<220>
   <221> misc_feature
   <222> (9)..(13)
   <223> At least one residue mutated or absent
<900> 168
   tttcattcca atggggtga 19
<210> 169
   <211> 19
   <212> DNA
   <213> Artificial sequence
<220>
   <223> Exemplary element X variant
<220>
   <221> misc_feature
   <222> (10)..(14)
   <223> At least one residue mutated or absent
<400> 169
   ttttcattcc aatggggtg 19
<210> 170
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 170
   cttttcattc caatggggt 19
<210> 171
   <211> 19
   <212> DNA
   <213> Artificial sequence
<220>
   <223> Exemplary element X variant
<220>
   <221> misc_feature
   <222> (12)..(16)
   <223> At least one residue mutated or absent
<400> 171
   acttttcatt ccaatgggg 19
<210> 172
   <211> 24
   <212> DNA
   <213> Artificial sequence
<220>
   <223> Exemplary element X variant
<220>
   <221> misc_feature
   <222> (4)..(13)
   <223> At least one residue mutated or absent
<400> 172
   tttcattcca atggggtgac tttg 24
<210> 173
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 173
   ttttcattcc aatggggtga cttt 24
<210> 174
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 174
   cttttcattc caatggggtg actt 24
<210> 175
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 175
   acttttcatt ccaatggggt gact 24
<210> 176
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 176
   tacttttcat tccaatgggg tgac 24
<210> 177
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 177
   atacttttca ttccaatggg gtga 24
<210> 178
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 178
   catacttttc attccaatgg ggtg 24
<210> 179
   <211> 24
   <212> DNA
   <213> Artificial sequence
<220>
   <223> Exemplary element X variant
<220>
   <221> misc_feature
   <222> (11)..(20)
   <223> At least one residue mutated or absent
<400> 179
   tcatactttt cattccaatg gggt 24
<210> 180
   <211> 24
   <212> DNA
   <213> Artificial sequence
<220>
   <223> Exemplary element X variant
<220>
   <221> misc_feature
   <222> (11)..(21)
   <223> At least one residue mutated or absent
<400> 180
   ttcatacttt tcattccaat gggg 24
<210> 181
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 181
   ttttcattcc aatggggtg 19
<210> 182
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 182
   tttcattcca atggggtga 19
<210> 183
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 183
   attccaatgg ggtgacttt 19
<210> 184
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 184
   ttccaatggg gtgactttg 19
<210> 185
   <211> 19
   <212> DNA
   <213> Artificial sequence
<220>
   <223> Exemplary element X variant
<220>
   <221> misc_feature
   <222> (6)-.(10)
   <223> At least one residue mutated or absent
<400> 185
   cattccaatg gggtgactt 19
<210> 186
   <211> 19
   <212> DNA
   <213> Artificial sequence
<220>
   <223> Exemplary element X variant
<220>
   <221> misc_feature
   <222> (7)..(11)
   <223> At least one residue mutated or absent
<400> 186
   tcattccaat ggggtgact 19
<210> 187
   <211> 19
   <212> DNA
   <213> Artificial sequence
<220>
   <223> Exemplary element X variant
<220>
   <221> misc_feature
   <222> (B)..(12)
   <223> At least one residue mutated or absent
<400> 187
   ttcattccaa tggggtgac 19
<210> 188
   <211> 19
   <212> DNA
   <213> Artificial sequence
<220>
   <223> Exemplary element X variant
<220>
   <221> misc_feature
   <222> (9)..(13)
   <223> At least one residue mutated or absent
<400> 188
   tttcattcca atggggtga 19
<210> 189
   <211> 19
   <212> DNA
   <213> Artificial sequence
<220>
   <223> Exemplary element x variant
<220>
   <221> misc_feature
   <222> (10)..(14)
   <223> At least one residue mutated or absent
<400> 189
   ttttcattcc aatggggtg 19
<210> 190
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 190
   ttttcattcc aatggggtga cttt 24
<210> 191
   <211> 25
   <212> DNA
   <213> Artificial sequence
<220>
   <223> Exemplary element X variant
<220>
   <221> misc_feature
   <222> (11)..(20)
   <223> At least one residue mutated or absent
<400> 191
   tcatactttt cattccaatg gggtg 25
<210> 192
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 192
   tttcattcca atggggtgac ttt 23
<210> 193
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 193
   ttttcattcg ggtgacttt 19
<210> 194
   <211> 24
   <212> DNA
   <213> Artificial sequence
<220>
   <223> Exemplary element X variant
<220>
   <221> misc_feature
   <222> (5)..(9)
   <223> At least one residue mutated or absent
<400> 194
   ttttcattcc aatgactttg cttc 24
<210> 195
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 195
   ttttcattca ctttgcttc 19
<210> 196
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 196
   attccaatgg ggtgacttt 19
<210> 197
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 197
   attccaatga ctttgcttc 19
<210> 198
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 198
   ttccaatgac tttgcttct 19
<210> 199
   <211> 24
   <212> DNA
   <213> Artificial sequence
<220>
   <223> Exemplary element X variant
<220>
   <221> misc_feature
   <222> (4)..(13)
   <223> At least one residue mutated or absent
<400> 199
   tttcattcca atggggtgac tttg 24
<210> 200
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 200
   ttttcattcc aatggggtga cttt 24
<210> 201
   <211> 25
   <212> DNA
   <213> Artificial sequence
<220>
   <223> Exemplary element X variant
<220>
   <221> misc_feature
   <222> (6)..(15)
   <223> At least one residue mutated or absent
<400> 201
   cttttcattc caatggggtg acttt 25
<210> 202
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 202
   ttttcattcc aatgactttg cttc 24
<210> 203
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 203
   tttcattcca atggggtgac ttt 23
<210> 204
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 204
   ttttcattcg ggtgacttt 19
<210> 205
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 205
   tttcattcgg gtgactttg 19
<210> 206
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 206
   attccaatgg ggtgacttt 19
<210> 207
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 207
   ttccaatggg gtgactttg 19
<210> 208
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 208
   ttttcattcc aatggggtga cttt 24
<210> 209
   <211> 24
   <212> DNA
   <213> Artificial sequence
<220>
   <223> Exemplary element X variant
<220>
   <221> misc_feature
   <222> (4)..(13)
   <223> At least one residue mutated or absent
<400> 209
   tttcattcca atggggtgac tttg 24
<210> 210
   <211> 24
   <212> DNA
   <213> Artificial sequence
<220>
   <223> Exemplary element X variant
<220>
   <221> misc_feature
   <222> (4)..(8)
   <223> At least one residue mutated or absent
<220>
   <221> misc_feature
   <222> (14)..(18)
   <223> At least one residue mutated or absent
<400> 210
   tttcattcca atggggtgac tttg 24
<210> 211
   <211> 24
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(19)
   <223> At least one residue mutated or absent
<400> 211
   ttttcattcc aatggggtga cttt 24
<210> 212
   <211> 24
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 212
   cttttcattc caatggggtg actt 24
<210> 213
   <211> 24
   <212> DNA
   <213> Artificial sequence
<220>
   <223> Exemplary element X variant
<220>
   <221> misc_feature
   <222> (7)..(11)
   <223> At least one residue mutated or absent
<220>
   <221> misc_feature
   <222> (17)..(21)
   <223> At least one residue mutated or absent
<400> 213
   acttttcatt ccaatggggt gact 24
<210> 214
   <211> 24
   <212> DNA
   <213> Artificial sequence
<220>
   <223> Exemplary element x variant
<220>
   <221> misc_feature
   <222> (4)..(13)
   <223> At least one residue mutated or absent
<400> 214
   ttccaatggg gtgactttgc ttct 24
<210> 215
   <211> 24
   <212> DNA
   <213> Artificial sequence
<220>
   <223> Exemplary element x variant
<220>
   <221> misc_feature
   <222> (5)..(14)
   <223> At least one residue mutated or absent
<400> 215
   attccaatgg ggtgactttg cttc 24
<210> 216
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 216
   cattccaatg gggtgacttt gctt 24
<210> 217
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 217
   tcattccaat ggggtgactt tgct 24
<210> 218
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 218
   ttcattccaa tggggtgact ttgc 24
<210> 219
   <211> 24
   <212> DNA
   <213> Artificial sequence
<220>
   <223> Exemplary element x variant
<220>
   <221> misc_feature
   <222> (9)..(18)
   <223> At least one residue mutated or absent
<400> 219
   tttcattcca atggggtgac tttg 24
<210> 220
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 220
   ttttcattcc aatggggtga cttt 24
<210> 221
   <211> 24
   <212> DNA
   <213> Artificial sequence
<220>
   <223> Exemplary element X variant
<220>
   <221> misc_feature
   <222> (11)..(20)
   <223> At least one residue mutated or absent
<400> 221
   cttttcattc caatggggtg actt 24
<210> 222
   <211> 24
   <212> DNA
   <213> Artificial sequence
<220>
   <223> Exemplary element X variant
<220>
   <221> misc_feature
   <222> (12)..(21)
   <223> At least one residue mutated or absent
<400> 222
   acttttcatt ccaatggggt gact 24
<210> 223
   <211> 29
   <212> DNA
   <213> Artificial sequence
<220>
   <223> Exemplary element X variant
<220>
   <221> misc_feature
   <222> (4)..(18)
   <223> At least one residue mutated or absent
<400> 223
   tttcattcca atggggtgac tttgcttct 29
<210> 224
   <211> 29
   <212> DNA
   <213> Artificial sequence
<220>
   <223> Exemplary element x variant
<220>
   <221> misc_feature
   <222> (5)..(19)
   <223> At least one residue mutated or absent
<400> 224
   ttttcattcc aatggggtga ctttgcttc 29
<210> 225
   <211> 29
   <212> DNA
   <213> Artificial sequence
<220>
   <223> Exemplary element X variant
<220>
   <221> misc_feature
   <222> (6)..(20)
   <223> At least one residue mutated or absent
<400> 225
   cttttcattc caatggggtg actttgctt 29
<210> 226
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 226
   acttttcatt ccaatggggt gactttgct 29
<210> 227
   <211> 29
   <212> DNA
   <213> Artificial sequence
<220>
   <223> Exemplary element x variant
<220>
   <221> misc_feature
   <222> (8)..(22)
   <223> At least one residue mutated or absent
<400> 227
   tacttttcat tccaatgggg tgactttgc 29
<210> 228
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 228
   atacttttca ttccaatggg gtgactttg 29
<210> 229
   <211> 29
   <212> DNA
   <213> Artificial sequence
<220>
   <223> Exemplary element X variant
<220>
   <221> misc_feature
   <222> (10)..(24)
   <223> At least one residue mutated or absent
<400> 229
   catacttttc attccaatgg ggtgacttt 29
<210> 230
   <211> 29
   <212> DNA
   <213> Artificial sequence
<220>
   <223> Exemplary element X variant
<220>
   <221> misc_feature
   <222> (11)..(25)
   <223> At least one residue mutated or absent
<400> 230
   tcatactttt cattccaatg gggtgactt 29
<210> 231
   <211> 29
   <212> DNA
   <213> Artificial sequence
<220>
   <223> Exemplary element x variant
<220>
   <221> misc_feature
   <222> (12)..(26)
   <223> At least one residue mutated or absent
<400> 231
   ttcatacttt tcattccaat ggggtgact 29
<210> 232
   <211> 26
   <212> DNA
   <213> Artificial sequence
<220>
   <223> Exemplary element X variant
<220>
   <221> misc_feature
   <222> (4)..(8)
   <223> At least one residue mutated or absent
<220>
   <221> misc_feature
   <222> (19)..(23)
   <223> At least one residue mutated or absent
<400> 232
   tttcattcca atggggtgac tttgct 26
<210> 233
   <211> 24
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 233
   cttttcattc caatgacttt gctt 24
<210> 234
   <211> 24
   <212> DNA
   <213> Artificial sequence
<220>
   <223> Exemplary element x variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 234
   cttttcattc gggtgacttt gctt 24
<210> 235
   <211> 24
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(19)
   <223> At least one residue mutated or absent
<400> 235
   ttttcattcc aatgactttg cttc 24
<210> 236
   <211> 24
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(19)
   <223> At least one residue mutated or absent
<400> 236
   ttttcattcg ggtgactttg cttc 24
<210> 237
   <211> 24
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(19)
   <223> At least one residue mutated or absent
<400> 237
   attccaatgg ggtgactttg cttc 24
<210> 238
   <211> 24
   <212> DNA
   <213> Artificial sequence
<220>
   <223> Exemplary element x variant
<220>
   <221> misc_feature
   <222> (4)..(8)
   <223> At least one residue mutated or absent
<220>
   <221> misc_feature
   <222> (14)..(18)
   <223> At least one residue mutated or absent
<400> 238
   ttccaatggg gtgactttgc ttct 24
<210> 239
   <211> 24
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 239
   cattccaatg gggtgacttt gctt 24
<210> 240
   <211> 24
   <212> DNA
   <213> Artificial sequence
<220>
   <223> Exemplary element X variant
<220>
   <221> misc_feature
   <222> (7)..(11)
   <223> At least one residue mutated or absent
<220>
   <221> misc_feature
   <222> (17)..(21)
   <223> At least one residue mutated or absent
<400> 240
   tcattccaat ggggtgactt tgct 24
<210> 241
   <211> 29
   <212> DNA
   <213> Artificial sequence
<220>
   <223> Exemplary element X variant
<220>
   <221> misc_feature
   <222> (4)..(13)
   <223> At least one residue mutated or absent
<220>
   <221> misc_feature
   <222> (19)..(23)
   <223> At least one residue mutated or absent
<400> 241
   tttcattcca atggggtgac tttgcttct 29
<210> 242
   <211> 29
   <212> DNA
   <213> Artificial sequence
<220>
   <223> Exemplary element X variant
<220>
   <221> misc_feature
   <222> (5)..(14)
   <223> At least one residue mutated or absent
<220>
   <221> misc_feature
   <222> (20)..(24)
   <223> At least one residue mutated or absent
<400> 242
   ttttcattcc aatggggtga ctttgcttc 29
<210> 243
   <211> 29
   <212> DNA
   <213> Artificial sequence
<220>
   <223> Exemplary element X variant
<220>
   <221> misc_feature
   <222> (6)..(15)
   <223> At least one residue mutated or absent
<220>
   <221> misc_feature
   <222> (21)..(25)
   <223> At least one residue mutated or absent
<400> 243
   cttttcattc caatggggtg actttgctt 29
<210> 244
   <211> 29
   <212> DNA
   <213> Artificial sequence
<220>
   <223> Exemplary element X variant
<220>
   <221> misc_feature
   <222> (11)..(25)
   <223> At least one residue mutated or absent
<400> 244
   tcatactttt cattccaatg actttgctt 29
<210> 245
   <211> 29
   <212> DNA
   <213> Artificial sequence
<220>
   <223> Exemplary element X variant
<220>
   <221> misc_feature
   <222> (10)..(24)
   <223> At least one residue mutated or absent
<400> 245
   catacttttc attccaatga ctttgcttc 29
<210> 246
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 246
   atacttttca ttccaatgac tttgcttct 29
<210> 247
   <211> 29
   <212> DNA
   <213> Artificial sequence
<220>
   <223> Exemplary element X variant
<220>
   <221> misc_feature
   <222> (8)..(22)
   <223> At least one residue mutated or absent
<400> 247
   tacttttcat tccaatgact ttgcttctg 29
<210> 248
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 248
   acttttcatt ccaatgactt tgcttctgg 29
<210> 249
   <211> 29
   <212> DNA
   <213> Artificial sequence
<220>
   <223> Exemplary element X variant
<220>
   <221> misc_feature
   <222> (4)..(8)
   <223> At least one residue mutated or absent
<220>
   <221> misc_feature
   <222> (14)..(23)
   <223> At least one residue mutated or absent
<400> 249
   tttcattcca atggggtgac tttgcttct 29
<210> 250
   <211> 29
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(24)
   <223> At least one residue mutated or absent
<400> 250
   ttttcattcc aatggggtga ctttgcttc 29
<210> 251
   <211> 29
   <212> DNA
   <213> Artificial sequence
<220>
   <223> Exemplary element x variant
<220>
   <221> misc_feature
   <222> (6)..(10)
   <223> At least one residue mutated or absent
<220>
   <221> misc_feature
   <222> (16)..(25)
   <223> At least one residue mutated or absent
<400> 251
   cttttcattc caatggggtg actttgctt 29
<210> 252
   <211> 29
   <212> DNA
   <213> Artificial sequence
<220>
   <223> Exemplary element X variant
<220>
   <221> misc_feature
   <222> (7)..(11)
   <223> At least one residue mutated or absent
<220>
   <221> misc_feature
   <222> (17)..(26)
   <223> At least one residue mutated or absent
<400> 252
   acttttcatt ccaatggggt gactttgct 29
<210> 253
   <211> 29
   <212> DNA
   <213> Artificial sequence
<220>
   <223> Exemplary element X variant
<220>
   <221> misc_feature
   <222> (12)..(26)
   <223> At least one residue mutated or absent
<400> 253
   ttcatacttt tcattcgggt gactttgct 29
<210> 254
   <211> 29
   <212> DNA
   <213> Artificial sequence
<220>
   <223> Exemplary element X variant
<220>
   <221> misc_feature
   <222> (11)..(25)
   <223> At least one residue mutated or absent
<400> 254
   tcatactttt cattcgggtg actttgctt 29
<210> 255
   <211> 29
   <212> DNA
   <213> Artificial sequence
<220>
   <223> Exemplary element X variant
<220>
   <221> misc_feature
   <222> (10)..(24)
   <223> At least one residue mutated or absent
<400> 255
   catacttttc attcgggtga ctttgcttc 29
<210> 256
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 256
   atacttttca ttcgggtgac tttgcttct 29
<210> 257
   <211> 29
   <212> DNA
   <213> Artificial sequence
<220>
   <223> Exemplary element X variant
<220>
   <221> misc_feature
   <222> (8)..(22)
   <223> At least one residue mutated or absent
<400> 257
   tacttttcat tcgggtgact ttgcttctg 29
<210> 258
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 258
   acttttcatt cgggtgactt tgcttctgg 29
<210> 259
   <211> 29
   <212> DNA
   <213> Artificial sequence
<220>
   <223> Exemplary element X variant
<220>
   <221> misc_feature
   <222> (12)..(26)
   <223> At least one residue mutated or absent
<400> 259
   acttttcatt ccaatggggt gactttgct 29
<210> 260
   <211> 29
   <212> DNA
   <213> Artificial sequence
<220>
   <223> Exemplary element X variant
<220>
   <221> misc_feature
   <222> (11)..(25)
   <223> At least one residue mutated or absent
<400> 260
   cttttcattc caatggggtg actttgctt 29
<210> 261
   <211> 29
   <212> DNA
   <213> Artificial sequence
<220>
   <223> Exemplary element X variant
<220>
   <221> misc_feature
   <222> (10)..(24)
   <223> At least one residue mutated or absent
<400> 261
   ttttcattcc aatggggtga ctttgcttc 29
<210> 262
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 262
   tttcattcca atggggtgac tttgcttct 29
<210> 263
   <211> 29
   <212> DNA
   <213> Artificial sequence
<220>
   <223> Exemplary element X variant
<220>
   <221> misc_feature
   <222> (8)..(22)
   <223> At least one residue mutated or absent
<400> 263
   ttcattccaa tggggtgact ttgcttctg 29
<210> 264
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 264
   tcattccaat ggggtgactt tgcttctgg 29
<210> 265
   <211> 34
   <212> DNA
   <213> Artificial sequence
<220>
   <223> Exemplary element x variant
<220>
   <221> misc_feature
   <222> (12)..(31)
   <223> At least one residue mutated or absent
<400> 265
   ttcatacttt tcattccaat ggggtgactt tgct 34
<210> 266
   <211> 34
   <212> DNA
   <213> Artificial sequence
<220>
   <223> Exemplary element X variant
<220>
   <221> misc_feature
   <222> (11)..(30)
   <223> At least one residue mutated or absent
<400> 266
   tcatactttt cattccaatg gggtgacttt gctt 34
<210> 267
   <211> 34
   <212> DNA
   <213> Artificial sequence
<220>
   <223> Exemplary element x variant
<220>
   <221> misc_feature
   <222> (10)..(29)
   <223> At least one residue mutated or absent
<400> 267
   catacttttc attccaatgg ggtgactttg cttc 34
<210> 268
   <211> 34
   <212> DNA
   <213> Artificial sequence
<220>
   <223> Exemplary element X variant
<220>
   <221> misc_feature
   <222> (9)..(28)
   <223> At least one residue mutated or absent
<400> 268
   atacttttca ttccaatggg gtgactttgc ttct 34
<210> 269
   <211> 34
   <212> DNA
   <213> Artificial sequence
<220>
   <223> Exemplary element X variant
<220>
   <221> misc_feature
   <222> (8)..(27)
   <223> At least one residue mutated or absent
<400> 269
   tacttttcat tccaatgggg tgactttgct tctg 34
<210> 270
   <211> 34
   <212> DNA
   <213> Artificial sequence
<220>
   <223> Exemplary element X variant
<220>
   <221> misc_feature
   <222> (7)..(26)
   <223> At least one residue mutated or absent
<400> 270
   acttttcatt ccaatggggt gactttgctt ctgg 34
<210> 271
   <211> 28
   <212> DNA
   <213> Artificial sequence
<220>
   <223> Exemplary element X variant
<220>
   <221> misc_feature
   <222> (4)..(8)
   <223> At least one residue mutated or absent
<400> 271
   tttcattcca atggggtgac tttgcttc 28
<210> 272
   <211> 24
   <212> DNA
   <213> Artificial sequence
<220>
   <223> Exemplary element X variant
<220>
   <221> misc_feature
   <222> (5)..(9)
   <223> At least one residue mutated or absent
<400> 272
   ttttcattcg ggtgactttg cttc 24
<210> 273
   <211> 24
   <212> DNA
   <213> Artificial sequence
<220>
   <223> Exemplary element X variant
<220>
   <221> misc_feature
   <222> (5)..(9)
   <223> At least one residue mutated or absent
<400> 273
   ttttcattcc aatgactttg cttc 24
<210> 274
   <211> 24
   <212> DNA
   <213> Artificial sequence
<220>
   <223> Exemplary element X variant
<220>
   <221> misc_feature
   <222> (5)..(9)
   <223> At least one residue mutated or absent
<400> 274
   ttttcattcc aatggggtgg cttc 24
<210> 275
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 275
   cttttcattc caatggggtg gcttc 25
<210> 276
   <211> 24
   <212> DNA
   <213> Artificial sequence
<220>
   <223> Exemplary element X variant
<220>
   <221> misc_feature
   <222> (10)..(14)
   <223> At least one residue mutated or absent
<400> 276
   catacttttc attcgggtgg cttc 24
<210> 277
   <211> 24
   <212> DNA
   <213> Artificial sequence
<220>
   <223> Exemplary element X variant
<220>
   <221> misc_feature
   <222> (10)..(14)
   <223> At least one residue mutated or absent
<400> 277
   catacttttc attcactttg cttc 24
<210> 278
   <211> 24
   <212> DNA
   <213> Artificial sequence
<220>
   <223> Exemplary element X variant
<220>
   <221> misc_feature
   <222> (10)..(14)
   <223> At least one residue mutated or absent
<400> 278
   catacttttc attccaatgg cttc 24
<210> 279
   <211> 24
   <212> DNA
   <213> Artificial sequence
<220>
   <223> Exemplary element X variant
<220>
   <221> misc_feature
   <222> (15)..(19)
   <223> At least one residue mutated or absent
<400> 279
   tacttcatac ttttcattcg cttc 24
<210> 280
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 280
   ttccaatggg gtgactttgc ttc 23
<210> 281
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 281
   ttccaatgac tttgcttct 19
<210> 282
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 282
   ttccaatggg gtggcttct 19
<210> 283
   <211> 19
   <212> DNA
   <213> Artificial sequence
<220>
   <223> Exemplary element X variant
<220>
   <221> misc_feature
   <222> (9)..(13)
   <223> At least one residue mutated or absent
<400> 283
   tttcattcca atggcttct 19
<210> 284
   <211> 19
   <212> DNA
   <213> Artificial sequence
<220>
   <223> Exemplary element X variant
<220>
   <221> misc_feature
   <222> (10)..(14)
   <223> At least one residue mutated or absent
<400> 284
   ttttcattcc aatggcttc 19
<210> 285
   <211> 24
   <212> DNA
   <213> Artificial sequence
<220>
   <223> Exemplary element X variant
<220>
   <221> misc_feature
   <222> (5)..(9)
   <223> At least one residue mutated or absent
<400> 285
   attccaatgg ggtgactttg cttc 24
<210> 286
   <211> 20
   <212> DNA
   <213> Artificial sequence
<220>
   <223> Exemplary element x variant
<220>
   <221> misc_feature
   <222> (5)..(9)
   <223> At least one residue mutated or absent
<400> 286
   attccaatga ctttgcttct 20
<210> 287
   <211> 20
   <212> DNA
   <213> Artificial sequence
<220>
   <223> Exemplary element X variant
<220>
   <221> misc_feature
   <222> (5)..(9)
   <223> At least one residue mutated or absent
<400> 287
   attccaatgg ggtggcttct 20
<210> 288
   <211> 28
   <212> DNA
   <213> Artificial sequence
<220>
   <223> Exemplary element X variant
<220>
   <221> misc_feature
   <222> (4)..(13)
   <223> At least one residue mutated or absent
<400> 288
   tttcattcca atggggtgac tttgcttc 28
<210> 289
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 289
   ttttcattcc aatgactttg cttc 24
<210> 290
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 290
   ttttcattcc aatggggtgg cttc 24
<210> 291
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 291
   catacttttc attccaatgg cttc 24
<210> 292
   <211> 28
   <212> DNA
   <213> Artificial sequence
<220>
   <223> Exemplary element X variant
<220>
   <221> misc_feature
   <222> (4)..(13)
   <223> At least one residue mutated or absent
<400> 292
   tttcattcca atggggtgac tttgcttc 28
<210> 293
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 293
   ttttcattcc aatgactttg cttc 24
<210> 294
   <211> 28
   <212> DNA
   <213> Artificial sequence
<220>
   <223> Exemplary element X variant
<220>
   <221> misc_feature
   <222> (4)..(8)
   <223> At least one residue mutated or absent
<400> 294
   tttcattcca atggggtgac tttgcttc 28
<210> 295
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 295
   tttcattcca atggggtggc ttc 23
<210> 296
   <211> 23
   <212> DNA
   <213> Artificial sequence
<220>
   <223> Exemplary element x variant
<220>
   <221> misc_feature
   <222> (4)..(8)
   <223> At least one residue mutated or absent
<400> 296
   tttcattcgg gtgactttgc ttc 23
<210> 297
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 297
   tttcattcgg gtggcttct 19
<210> 298
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 298
   ttttcattcg ggtggcttc 19
<210> 299
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 299
   ttccaatggg gtgactttgc ttc 23
<210> 300
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 300
   attccaatgg ggtggcttc 19
<210> 301
   <211> 19
   <212> DNA
   <213> Artificial sequence
<220>
   <223> Exemplary element x variant
<220>
   <221> misc_feature
   <222> (4)..(8)
   <223> At least one residue mutated or absent
<400> 301
   attcaatggg gtggcttct 19
<210> 302
   <211> 28
   <212> DNA
   <213> Artificial sequence
<220>
   <223> Exemplary element X variant
<220>
   <221> misc_feature
   <222> (4)..(13)
   <223> At least one residue mutated or absent
<400> 302
   tttcattcca atggggtgac tttgcttc 28
<210> 303
   <211> 24
   <212> DNA
   <213> Artificial sequence
<220>
   <223> Exemplary element x variant
<220>
   <221> misc_feature
   <222> (5)..(14)
   <223> At least one residue mutated or absent
<400> 303
   ttttcattcc aatggggtgg cttc 24
<210> 304
   <211> 28
   <212> DNA
   <213> Artificial sequence
<220>
   <223> Exemplary element X variant
<220>
   <221> misc_feature
   <222> (4)..(8)
   <223> At least one residue mutated or absent
<400> 304
   tttcattcca atggggtgac tttgcttc 28
<210> 305
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 305
   tttcattcgg gtgactttgc ttc 23
<210> 306
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 306
   tttcattcca atgactttgc ttc 23
<210> 307
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 307
   ttttcattca ctttgcttc 19
<210> 308
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 308
   tttcattcac tttgcttct 19
<210> 309
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 309
   ttccaatggg gtgactttgc ttc 23
<210> 310
   <211> 19
   <212> DNA
   <213> Artificial sequence
<220>
   <223> Exemplary element X variant
<220>
   <221> misc_feature
   <222> (4)..(8)
   <223> At least one residue mutated or absent
<400> 310
   ttccaatgac tttgcttct 19
<210> 311
   <211> 19
   <212> DNA
   <213> Artificial sequence
<220>
   <223> Exemplary element X variant
<220>
   <221> misc_feature
   <222> (5)..(9)
   <223> At least one residue mutated or absent
<400> 311
   attccaatga ctttgcttc 19
<210> 312
   <211> 28
   <212> DNA
   <213> Artificial sequence
<220>
   <223> Exemplary element X variant
<220>
   <221> misc_feature
   <222> (4)..(13)
   <223> At least one residue mutated or absent
<400> 312
   tttcattcca atggggtgac tttgcttc 28
<210> 313
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 313
   ttttcattcc aatgactttg cttc 24
<210> 314
   <211> 24
   <212> DNA
   <213> Artificial sequence
<220>
   <223> Exemplary element X variant
<220>
   <221> misc_feature
   <222> (4)..(13)
   <223> At least one residue mutated or absent
<400> 314
   tttcattcca atgactttgc ttct 24
<210> 315
   <211> 28
   <212> DNA
   <213> Artificial sequence
<220>
   <223> Exemplary element X variant
<220>
   <221> misc_feature
   <222> (4)..(8)
   <223> At least one residue mutated or absent
<400> 315
   tttcattcca atggggtgac tttgcttc 28
<210> 316
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 316
   tttcattcgg gtgactttgc ttc 23
<210> 317
   <211> 23
   <212> DNA
   <213> Artificial sequence
<220>
   <223> Exemplary element X variant
<220>
   <221> misc_feature
   <222> (4)..(8)
   <223> At least one residue mutated or absent
<400> 317
   ttccaatggg gtgactttgc ttc 23
<210> 318
   <211> 28
   <212> DNA
   <213> Artificial sequence
<220>
   <223> Exemplary element x variant
<220>
   <221> misc_feature
   <222> (4)..(13)
   <223> At least one residue mutated or absent
<400> 318
   tttcattcca atggggtgac tttgcttc 28
<210> 319
   <211> 28
   <212> DNA
   <213> Artificial sequence
<220>
   <223> Exemplary element X variant
<220>
   <221> misc_feature
   <222> (4)..(8)
   <223> At least one residue mutated or absent
<220>
   <221> misc_feature
   <222> (14)..(18)
   <223> At least one residue mutated or absent
<400> 319
   tttcattcca atggggtgac tttgcttc 28
<210> 320
   <211> 24
   <212> DNA
   <213> Artificial sequence
<220>
   <223> Exemplary element X variant
<220>
   <221> misc_feature
   <222> (4)..(13)
   <223> At least one residue mutated or absent
<400> 320
   ttttcattcg ggtgactttg cttc 24
<210> 321
   <211> 24
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(19)
   <223> At least one residue mutated or absent
<400> 321
   ttttcattcc aatggggtgg cttc 24
<210> 322
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 322
   catacttttc attcgggtgg cttc 24
<210> 323
   <211> 24
   <212> DNA
   <213> Artificial sequence
<220>
   <223> Exemplary element x variant
<220>
   <221> misc_feature
   <222> (4)..(13)
   <223> At least one residue mutated or absent
<400> 323
   tttcattcgg gtgactttgc ttct 24
<210> 324
   <211> 24
   <212> DNA
   <213> Artificial sequence
<220>
   <223> Exemplary element X variant
<220>
   <221> misc_feature
   <222> (4)..(8)
   <223> At least one residue mutated or absent
<220>
   <221> misc_feature
   <222> (14)..(18)
   <223> At least one residue mutated or absent
<400> 324
   tttcattcca atggggtggc ttct 24
<210> 325
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 325
   atacttttca ttcgggtggc ttct 24
<210> 326
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 326
   tacttttcat tcgggtggct tctg 24
<210> 327
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 327
   acttttcatt cgggtggctt ctgg 24
<210> 328
   <211> 24
   <212> DNA
   <213> Artificial sequence
<220>
   <223> Exemplary element X variant
<220>
   <221> misc_feature
   <222> (4)..(13)
   <223> At least one residue mutated or absent
<400> 328
   ttccaatggg gtgactttgc ttct 24
<210> 329
   <211> 24
   <212> DNA
   <213> Artificial sequence
<220>
   <223> Exemplary element X variant
<220>
   <221> misc_feature
   <222> (5)..(14)
   <223> At least one residue mutated or absent
<400> 329
   attccaatgg ggtgactttg cttc 24
<210> 330
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 330
   ttttcattcc aatggggtgg cttc 24
<210> 331
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 331
   tttcattcca atggggtggc ttct 24
<210> 332
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 332
   ttcattccaa tggggtggct tctg 24
<210> 333
   <211> 29
   <212> DNA
   <213> Artificial sequence
<220>
   <223> Exemplary element X variant
<220>
   <221> misc_feature
   <222> (7)..(16)
   <223> At least one residue mutated or absent
<400> 333
   tcattccaat ggggtgactt tgcttctgg 29
<210> 334
   <211> 28
   <212> DNA
   <213> Artificial sequence
<220>
   <223> Exemplary element X variant
<220>
   <221> misc_feature
   <222> (4)..(18)
   <223> At least one residue mutated or absent
<400> 334
   tttcattcca atggggtgac tttgcttc 28
<210> 335
   <211> 30
   <212> DNA
   <213> Artificial sequence
<220>
   <223> Exemplary element X variant
<220>
   <221> misc_feature
   <222> (5)..(19)
   <223> At least one residue mutated or absent
<400> 335
   ttttcattcc aatggggtga ctttgcttct 30
<210> 336
   <211> 29
   <212> DNA
   <213> Artificial sequence
<220>
   <223> Exemplary element x variant
<220>
   <221> misc_feature
   <222> (9)..(23)
   <223> At least one residue mutated or absent
<400> 336
   atacttttca ttccaatggg gtggcttct 29
<210> 337
   <211> 29
   <212> DNA
   <213> Artificial sequence
<220>
   <223> Exemplary element X variant
<220>
   <221> misc_feature
   <222> (10)..(24)
   <223> At least one residue mutated or absent
<400> 337
   catacttttc attccaatgg ggtggcttc 29
<210> 338
   <211> 28
   <212> DNA
   <213> Artificial sequence
<220>
   <223> Exemplary element X variant
<220>
   <221> misc_feature
   <222> (4)..(8)
   <223> At least one residue mutated or absent
<220>
   <221> misc_feature
   <222> (19)..(23)
   <223> At least one residue mutated or absent
<400> 338
   tttcattcca atggggtgac tttgcttc 28
<210> 339
   <211> 24
   <212> DNA
   <213> Artificial sequence
<220>
   <223> Exemplary element x variant
<220>
   <221> misc_feature
   <222> (5)..(9)
   <223> At least one residue mutated or absent
<220>
   <221> misc_feature
   <222> (15)..(19)
   <223> At least one residue mutated or absent
<400> 339
   ttttcattcc aatgactttg cttc 24
<210> 340
   <211> 24
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(19)
   <223> At least one residue mutated or absent
<400> 340
   ttttcattcg ggtgactttg cttc 24
<210> 341
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 341
   catacttttc attcactttg cttc 24
<210> 342
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 342
   atacttttca ttcactttgc ttct 24
<210> 343
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 343
   tacttttcat tcactttgct tctg 24
<210> 344
   <211> 24
   <212> DNA
   <213> Artificial sequence
<220>
   <223> Exemplary element x variant
<220>
   <221> misc_feature
   <222> (7)..(16)
   <223> At least one residue mutated or absent
<400> 344
   acttttcatt cactttgctt ctgg 24
<210> 345
   <211> 24
   <212> DNA
   <213> Artificial sequence
<220>
   <223> Exemplary element X variant
<220>
   <221> misc_feature
   <222> (4)..(8)
   <223> At least one residue mutated or absent
<220>
   <221> misc_feature
   <222> (14)..(18)
   <223> At least one residue mutated or absent
<400> 345
   ttccaatggg gtgactttgc ttct 24
<210> 346
   <211> 24
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(19)
   <223> At least one residue mutated or absent
<400> 346
   attccaatgg ggtgactttg cttc 24
<210> 347
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 347
   tttcattcca atgactttgc ttct 24
<210> 348
   <211> 24
   <212> DNA
   <213> Artificial sequence
<220>
   <223> Exemplary element x variant
<220>
   <221> misc_feature
   <222> (10)..(19)
   <223> At least one residue mutated or absent
<400> 348
   ttttcattcc aatgactttg cttc 24
<210> 349
   <211> 29
   <212> DNA
   <213> Artificial sequence
<220>
   <223> Exemplary element X variant
<220>
   <221> misc_feature
   <222> (5)..(14)
   <223> At least one residue mutated or absent
<220>
   <221> misc_feature
   <222> (20)..(24)
   <223> At least one residue mutated or absent
<400> 349
   ttttcattcc aatggggtga ctttgcttc 29
<210> 350
   <211> 29
   <212> DNA
   <213> Artificial sequence
<220>
   <223> Exemplary element X variant
<220>
   <221> misc_feature
   <222> (4)..(13)
   <223> At least one residue mutated or absent
<220>
   <221> misc_feature
   <222> (19)..(23)
   <223> At least one residue mutated or absent
<400> 350
   tttcattcca atggggtgac tttgcttct 29
<210> 351
   <211> 29
   <212> DNA
   <213> Artificial sequence
<220>
   <223> Exemplary element X variant
<220>
   <221> misc_feature
   <222> (10)..(24)
   <223> At least one residue mutated or absent
<400> 351
   catacttttc attccaatga ctttgcttc 29
<210> 352
   <211> 29
   <212> DNA
   <213> Artificial sequence
<220>
   <223> Exemplary element x variant
<220>
   <221> misc_feature
   <222> (9)..(23)
   <223> At least one residue mutated or absent
<400> 352
   atacttttca ttccaatgac tttgcttct 29
<210> 353
   <211> 29
   <212> DNA
   <213> Artificial sequence
<220>
   <223> Exemplary element x variant
<220>
   <221> misc_feature
   <222> (8)..(22)
   <223> At least one residue mutated or absent
<400> 353
   tacttttcat tccaatgact ttgcttctg 29
<210> 354
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 354
   acttttcatt ccaatgactt tgcttctgg 29
<210> 355
   <211> 29
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(24)
   <223> At least one residue mutated or absent
<400> 355
   ttttcattcc aatggggtga ctttgcttc 29
<210> 356
   <211> 29
   <212> DNA
   <213> Artificial sequence
<220>
   <223> Exemplary element X variant
<220>
   <221> misc_feature
   <222> (5)..(9)
   <223> At least one residue mutated or absent
<220>
   <221> misc_feature
   <222> (15)..(24)
   <223> At least one residue mutated or absent
<400> 356
   ttttcattcc aatggggtga ctttgcttc 29
<210> 357
   <211> 24
   <212> DNA
   <213> Artificial sequence
<220>
   <223> Exemplary element X variant
<220>
   <221> misc_feature
   <222> (4)..(18)
   <223> At least one residue mutated or absent
<400> 357
   tttcattcgg gtgactttgc ttct 24
<210> 358
   <211> 29
   <212> DNA
   <213> Artificial sequence
<220>
   <223> Exemplary element X variant
<220>
   <221> misc_feature
   <222> (10)..(24)
   <223> At least one residue mutated or absent
<400> 358
   catacttttc attcgggtga ctttgcttc 29
<210> 359
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 359
   atacttttca ttcgggtgac tttgcttct 29
<210> 360
   <211> 29
   <212> DNA
   <213> Artificial sequence
<220>
   <223> Exemplary element x variant
<220>
   <221> misc_feature
   <222> (8)..(22)
   <223> At least one residue mutated or absent
<400> 360
   tacttttcat tcgggtgact ttgcttctg 29
<210> 361
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 361
   acttttcatt cgggtgactt tgcttctgg 29
<210> 362
   <211> 29
   <212> DNA
   <213> Artificial sequence
<220>
   <223> Exemplary element X variant
<220>
   <221> misc_feature
   <222> (10)..(24)
   <223> At least one residue mutated or absent
<400> 362
   ttttcattcc aatggggtga ctttgcttc 29
<210> 363
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 363
   tttcattcca atggggtgac tttgcttct 29
<210> 364
   <211> 29
   <212> DNA
   <213> Artificial sequence
<220>
   <223> Exemplary element X variant
<220>
   <221> misc_feature
   <222> (8)..(22)
   <223> At least one residue mutated or absent
<400> 364
   ttcattccaa tggggtgact ttgcttctg 29
<210> 365
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 365
   tcattccaat ggggtgactt tgcttctgg 29
<210> 366
   <211> 34
   <212> DNA
   <213> Artificial sequence
<220>
   <223> Exemplary element X variant
<220>
   <221> misc_feature
   <222> (7)..(26)
   <223> At least one residue mutated or absent
<400> 366
   acttttcatt ccaatggggt gactttgctt ctgg 34
<210> 367
   <211> 34
   <212> DNA
   <213> Artificial sequence
<220>
   <223> Exemplary element x variant
<220>
   <221> misc_feature
   <222> (8)..(27)
   <223> At least one residue mutated or absent
<400> 367
   tacttttcat tccaatgggg tgactttgct tctg 34
<210> 368
   <211> 34
   <212> DNA
   <213> Artificial sequence
<220>
   <223> Exemplary element X variant
<220>
   <221> misc_feature
   <222> (9)..(28)
   <223> At least one residue mutated or absent
<400> 368
   atacttttca ttccaatggg gtgactttgc ttct 34
<210> 369
   <211> 34
   <212> DNA
   <213> Artificial sequence
<220>
   <223> Exemplary element x variant
<220>
   <221> misc_feature
   <222> (10)..(29)
   <223> At least one residue mutated or absent
<400> 369
   catacttttc attccaatgg ggtgactttg cttc 34
<210> 370
   <211> 25
   <212> DNA
   <213> Artificial sequence
<220>
   <223> Exemplary element X variant
<220>
   <221> misc_feature
   <222> (6)..(15)
   <223> At least one residue mutated or absent
<400> 370
   cttttcattc caatggggtg acttt 25
<210> 371
   <211> 25
   <212> DNA
   <213> Artificial sequence
<220>
   <223> Exemplary element x variant
<220>
   <221> misc_feature
   <222> (7)..(16)
   <223> At least one residue mutated or absent
<400> 371
   acttttcatt ccaatggggt gactt 25
<210> 372
   <211> 25
   <212> DNA
   <213> Artificial sequence
<220>
   <223> Exemplary element X variant
<220>
   <221> misc_feature
   <222> (8)..(17)
   <223> At least one residue mutated or absent
<400> 372
   tacttttcat tccaatgggg tgact 25
<210> 373
   <211> 25
   <212> DNA
   <213> Artificial sequence
<220>
   <223> Exemplary element X variant
<220>
   <221> misc_feature
   <222> (9)..(18)
   <223> At least one residue mutated or absent
<400> 373
   atacttttca ttccaatggg gtgac 25
<210> 374
   <211> 25
   <212> DNA
   <213> Artificial sequence
<220>
   <223> Exemplary element X variant
<220>
   <221> misc_feature
   <222> (10)..(19)
   <223> At least one residue mutated or absent
<400> 374
   catacttttc attccaatgg ggtga 25
<210> 375
   <211> 25
   <212> DNA
   <213> Artificial sequence
<220>
   <223> Exemplary element X variant
<220>
   <221> misc_feature
   <222> (11)..(20)
   <223> At least one residue mutated or absent
<400> 375
   tcatactttt cattccaatg gggtg 25
<210> 376
   <211> 25
   <212> DNA
   <213> Artificial sequence
<220>
   <223> Exemplary element X variant
<220>
   <221> misc_feature
   <222> (12)..(21)
   <223> At least one residue mutated or absent
<400> 376
   ttcatacttt tcattccaat ggggt 25
<210> 377
   <211> 25
   <212> DNA
   <213> Artificial sequence
<220>
   <223> Exemplary element X variant
<220>
   <221> misc_feature
   <222> (13)..(22)
   <223> At least one residue mutated or absent
<400> 377
   cttcatactt ttcattccaa tgggg 25
<210> 378
   <211> 19
   <212> DNA
   <213> Artificial sequence
<220>
   <223> Exemplary element X variant
<220>
   <221> misc_feature
   <222> (12)..(16)
   <223> At least one residue mutated or absent
<400> 378
   ttcatacttt tcattccaa 19
<210> 379
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 379
   tcatactttt cattccaat 19
<210> 380
   <211> 19
   <212> DNA
   <213> Artificial sequence
<220>
   <223> Exemplary element X variant
<220>
   <221> misc_feature
   <222> (10)..(14)
   <223> At least one residue mutated or absent
<400> 380
   catacttttc attccaatg 19
<210> 381
   <211> 19
   <212> DNA
   <213> Artificial sequence
<220>
   <223> Exemplary element X variant
<220>
   <221> misc_feature
   <222> (9)..(13)
   <223> At least one residue mutated or absent
<400> 381
   atacttttca ttccaatgg 19
<210> 382
   <211> 19
   <212> DNA
   <213> Artificial sequence
<220>
   <223> Exemplary element X variant
<220>
   <221> misc_feature
   <222> (8)..(12)
   <223> At least one residue mutated or absent
<400> 382
   tacttttcat tccaatggg 19
<210> 383
   <211> 19
   <212> DNA
   <213> Artificial sequence
<220>
   <223> Exemplary element X variant
<220>
   <221> misc_feature
   <222> (7)..(11)
   <223> At least one residue mutated or absent
<400> 383
   acttttcatt ccaatgggg 19
<210> 384
   <211> 19
   <212> DNA
   <213> Artificial sequence
<220>
   <223> Exemplary element X variant
<220>
   <221> misc_feature
   <222> (6)..(10)
   <223> At least one residue mutated or absent
<400> 384
   cttttcattc caatggggt 19
<210> 385
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 385
   cttttcattc caatggggtg 20
<210> 386
   <211> 20
   <212> DNA
   <213> Artificial sequence
<220>
   <223> Exemplary element x variant
<220>
   <221> misc_feature
   <222> (10)..(14)
   <223> At least one residue mutated or absent
<400> 386
   catacttttc attcgggtga 20
<210> 387
   <211> 20
   <212> DNA
   <213> Artificial sequence
<220>
   <223> Exemplary element x variant
<220>
   <221> misc_feature
   <222> (9)..(13)
   <223> At least one residue mutated or absent
<400> 387
   atacttttca ttcgggtgac 20
<210> 388
   <211> 20
   <212> DNA
   <213> Artificial sequence
<220>
   <223> Exemplary element X variant
<220>
   <221> misc_feature
   <222> (8)..(12)
   <223> At least one residue mutated or absent
<400> 388
   tacttttcat tcgggtgact 20
<210> 389
   <211> 20
   <212> DNA
   <213> Artificial sequence
<220>
   <223> Exemplary element X variant
<220>
   <221> misc_feature
   <222> (7)..(11)
   <223> At least one residue mutated or absent
<400> 389
   acttttcatt cgggtgactt 20
<210> 390
   <211> 20
   <212> DNA
   <213> Artificial sequence
<220>
   <223> Exemplary element x variant
<220>
   <221> misc_feature
   <222> (6)..(10)
   <223> At least one residue mutated or absent
<400> 390
   cttttcattc gggtgacttt 20
<210> 391
   <211> 20
   <212> DNA
   <213> Artificial sequence
<220>
   <223> Exemplary element X variant
<220>
   <221> misc_feature
   <222> (11)..(15)
   <223> At least one residue mutated or absent
<400> 391
   cttttcattc caatggggtg 20
<210> 392
   <211> 19
   <212> DNA
   <213> Artificial sequence
<220>
   <223> Exemplary element X variant
<220>
   <221> misc_feature
   <222> (10)..(14)
   <223> At least one residue mutated or absent
<400> 392
   catacttttc aatggggtg 19
<210> 393
   <211> 19
   <212> DNA
   <213> Artificial sequence
<220>
   <223> Exemplary element x variant
<220>
   <221> misc_feature
   <222> (9)..(13)
   <223> At least one residue mutated or absent
<400> 393
   atacttttca atggggtga 19
<210> 394
   <211> 19
   <212> DNA
   <213> Artificial sequence
<220>
   <223> Exemplary element x variant
<220>
   <221> misc_feature
   <222> (8)..(12)
   <223> At least one residue mutated or absent
<400> 394
   tacttttcaa tggggtgac 19
<210> 395
   <211> 19
   <212> DNA
   <213> Artificial sequence
<220>
   <223> Exemplary element X variant
<220>
   <221> misc_feature
   <222> (7)..(11)
   <223> At least one residue mutated or absent
<400> 395
   acttttcaat ggggtgact 19
<210> 396
   <211> 19
   <212> DNA
   <213> Artificial sequence
<220>
   <223> Exemplary element x variant
<220>
   <221> misc_feature
   <222> (6)..(10)
   <223> At least one residue mutated or absent
<400> 396
   cttttcaatg gggtgactt 19
<210> 397
   <211> 24
   <212> DNA
   <213> Artificial sequence
<220>
   <223> Exemplary element x variant
<220>
   <221> misc_feature
   <222> (10)..(19)
   <223> At least one residue mutated or absent
<400> 397
   catacttttc attccaatgg ggtg 24
<210> 398
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 398
   atacttttca ttccaatggg gtga 24
<210> 399
   <211> 24
   <212> DNA
   <213> Artificial sequence
<220>
   <223> Exemplary element x variant
<220>
   <221> misc_feature
   <222> (8)..(17)
   <223> At least one residue mutated or absent
<400> 399
   tacttttcat tccaatgggg tgac 24
<210> 400
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 400
   acttttcatt ccaatggggt gact 24
<210> 401
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 401
   cttttcattc caatggggtg actt 24
<210> 402
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 402
   cttttcattc caatggggtg acttt 25
<210> 403
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 403
   cttttcattc caatgacttt 20
<210> 404
   <211> 20
   <212> DNA
   <213> Artificial sequence
<220>
   <223> Exemplary element X variant
<220>
   <221> misc feature
   <222> (6)..(10)
   <223> At least one residue mutated or absent
<400> 404
   cttttcattc gggtgacttt 20
<210> 405
   <211> 19
   <212> DNA
   <213> Artificial sequence
<220>
   <223> Exemplary element X variant
<220>
   <221> misc_feature
   <222> (10)..(14)
   <223> At least one residue mutated or absent
<400> 405
   catacttttc attcacttt 19
<210> 406
   <211> 19
   <212> DNA
   <213> Artificial sequence
<220>
   <223> Exemplary element X variant
<220>
   <221> misc_feature
   <222> (9)..(13)
   <223> At least one residue mutated or absent
<400> 406
   atacttttca ttcactttg 19
<210> 407
   <211> 19
   <212> DNA
   <213> Artificial sequence
<220>
   <223> Exemplary element X variant
<220>
   <221> misc_feature
   <222> (8)..(12)
   <223> At least one residue mutated or absent
<400> 407
   tacttttcat tcactttgc 19
<210> 408
   <211> 19
   <212> DNA
   <213> Artificial sequence
<220>
   <223> Exemplary element X variant
<220>
   <221> misc_feature
   <222> (7)..(11)
   <223> At least one residue mutated or absent
<400> 408
   acttttcatt cactttgct 19
<210> 409
   <211> 19
   <212> DNA
   <213> Artificial sequence
<220>
   <223> Exemplary element X variant
<220>
   <221> misc_feature
   <222> (6)..(10)
   <223> At least one residue mutated or absent
<400> 409
   cttttcattc actttgctt 19
<210> 410
   <211> 25
   <212> DNA
   <213> Artificial sequence
<220>
   <223> Exemplary element X variant
<220>
   <221> misc_feature
   <222> (11)..(15)
   <223> At least one residue mutated or absent
<400> 410
   cttttcattc caatggggtg acttt 25
<210> 411
   <211> 20
   <212> DNA
   <213> Artificial sequence
<220>
   <223> Exemplary element X variant
<220>
   <221> misc_feature
   <222> (11)..(15)
   <223> At least one residue mutated or absent
<400> 411
   cttttcattc caatgacttt 20
<210> 412
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 412
   cttttcaatg gggtgacttt 20
<210> 413
   <211> 19
   <212> DNA
   <213> Artificial sequence
<220>
   <223> Exemplary element X variant
<220>
   <221> misc_feature
   <222> (10)..(14)
   <223> At least one residue mutated or absent
<400> 413
   catacttttc aatgacttt 19
<210> 414
   <211> 19
   <212> DNA
   <213> Artificial sequence
<220>
   <223> Exemplary element X variant
<220>
   <221> misc_feature
   <222> (9)..(13)
   <223> At least one residue mutated or absent
<400> 414
   atacttttca atgactttg 19
<210> 415
   <211> 19
   <212> DNA
   <213> Artificial sequence
<220>
   <223> Exemplary element X variant
<220>
   <221> misc_feature
   <222> (8)..(12)
   <223> At least one residue mutated or absent
<400> 415
   tacttttcaa tgactttgc 19
<210> 416
   <211> 19
   <212> DNA
   <213> Artificial sequence
<220>
   <223> Exemplary element X variant
<220>
   <221> misc_feature
   <222> (7)..(11)
   <223> At least one residue mutated or absent
<400> 416
   acttttcaat gactttgct 19
<210> 417
   <211> 19
   <212> DNA
   <213> Artificial sequence
<220>
   <223> Exemplary element X variant
<220>
   <221> misc_feature
   <222> (6)..(10)
   <223> At least one residue mutated or absent
<400> 417
   cttttcaatg actttgctt 19
<210> 418
   <211> 25
   <212> DNA
   <213> Artificial sequence
<220>
   <223> Exemplary element X variant
<220>
   <221> misc_feature
   <222> (6)..(15)
   <223> At least one residue mutated or absent
<400> 418
   cttttcattc caatggggtg acttt 25
<210> 419
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 419
   catacttttc attccaatga cttt 24
<210> 420
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 420
   atacttttca ttccaatgac tttg 24
<210> 421
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 421
   tacttttcat tccaatgact ttgc 24
<210> 422
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 422
   acttttcatt ccaatgactt tgct 24
<210> 423
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 423
   cttttcattc caatgacttt gctt 24
<210> 424
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 424
   cttttcattc caatggggtg acttt 25
<210> 425
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 425
   cttttcattc gggtgacttt 20
<210> 426
   <211> 25
   <212> DNA
   <213> Artificial sequence
<220>
   <223> Exemplary element X variant
<220>
   <221> misc_feature
   <222> (6)..(15)
   <223> At least one residue mutated or absent
<400> 426
   cttttcattc caatggggtg acttt 25
<210> 427
   <211> 24
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 427
   cttttcattc caatggggtg actt 24
<210> 428
   <211> 24
   <212> DNA
   <213> Artificial sequence
<220>
   <223> Exemplary element x variant
<220>
   <221> misc_feature
   <222> (7)..(11)
   <223> At least one residue mutated or absent
<220>
   <221> misc_feature
   <222> (17)..(21)
   <223> At least one residue mutated or absent
<400> 428
   acttttcatt ccaatggggt gact 24
<210> 429
   <211> 24
   <212> DNA
   <213> Artificial sequence
<220>
   <223> Exemplary element X variant
<220>
   <221> misc_feature
   <222> (12)..(21)
   <223> At least one residue mutated or absent
<400> 429
   ttcatacttt tcattcgggt gact 24
<210> 430
   <211> 24
   <212> DNA
   <213> Artificial sequence
<220>
   <223> Exemplary element X variant
<220>
   <221> misc_feature
   <222> (11)..(20)
   <223> At least one residue mutated or absent
<400> 430
   tcatactttt cattcgggtg actt 24
<210> 431
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 431
   catacttttc attcgggtga cttt 24
<210> 432
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 432
   atacttttca ttcgggtgac tttg 24
<210> 433
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 433
   tacttttcat tcgggtgact ttgc 24
<210> 434
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 434
   acttttcatt cgggtgactt tgct 24
<210> 435
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 435
   cttttcattc gggtgacttt gctt 24
<210> 436
   <211> 24
   <212> DNA
   <213> Artificial sequence
<220>
   <223> Exemplary element X variant
<220>
   <221> misc_feature
   <222> (11)..(20)
   <223> At least one residue mutated or absent
<400> 436
   cttttcattc caatggggtg actt 24
<210> 437
   <211> 24
   <212> DNA
   <213> Artificial sequence
<220>
   <223> Exemplary element X variant
<220>
   <221> misc_feature
   <222> (12)..(21)
   <223> At least one residue mutated or absent
<400> 437
   acttttcatt ccaatggggt gact 24
<210> 438
   <211> 24
   <212> DNA
   <213> Artificial sequence
<220>
   <223> Exemplary element X variant
<220>
   <221> misc_feature
   <222> (12)..(21)
   <223> At least one residue mutated or absent
<400> 438
   ttcatacttt tcaatggggt gact 24
<210> 439
   <211> 24
   <212> DNA
   <213> Artificial sequence
<220>
   <223> Exemplary element X variant
<220>
   <221> misc_feature
   <222> (11)..(20)
   <223> At least one residue mutated or absent
<400> 439
   tcatactttt caatggggtg actt 24
<210> 440
   <211> 24
   <212> DNA
   <213> Artificial sequence
<220>
   <223> Exemplary element x variant
<220>
   <221> misc_feature
   <222> (10)..(19)
   <223> At least one residue mutated or absent
<400> 440
   catacttttc aatggggtga cttt 24
<210> 441
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 441
   atacttttca atggggtgac tttg 24
<210> 442
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 442
   tacttttcaa tggggtgact ttgc 24
<210> 443
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 443
   acttttcaat ggggtgactt tgct 24
<210> 444
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 444
   cttttcaatg gggtgacttt gctt 24
<210> 445
   <211> 29
   <212> DNA
   <213> Artificial sequence
<220>
   <223> Exemplary element X variant
<220>
   <221> misc_feature
   <222> (12)..(26)
   <223> At least one residue mutated or absent
<400> 445
   ttcatacttt tcattccaat ggggtgact 29
<210> 446
   <211> 29
   <212> DNA
   <213> Artificial sequence
<220>
   <223> Exemplary element X variant
<220>
   <221> misc_feature
   <222> (11)..(25)
   <223> At least one residue mutated or absent
<400> 446
   tcatactttt cattccaatg gggtgactt 29
<210> 447
   <211> 29
   <212> DNA
   <213> Artificial sequence
<220>
   <223> Exemplary element X variant
<220>
   <221> misc_feature
   <222> (10)..(24)
   <223> At least one residue mutated or absent
<400> 447
   catacttttc attccaatgg ggtgacttt 29
<210> 448
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 448
   atacttttca ttccaatggg gtgactttg 29
<210> 449
   <211> 29
   <212> DNA
   <213> Artificial sequence
<220>
   <223> Exemplary element x variant
<220>
   <221> misc_feature
   <222> (8)..(22)
   <223> At least one residue mutated or absent
<400> 449
   tacttttcat tccaatgggg tgactttgc 29
<210> 450
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 450
   acttttcatt ccaatggggt gactttgct 29
<210> 451
   <211> 29
   <212> DNA
   <213> Artificial sequence
<220>
   <223> Exemplary element X variant
<220>
   <221> misc_feature
   <222> (6)..(20)
   <223> At least one residue mutated or absent
<400> 451
   cttttcattc caatggggtg actttgctt 29
<210> 452
   <211> 28
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (21)..(25)
   <223> At least one residue mutated or absent
<400> 452
   cttttcattc caatggggtg actttgct 28
<210> 453
   <211> 24
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc feature
   <222> (16)..(20)
   <223> At least one residue mutated or absent
<400> 453
   cttttcattc gggtgacttt gctt 24
<210> 454
   <211> 24
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 454
   cttttcattc caatgacttt gctt 24
<210> 455
   <211> 24
   <212> DNA
   <213> Artificial sequence
<220>
   <223> Exemplary element X variant
<220>
   <221> misc_feature
   <222> (11)..(20)
   <223> At least one residue mutated or absent
<400> 455
   tcatactttt cattcacttt gctt 24
<210> 456
   <211> 24
   <212> DNA
   <213> Artificial sequence
<220>
   <223> Exemplary element X variant
<220>
   <221> misc_feature
   <222> (7)..(11)
   <223> At least one residue mutated or absent
<220>
   <221> misc_feature
   <222> (17)..(21)
   <223> At least one residue mutated or absent
<400> 456
   acttttcatt cgggtgactt tgct 24
<210> 457
   <211> 24
   <212> DNA
   <213> Artificial sequences
<220>
   <223> Exemplary element X variant
<220>
   <221> misc_feature
   <222> (7)..(11)
   <223> At least one residue mutated or absent
<220>
   <221> misc_feature
   <222> (17)..(21)
   <223> At least one residue mutated or absent
<400> 457
   acttttcatt ccaatgactt tgct 24
<210> 458
   <211> 24
   <212> DNA
   <213> Artificial sequence
<220>
   <223> Exemplary element X variant
<220>
   <221> misc_feature
   <222> (12)..(21)
   <223> At least one residue mutated or absent
<400> 458
   ttcatacttt tcattcactt tgct 24
<210> 459
   <211> 28
   <212> DNA
   <213> Artificial sequence
<220>
   <223> Exemplary element X variant
<220>
   <221> misc_feature
   <222> (11)..(15)
   <223> At least one residue mutated or absent
<220>
   <221> misc_feature
   <222> (21)..(25)
   <223> At least one residue mutated or absent
<400> 459
   cttttcattc caatggggtg actttgct 28
<210> 460
   <211> 24
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 460
   cttttcaatg gggtgacttt gctt 24
<210> 461
   <211> 24
   <212> DNA
   <213> Artificial sequence
<220>
   <223> Exemplary element X variant
<220>
   <221> misc_feature
   <222> (11)..(20)
   <223> At least one residue mutated or absent
<400> 461
   cttttcattc caatgacttt gctt 24
<210> 462
   <211> 24
   <212> DNA
   <213> Artificial sequence
<220>
   <223> Exemplary element X variant
<220>
   <221> misc_feature
   <222> (11)..(20)
   <223> At least one residue mutated or absent
<400> 462
   tcatactttt caatgacttt gctt 24
<210> 463
   <211> 24
   <212> DNA
   <213> Artificial sequence
<220>
   <223> Exemplary element X variant
<220>
   <221> misc_feature
   <222> (7)..(11)
   <223> At least one residue mutated or absent
<220>
   <221> misc_feature
   <222> (17)..(21)
   <223> At least one residue mutated or absent
<400> 463
   acttttcaat ggggtgactt tgct 24
<210> 464
   <211> 24
   <212> DNA
   <213> Artificial sequence
<220>
   <223> Exemplary element X variant
<220>
   <221> misc_feature
   <222> (12)..(21)
   <223> At least one residue mutated or absent
<400> 464
   acttttcatt ccaatgactt tgct 24
<210> 465
   <211> 24
   <212> DNA
   <213> Artificial sequence
<220>
   <223> Exemplary element X variant
<220>
   <221> misc_feature
   <222> (12)..(21)
   <223> At least one residue mutated or absent
<400> 465
   ttcatacttt tcaatgactt tgct 24
<210> 466
   <211> 29
   <212> DNA
   <213> Artificial sequence
<220>
   <223> Exemplary element X variant
<220>
   <221> misc_feature
   <222> (6)..(15)
   <223> At least one residue mutated or absent
<220>
   <221> misc_feature
   <222> (21)..(25)
   <223> At least one residue mutated or absent
<400> 466
   cttttcattc caatggggtg actttgctt 29
<210> 467
   <211> 29
   <212> DNA
   <213> Artificial sequence
<220>
   <223> Exemplary element X variant
<220>
   <221> misc_feature
   <222> (7)..(16)
   <223> At least one residue mutated or absent
<220>
   <221> misc_feature
   <222> (22)..(26)
   <223> At least one residue mutated or absent
<400> 467
   acttttcatt ccaatggggt gactttgct 29
<210> 468
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 468
   acttttcatt ccaatgactt tgcttctgg 29
<210> 469
   <211> 29
   <212> DNA
   <213> Artificial sequence
<220>
   <223> Exemplary element X variant
<220>
   <221> misc_feature
   <222> (6)..(20)
   <223> At least one residue mutated or absent
<400> 469
   cttttcattc caatgacttt gcttctgga 29
<210> 470
   <211> 29
   <212> DNA
   <213> Artificial sequence
<220>
   <223> Exemplary element X variant
<220>
   <221> misc_feature
   <222> (11)..(25)
   <223> At least one residue mutated or absent
<400> 470
   tcatactttt cattccaatg actttgctt 29
<210> 471
   <211> 29
   <212> DNA
   <213> Artificial sequence
<220>
   <223> Exemplary element X variant
<220>
   <221> misc_feature
   <222> (12)..(26)
   <223> At least one residue mutated or absent
<400> 471
   ttcatacttt tcattccaat gactttgct 29
<210> 472
   <211> 29
   <212> DNA
   <213> Artificial sequence
<220>
   <223> Exemplary element X variant
<220>
   <221> misc feature
   <222> (11)..(25)
   <223> At least one residue mutated or absent
<400> 472
   cttttcattc caatggggtg actttgctt 29
<210> 473
   <211> 29
   <212> DNA
   <213> Artificial sequence
<220>
   <223> Exemplary element X variant
<220>
   <221> misc_feature
   <222> (12)..(26)
   <223> At least one residue mutated or absent
<400> 473
   acttttcatt ccaatggggt gactttgct 29
<210> 474
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 474
   acttttcaat ggggtgactt tgcttctgg 29
<210> 475
   <211> 29
   <212> DNA
   <213> Artificial sequence
<220>
   <223> Exemplary element X variant
<220>
   <221> misc_feature
   <222> (6)..(20)
   <223> At least one residue mutated or absent
<400> 475
   cttttcaatg gggtgacttt gcttctgga 29
<210> 476
   <211> 29
   <212> DNA
   <213> Artificial sequence
<220>
   <223> Exemplary element X variant
<220>
   <221> misc_feature
   <222> (11)..(25)
   <223> At least one residue mutated or absent
<400> 476
   tcatactttt caatggggtg actttgctt 29
<210> 477
   <211> 29
   <212> DNA
   <213> Artificial sequence
<220>
   <223> Exemplary element X variant
<220>
   <221> misc_feature
   <222> (12)..(26)
   <223> At least one residue mutated or absent
<400> 477
   ttcatacttt tcaatggggt gactttgct 29
<210> 478
   <211> 34
   <212> DNA
   <213> Artificial sequence
<220>
   <223> Exemplary element X variant
<220>
   <221> misc_feature
   <222> (7)..(26)
   <223> At least one residue mutated or absent
<400> 478
   acttttcatt ccaatggggt gactttgctt ctgg 34
<210> 479
   <211> 34
   <212> DNA
   <213> Artificial sequence
<220>
   <223> Exemplary element X variant
<220>
   <221> misc_feature
   <222> (8)..(27)
   <223> At least one residue mutated or absent
<400> 479
   tacttttcat tccaatgggg tgactttgct tctg 34
<210> 480
   <211> 34
   <212> DNA
   <213> Artificial sequence
<220>
   <223> Exemplary element X variant
<220>
   <221> misc_feature
   <222> (9)..(28)
   <223> At least one residue mutated or absent
<400> 480
   atacttttca ttccaatggg gtgactttgc ttct 34
<210> 481
   <211> 34
   <212> DNA
   <213> Artificial sequence
<220>
   <223> Exemplary element X variant
<220>
   <221> misc_feature
   <222> (10)..(29)
   <223> At least one residue mutated or absent
<400> 481
   catacttttc attccaatgg ggtgactttg cttc 34
<210> 482
   <211> 34
   <212> DNA
   <213> Artificial sequence
<220>
   <223> Exemplary element X variant
<220>
   <221> misc_feature
   <222> (11)..(30)
   <223> At least one residue mutated or absent
<400> 482
   tcatactttt cattccaatg gggtgacttt gctt 34
<210> 483
   <211> 34
   <212> DNA
   <213> Artificial sequence
<220>
   <223> Exemplary element X variant
<220>
   <221> misc_feature
   <222> (12)..(31)
   <223> At least one residue mutated or absent
<400> 483
   ttcatacttt tcattccaat ggggtgactt tgct 34
<210> 484
   <211> 30
   <212> DNA
   <213> Artificial sequence
<220>
   <223> Exemplary element X variant
<220>
   <221> misc_feature
   <222> (6)..(10)
   <223> At least one residue mutated or absent
<400> 484
   cttttcattc caatggggtg actttgcttc 30
<210> 485
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 485
   cttttcattc gggtgacttt gcttc 25
<210> 486
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 486
   cttttcattc caatgacttt gcttc 25
<210> 487
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 487.
   cttttcattc caatggggtg gcttc 25
<210> 488
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 488
   cttttcattc actttgcttc 20
<210> 489
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 489
   cttttcattc caatggcttc 20
<210> 490
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 490
   cttttcattc gggtggcttc 20
<210> 491
   <211> 19
   <212> DNA
   <213> Artificial sequence
<220>
   <223> Exemplary element X variant
<220>
   <221> misc feature
   <222> (6)..(10)
   <223> At least one residue mutated or absent
<400> 491
   cttttcattc gcttctgga 19
<210> 492
   <211> 19
   <212> DNA
   <213> Artificial sequence
<220>
   <223> Exemplary element X variant
<220>
   <221> mise_feature
   <222> (7)..(11)
   <223> At least one residue mutated or absent
<400> 492
   acttttcatt cgcttctgg 19
<210> 493
   <211> 19
   <212> DNA
   <213> Artificial sequence
<220>
   <223> Exemplary element X variant
<220>
   <221> misc_feature
   <222> (8)..(12)
   <223> At least one residue mutated or absent
<400> 493
   tacttttcat tcgcttctg 19
<210> 494
   <211> 19
   <212> DNA
   <213> Artificial sequence
<220>
   <223> Exemplary element X variant
<220>
   <221> misc_feature
   <222> (9)..(13)
   <223> At least one residue mutated or absent
<400> 494
   atacttttca ttcgcttct 19
<210> 495
   <211> 19
   <212> DNA
   <213> Artificial sequence
<220>
   <223> Exemplary element X variant
<220>
   <221> misc_feature
   <222> (10)..(14)
   <223> At least one residue mutated or absent
<400> 495
   catacttttc attcgcttc 19
<210> 496
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 496
   cttttcaatg gggtgacttt gcttc 25
<210> 497
   <211> 25
   <212> DNA
   <213> Artificial sequence
<220>
   <223> Exemplary element X variant
<220>
   <221> misc_feature
   <222> (11)..(15)
   <223> At least one residue mutated or absent
<400> 497
   cttttcattc caatgacttt gcttc 25
<210> 498
   <211> 25
   <212> DNA
   <213> Artificial sequence
<220>
   <223> Exemplary element X variant
<220>
   <221> misc_feature
   <222> (11)..(15)
   <223> At least one residue mutated or absent
<400> 498
   cttttcattc caatggggtg gcttc 25
<210> 499
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 499
   cttttcaatg actttgcttc 20
<210> 500
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 500
   cttttcaatg gggtggcttc 20
<210> 501
   <211> 20
   <212> DNA
   <213> Artificial sequence
<220>
   <223> Exemplary element X variant
<220>
   <221> misc_feature
   <222> (11)..(15)
   <223> At least one residue mutated or absent
<400> 501
   cttttcattc caatggcttc 20
<210> 502
   <211> 30
   <212> DNA
   <213> Artificial sequence
<220>
   <223> Exemplary element X variant
<220>
   <221> misc_feature
   <222> (11)..(15)
   <223> At least one residue mutated or absent
<400> 502
   cttttcattc caatggggtg actttgcttc 30
<210> 503
   <211> 19
   <212> DNA
   <213> Artificial sequence
<220>
   <223> Exemplary element X variant
<220>
   <221> misc_feature
   <222> (6)..(10)
   <223> At least one residue mutated or absent 196
<400> 503
   cttttcaatg gcttctgga 19
<210> 504
   <211> 19
   <212> DNA
   <213> Artificial sequence
<220>
   <223> Exemplary element X variant
<220>
   <221> misc_feature
   <222> (7)..(11)
   <223> At least one residue mutated or absent
<400> 504
   acttttcaat ggcttctgg 19
<210> 505
   <211> 19
   <212> DNA
   <213> Artificial sequence
<220>
   <223> Exemplary element X variant
<220>
   <221> misc_feature
   <222> (8)..(12)
   <223> At least one residue mutated or absent
<400> 505
   tacttttcaa tggcttctg 19
<210> 506
   <211> 19
   <212> DNA
   <213> Artificial sequence
<220>
   <223> Exemplary element X variant
<220>
   <221> misc_feature
   <222> (9)..(13)
   <223> At least one residue mutated or absent
<400> 506
   atacttttca atggcttct 19
<210> 507
   <211> 19
   <212> DNA
   <213> Artificial sequence
<220>
   <223> Exemplary element X variant
<220>
   <221> misc_feature
   <222> (10)..(14)
   <223> At least one residue mutated or absent
<400> 507
   catacttttc aatggcttc 19
<210> 508
   <211> 30
   <212> DNA
   <213> Artificial sequence
<220>
   <223> Exemplary element X variant
<220>
   <221> misc_feature
   <222> (6)..(15)
   <223> At least one residue mutated or absent
<400> 508
   cttttcattc caatggggtg actttgcttc 30
<210> 509
   <211> 25
   <212> DNA
   <213> Artificial sequence
<220>
   <223> Exemplary element X variant
<220>
   <221> misc_feature
   <222> (6)..(15)
   <223> At least one residue mutated or absent
<400> 509
   cttttcattc caatgacttt gcttc 25
<210> 510
   <211> 25
   <212> DNA
   <213> Artificial sequence
<220>
   <223> Exemplary element X variant
<220>
   <221> misc_feature
   <222> (6)..(15)
   <223> At least one residue mutated or absent
<400> 510
   cttttcattc caatggggtg gcttc 25
<210> 511
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 511
   cttttcattc caatggcttc tgga 24
<210> 512
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 512
   acttttcatt ccaatggctt ctgg 24
<210> 513
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 513
   tacttttcat tccaatggct tctg 24
<210> 514
   <211> 24
   <212> DNA
   <213> Artificial sequence
<220>
   <223> Exemplary element X variant
<220>
   <221> mise_feature
   <222> (9)..(18)
   <223> At least one residue mutated or absent
<400> 514
   atacttttca ttccaatggc ttct 24
<210> 515
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 515
   catacttttc attccaatgg cttc 24
<210> 516
   <211> 30
   <212> DNA
   <213> Artificial sequence
<220>
   <223> Exemplary element X variant
<220>
   <221> misc_feature
   <222> (6)..(10)
   <223> At least one residue mutated or absent
<400> 516
   cttttcattc caatggggtg actttgcttc 30
<210> 517
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 517
   cttttcattc gggtgacttt cttc 25
<210> 518
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 518
   cttttcattc caatggggtg gcttc 25
<210> 519
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 519
   cttttcattc gggtggcttc 20
<210> 520
   <211> 30
   <212> DNA
   <213> Artificial sequence
<220>
   <223> Exemplary element X variant
<220>
   <221> misc_feature
   <222> (11)..(15)
   <223> At least one residue mutated or absent
<400> 520
   cttttcattc caatggggtg aetttgcttc 30
<210> 521
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 521
   cttttcaatg gggtgacttt gcttc 25
<210> 522
   <211> 25
   <212> DNA
   <213> Artificial sequence
<220>
   <223> Exemplary element X variant
<220>
   <221> misc_feature
   <222> (11)..(15)
   <223> At least one residue mutated or absent
<400> 522
   cttttcattc caatggggtg gcttc 25
<210> 523
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 523
   cttttcaatg gggtggcttc 20
<210> 524
   <211> 30
   <212> DNA
   <213> Artificial sequence
<220>
   <223> Exemplary element X variant
<220>
   <221> misc_feature
   <222> (6)..(15)
   <223> At least one residue mutated or absent
<400> 524
   cttttcattc caatggggtg actttgcttc 30
<210> 525
   <211> 25
   <212> DNA
   <213> Artificial sequence
<220>
   <223> Exemplary element X variant
<220>
   <221> miac_feature
   <222> (6)..(15)
   <223> At least one residue mutated or absent
<400> 525
   cttttcattc caatggggtg gcttc 25
<210> 526
   <211> 30
   <212> DNA
   <213> Artificial sequence
<220>
   <223> Exemplary element X variant
<220>
   <221> misc_feature
   <222> (6)..(10)
   <223> At least one residue mutated or absent
<400> 526
   cttttcattc caatggggtg actttgcttc 30
<210> 527
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 527
   cttttcattc gggtgacttt gcttc 25
<210> 528
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 528
   cttttcattc caatgacttt gcttc 25
<210> 529
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 529
   cttttcattc actttgcttc 20
<210> 530
   <211> 30
   <212> DNA
   <213> Artificial sequence
<220>
   <223> Exemplary element X variant
<220>
   <221> misc_feature
   <222> (11)..(15)
   <223> At least one residue mutated or absent
<400> 530
   cttttcattc caatggggtg actttgcttc 30
<210> 531
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 531
   cttttcaatg gggtgacttt gcttc 25
<210> 532
   <211> 25
   <212> DNA
   <213> Artificial sequence
<220>
   <223> Exemplary element X variant
<220>
   <221> misc_feature
   <222> (11)..(15)
   <223> At least one residue mutated or absent
<400> 532
   cttttcattc caatgacttt gcttc 25
<210> 533
   <211> 20
   <212> DNA
   <213> Artificial sequence
<220>
   <223> Exemplary element X variant
<220>
   <221> misc_feature
   <222> (6)..(10)
   <223> At least one residue mutated or absent
<400> 533
   cttttcaatg actttgcttc 20
<210> 534
   <211> 30
   <212> DNA
   <213> Artificial sequence
<220>
   <223> Exemplary element X variant
<220>
   <221> misc_feature
   <222> (6)..(15)
   <223> At least one residue mutated or absent
<400> 534
   cttttcattc caatggggtg actttgcttc 30
<210> 535
   <211> 25
   <212> DNA
   <213> Artificial sequence
<220>
   <223> Exemplary element X variant
<220>
   <221> misc_feature
   <222> (6)..(15)
   <223> At least one residue mutated or absent
<400> 535
   cttttcattc caatgacttt gcttc 25
<210> 536
   <211> 30
   <212> DNA
   <213> Artificial sequence
<220>
   <223> Exemplary element X variant
<220>
   <221> misc feature
   <222> (6)..(10)
   <223> At least one residue mutated or absent
<400> 536
   cttttcattc caatggggtg actttgcttc 30
<210> 537
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 537
   cttttcattc gggtgacttt gcttc 25
<210> 538
   <211> 30
   <212> DNA
   <213> Artificial sequence
<220>
   <223> Exemplary element X variant
<220>
   <221> misc_feature
   <222> (11)..(15)
   <223> At least one residue mutated or absent
<400> 538
   cttttcattc caatggggtg actttgcttc 30
<210> 539
   <211> 25
   <212> DNA
   <213> Artificial sequence
<220>
   <223> Exemplary element X variant
<220>
   <221> misc_feature
   <222> (6)..(10)
   <223> At least one residue mutated or absent
<400> 539
   cttttcaatg gggtgacttt gcttc 25
<210> 540
   <211> 30
   <212> DNA
   <213> Artificial sequence
<220>
   <223> Exemplary element X variant
<220>
   <221> misc_feature
   <222> (6)..(15)
   <223> At least one residue mutated or absent
<400> 540
   cttttcattc caatggggtg actttgcttc 30
<210> 541
   <211> 30
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 541
   cttttcattc caatggggtg actttgcttc 30
<210> 542
   <211> 25
   <212> DNA
   <213> Artificial sequence
<220>
   <223> Exemplary element X variant
<220>
   <221> misc_feature
   <222> (6)..(15)
   <223> At least one residue mutated or absent
<400> 542
   cttttcattc gggtgacttt gcttc 25
<210> 543
   <211> 25
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 543
   cttttcattc caatggggtg gcttc 25
<210> 544
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 544
   atacttttca ttcgggtggc ttct 24
<210> 545
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 545
   tacttttcat tcgggtggct tctg 24
<210> 546
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 546
   acttttcatt cgggtggctt ctgg 24
<210> 547
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 547
   cttttcattc gggtggcttc tgga 24
<210> 548
   <211> 30
   <212> DNA
   <213> Artificial sequence
<220>
   <223> Exemplary element X variant
<220>
   <221> misc_feature
   <222> (11)..(20)
   <223> At least one residue mutated or absent
<400> 548
   cttttcattc caatggggtg actttgcttc 30
<210> 549
   <211> 25
   <212> DNA
   <213> Artificial sequence
<220>
   <223> Exemplary element X variant
<220>
   <221> misc_feature
   <222> (6)..(15)
   <223> At least one residue mutated or absent
<400> 549
   cttttcaatg gggtgacttt gcttc 25
<210> 550
   <211> 25
   <212> DNA
   <213> Artificial sequence
<220>
   <223> Exemplary element X variant
<220>
   <221> misc_feature
   <222> (11)..(20)
   <223> At least one residue mutated or absent
<400> 550
   cttttcattc caatggggtg gcttc 25
<210> 551
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 551
   atacttttca atggggtggc ttct 24
<210> 552
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 552
   tacttttcaa tggggtggct tctg 24
<210> 553
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 553
   acttttcaat ggggtggctt ctgg 24
<210> 554
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 554
   cttttcaatg gggtggcttc tgga 24
<210> 555
   <211> 30
   <212> DNA
   <213> Artificial sequence
<220>
   <223> Exemplary element X variant
<220>
   <221> misc_feature
   <222> (6)..(20)
   <223> At least one residue mutated or absent
<400> 555
   cttttcattc caatggggtg actttgcttc 30
<210> 556
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 556
   atacttttca ttccaatggg gtggcttct 29
<210> 557
   <211> 29
   <212> DNA
   <213> Artificial sequence
<220>
   <223> Exemplary element X variant
<220>
   <221> misc_feature
   <222> (8)..(22)
   <223> At least one residue mutated or absent
<400> 557
   tacttttcat tccaatgggg tggcttctg 29
<210> 558
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 558
   acttttcatt ccaatggggt ggcttctgg 29
<210> 559
   <211> 29
   <212> DNA
   <213> Artificial sequence
<220>
   <223> Exemplary element X variant
<220>
   <221> misc_feature
   <222> (6)..(20)
   <223> At least one residue mutated or absent
<400> 559
   cttttcattc caatggggtg gcttctgga 29
<210> 560
   <211> 30
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (21)..(25)
   <223> At least one residue mutated or absent
<400> 560
   cttttcattc caatggggtg actttgcttc 30
<210> 561
   <211> 25
   <212> DNA
   <213> Artificial sequence
<220> .
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 561
   cttttcattc gggtgacttt gcttc 25
<210> 562
   <211> 25
   <212> DNA
   <213> Artificial sequence
<220>
   <223> Exemplary element X variant
<220>
   <221> misc_feature
   <222> (6)..(10)
   <223> At least one residue mutated or absent
<220>
   <221> mise_feature
   <222> (16)..(20)
   <223> At least one residue mutated or absent
<400> 562
   cttttcattc caatgacttt gcttc 25
<210> 563
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 563
   atacttttca ttcactttgc ttct 24
<210> 564
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 564
   tacttttcat tcactttgct tctg 24
<210> 565
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 565
   acttttcatt cactttgctt ctgg 24
<210> 566
   <211> 24
   <212> DNA
   <213> Artificial sequence
<220>
   <223> Exemplary element X variant
<220>
   <221> misc_feature
   <222> (6)..(15)
   <223> At least one residue mutated or absent
<400> 566
   cttttcattc actttgcttc tgga 24
<210> 567
   <211> 30
   <212> DNA
   <213> Artificial sequence
<220>
   <223> Exemplary element X variant
<220>
   <221> misc_feature
   <222> (11)..(15)
   <223> At least one residue mutated or absent
<220>
   <221> misc_feature
   <222> (21)..(25)
   <223> At least one residue mutated or absent
<400> 567
   cttttcattc caatggggtg actttgcttc 30
<210> 568
   <211> 25
   <212> DNA
   <213> Artificial sequence
<220>
   <223> Exemplary element X variant
<220>
   <221> misc_feature
   <222> (6)..(10)
   <223> At least one residue mutated or absent
<220>
   <221> misc_feature
   <222> (16)..(20)
   <223> At least one residue mutated or absent
<400> 568
   cttttcaatg gggtgacttt gcttc 25
<210> 569
   <211> 25
   <212> DNA
   <213> Artificial sequence
<220>
   <223> Exemplary element X variant
<220>
   <221> misc_feature
   <222> (11)..(20)
   <223> At least one residue mutated or absent
<400> 569
   cttttcattc caatgacttt gcttc 25
<210> 570
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 570
   atacttttca atgactttgc ttct 24
<210> 571
   <211> 24
   <212> DNA
   <213> Artificial sequence
<220>
   <223> Exemplary element X variant
<220>
   <221> misc_feature
   <222> (8)..(17)
   <223> At least one residue mutated or absent
<400> 571
   tacttttcaa tgactttgct tctg 24
<210> 572
   <211> 24
   <212> DNA
   <213> Artificial sequence
<220>
   <223> Exemplary element X variant
<220>
   <221> misc_feature
   <222> (7)..(16)
   <223> At least one residue mutated or absent
<400> 572
   acttttcaat gactttgctt ctgg 24
<210> 573
   <211> 24
   <212> DNA
   <213> Artificial sequence
<220>
   <223> Exemplary element x variant
<220>
   <221> misc_feature
   <222> (6)..(15)
   <223> At least one residue mutated or absent
<400> 573
   cttttcaatg actttgcttc tgga 24
<210> 574
   <211> 30
   <212> DNA
   <213> Artificial sequence
<220>
   <223> Exemplary element X variant
<220>
   <221> misc_feature
   <222> (6)..(15)
   <223> At least one residue mutated or absent
<220>
   <221> misc_feature
   <222> (21)..(25)
   <223> At least one residue mutated or absent
<400> 574
   cttttcattc caatggggtg actttgcttc 30
<210> 575
   <211> 29
   <212> DNA
   <213> Artificial sequence
<220>
   <223> Exemplary element X variant
<220>
   <221> misc feature
   <222> (9)..(23)
   <223> At least one residue mutated or absent
<400> 575
   atacttttca ttccaatgac tttgcttct 29
<210> 576
   <211> 29
   <212> DNA
   <213> Artificial sequence
<220>
   <223> Exemplary element X variant
<220>
   <221> misc_feature
   <222> (8)..(22)
   <223> At least one residue mutated or absent
<400> 576
   tacttttcat tccaatgact ttgcttctg 29
<210> 577
   <211> 29
   <212> DNA
   <213> Artificial sequence
<220>
   <223> Exemplary element x variant
<220>
   <221> misc feature
   <222> (7)..(21)
   <223> At least one residue mutated or absent
<400> 577
   acttttcatt ccaatgactt tgcttctgg 29
<210> 578
   <211> 29
   <212> DNA
   <213> Artificial sequence
<220>
   <223> Exemplary element X variant
<220>
   <221> misc_feature
   <222> (6)..(20)
   <223> At least one residue mutated or absent
<400> 578
   cttttcattc caatgacttt gcttctgga 29
<210> 579
   <211> 30
   <212> DNA
   <213> Artificial sequence
<220>
   <223> Exemplary element X variant
<220>
   <221> mise_feature
   <222> (6)..(10)
   <223> At least one residue mutated or absent
<220>
   <221> misc_feature
   <222> (16)..(25)
   <223> At least one residue mutated or absent
<400> 579
   cttttcattc caatggggtg actttgcttc 30
<210> 580
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 580
   atacttttca ttcgggtgac tttgcttct 29
<210> 581
   <211> 29
   <212> DNA
   <213> Artificial sequence
<220>
   <223> Exemplary element X variant
<220>
   <221> misc_feature
   <222> (8)..(22)
   <223> At least one residue mutated or absent
<400> 581
   tacttttcat tcgggtgact ttgcttctg 29
<210> 582
   <211> 29
   <212> DNA
   <213> Artificial sequence
<220>
   <223> Exemplary element X variant
<220>
   <221> misc_feature
   <222> (7)..(21)
   <223> At least one residue mutated or absent
<400> 582
   acttttcatt cgggtgactt tgcttctgg 29
<210> 583
   <211> 29
   <212> DNA
   <213> Artificial sequence
<220>
   <223> Exemplary element X variant
<220>
   <221> misc feature
   <222> (6)..(20)
   <223> At least one residue mutated or absent
<400> 583
   cttttcattc gggtgacttt gcttctgga 29
<210> 584
   <211> 30
   <212> DNA
   <213> Artificial sequence
<220>
   <223> Exemplary element X variant
<220>
   <221> misc_feature
   <222> (11)..(25)
   <223> At least one residue mutated or absent
<400> 584
   cttttcattc caatggggtg actttgcttc 30
<210> 585
   <211> 29
   <212> DNA
   <213> Artificial sequence
<220>
   <223> Exemplary element X variant
<220>
   <221> misc_feature
   <222> (9)..(23)
   <223> At least one residue mutated or absent
<400> 585
   atacttttca atggggtgac tttgcttct 29
<210> 586
   <211> 29
   <212> DNA
   <213> Artificial sequence
<220>
   <223> Exemplary element x variant
<220>
   <221> misc_feature
   <222> (8)..(22)
   <223> At least one residue mutated or absent
<400> 586
   tacttttcaa tggggtgact ttgcttctg 29
<210> 587
   <211> 29
   <212> DNA
   <213> Artificial sequence
<220>
   <223> Exemplary element X variant
<220>
   <221> mise_feature
   <222> (7)..(21)
   <223> At least one residue mutated or absent
<400> 587
   acttttcaat ggggtgactt tgcttctgg 29
<210> 588
   <211> 29
   <212> DNA
   <213> Artificial sequence
<220>
   <223> Exemplary element X variant
<220>
   <221> misc_feature
   <222> (6)..(20)
   <223> At least one residue mutated or absent
<400> 588
   cttttcaatg gggtgacttt gcttctgga 29
<210> 589
   <211> 34
   <212> DNA
   <213> Artificial sequence
<220>
   <223> Exemplary element X variant
<220>
   <221> misc_feature
   <222> (9)..(28)
   <223> At least one residue mutated or absent
<400> 589
   atacttttca ttccaatggg gtgactttgc ttct 34
<210> 590
   <211> 34
   <212> DNA
   <213> Artificial sequence
<220>
   <223> Exemplary element X variant
<220>
   <221> misc_feature
   <222> (8)..(27)
   <223> At least one residue mutated or absent
<400> 590
   tacttttcat tccaatgggg tgactttgct tctg 34
<210> 591
   <211> 34
   <212> DNA
   <213> Artificial sequence
<220>
   <223> Exemplary element X variant
<220>
   <221> misc_feature
   <222> (7)..(26)
   <223> At least one residue mutated or absent
<400> 591
   acttttcatt ccaatggggt gactttgctt ctgg 34
<210> 592
   <211> 34
   <212> DNA
   <213> Artificial sequence
<220>
   <223> Exemplary element X variant
<220>
   <221> misc_feature
   <222> (6)..(25)
   <223> At least one residue mutated or absent
<400> 592
   cttttcattc caatggggtg actttgcttc tgga 34
<210> 593
   <211> 30
   <212> DNA
   <213> Artificial sequence
<220>
   <223> Exemplary element X variant
<220>
   <221> misc_feature
   <222> (16)..(25)
   <223> At least one residue mutated or absent
<400> 593
   cttttcattc caatggggtg actttgcttc 30
<210> 594
   <211> 25
   <212> DNA
   <213> Artificial sequence
<220>
   <223> Exemplary element X variant
<220>
   <221> misc_feature
   <222> (11)..(20)
   <223> At least one residue mutated or absent
<400> 594
   cttttcaatg gggtgacttt gcttc 25
<210> 595
   <211> 25
   <212> DNA
   <213> Artificial sequence
<220>
   <223> Exemplary element X variant
<220>
   <221> misc_feature
   <222> (11)..(20)
   <223> At least one residue mutated or absent
<400> 595
   cttttcattc gggtgacttt gcttc 25
<210> 596
   <211> 24
   <212> DNA
   <213> Artificial sequence
<220>
   <223> Exemplary element X variant
<220>
   <221> misc feature
   <222> (6)..(15)
   <223> At least one residue mutated or absent
<400> 596
   cttttgggtg actttgcttc tgga 24
<210> 597
   <211> 24
   <212> DNA
   <213> Artificial sequence
<220>
   <223> Exemplary element X variant
<220>
   <221> misc_feature
   <222> (7)-.(16)
   <223> At least one residue mutated or absent
<400> 597
   acttttgggt gactttgctt ctgg 24
<210> 598
   <211> 24
   <212> DNA
   <213> Artificial sequence
<220>
   <223> Exemplary element x variant
<220>
   <221> misc_feature
   <222> (8)..(17)
   <223> At least one residue mutated or absent
<400> 598
   tacttttggg tgactttgct tctg 24
<210> 599
   <211> 24
   <212> DNA
   <213> Artificial sequence
<220>
   <223> Exemplary element X variant
<220>
   <221> misc_feature
   <222> (9)..(18)
   <223> At least one residue mutated or absent
<400> 599
   atacttttgg gtgactttgc ttct 24
<210> 600
   <211> 24
   <212> DNA
   <213> Artificial sequence
<220>
   <223> Exemplary element X variant
<220>
   <221> misc_feature
   <222> (10)..(19)
   <223> At least one residue mutated or absent
<400> 600
   catacttttg ggtgactttg cttc 24
<210> 601
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 601
   gtacttcata gggtgacttt gcttctgga 29
<210> 602
   <211> 24
   <212> DNA
   <213> Artificial sequence
<220>
   <223> Exemplary element X variant
<220>
   <221> misc_feature
   <222> (16)..(20)
   <223> At least one residue mutated or absent
<400> 602
   gtacttcata actttgcttc tgga 24
<210> 603
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 603
   gtacttcata gcttctgga 19
<210> 604
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 604
   gtacttcata cttttgggtg actttgcttc tgga 34
<210> 605
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 605
   gtacttcata cttttacttt gcttctgga 29
<210> 606
   <211> 24
   <212> DNA
   <213> Artificial sequence
<220>
   <223> Exemplary element X variant
<220>
   <221> misc_feature
   <222> (16)..(20)
   <223> At least one residue mutated or absent
<400> 606
   gtacttcata cttttgcttc tgga 24
<210> 607
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 607
   gtacttcata cttttcattc actttgcttc tgga 34
<210> 608
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 608
   gtacttcata cttttcattc gcttctgga 29
<210> 609
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 609
   gtacttcata cttttcattc caatggcttc tgga 34
<210> 610
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 610
   gtactctttt caatggggtg actttgcttc tgga 34
<210> 611
   <211> 29
   <212> DNA
   <213> Artificial sequence
<220>
   <223> Exemplary element X variant
<220>
   <221> mise_feature
   <222> (21)..(25)
   <223> At least one residue mutated or absent
<400> 611
   gtactctttt gggtgacttt gcttctgga 29
<210> 612
   <211> 24
   <212> DNA
   <213> Artificial sequence
<220>
   <223> Exemplary element X variant
<220>
   <221> misc_feature
   <222> (16)..(20)
   <223> At least one residue mutated or absent
<400> 612
   gtactctttt actttgcttc tgga 24
<210> 613
   <211> 19
   <212> DNA
   <213> Artificial sequence
<220>
   <223> Exemplary element X variant
<220>
   <221> misc_feature
   <222> (11)..(15)
   <223> At least one residue mutated or absent
<400> 613
   gtactctttt gcttctgga 19
<210> 614
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 614
   gtactctttt cattcgggtg actttgcttc tgga 34
<210> 615
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 615
   gtactctttt cattcacttt gcttctgga 29
<210> 616
   <211> 24
   <212> DNA
   <213> Artificial sequence
<220>
   <223> Exemplary element X variant
<220>
   <221> misc_feature
   <222> (16)..(20)
   <223> At least one residue mutated or absent
<400> 616
   gtactctttt cattcgcttc tgga 24
<210> 617
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 617
   gtactctttt cattccaatg actttgcttc tgga 34
<210> 618
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 618
   gtactctttt cattccaatg gcttctgga 29
<210> 619
   <211> 34
   <212> DNA
   <213> Artificial sequence
<220>
   <223> Exemplary element X variant
<220>
   <221> misc_feature
   <222> (26)..(30)
   <223> At least one residue mutated or absent
<900> 619
   gtactctttt cattccaatg gggtggcttc tgga 34
<210> 620
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 620
   gtacttcata cattcgggtg actttgcttc tgga 34
<210> 621
   <211> 29
   <212> DNA
   <213> Artificial sequence
<220>
   <223> Exemplary element x variant
<220>
   <221> misc_feature
   <222> (21)..(25)
   <223> At least one residue mutated or absent
<400> 621
   gtacttcata cattcacttt gcttctgga 29
<210> 622
   <211> 24
   <212> DNA
   <213> Artificial sequence
<220>
   <223> Exemplary element X variant
<220>
   <221> misc_feature
   <222> (16)..(20)
   <223> At least one residue mutated or absent
<400> 622
   gtacttcata cattcgcttc tgga 24
<210> 623
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 623
   gtacttcata cattccaatg actttgcttc tgga 34
<210> 624
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 624
   gtacttcata cattccaatg gcttctgga 29
<210> 625
   <211> 34
   <212> DNA
   <213> Artificial sequence
<220>
   <223> Exemplary element x variant
<220>
   <221> misc_feature
   <222> (26)..(30)
   <223> At least one residue mutated or absent
<400> 625
   gtacttcata cattccaatg gggtggcttc tgga 34
<210> 626
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 626
   gtacttcata cttttcaatg actttgcttc tgga 34
<210> 627
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 627
   gtacttcata cttttcaatg gcttctgga 29
<210> 628
   <211> 34
   <212> DNA
   <213> Artificial sequence
<220>
   <223> Exemplary element X variant
<220>
   <221> misc_feature
   <222> (26)..(30)
   <223> At least one residue mutated or absent
<400> 628
   gtacttcata cttttcattc gggtggcttc tgga 34
<210> 629
   <211> 24
   <212> DNA
   <213> Artificial sequence
<220>
   <223> Exemplary element X variant
<220>
   <221> misc_feature
   <222> (16)..(20)
   <223> At least one residue mutated or absent
<400> 629
   gtactctttt caatggcttc tgga 24
<210> 630
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 630
   gtactctttt caatggggtg gcttctgga 29
<210> 631
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 631
   gtactctttt cattcacttt gcttctgga 29
<210> 632
   <211> 29
   <212> DNA
   <213> Artificial sequence
<220>
   <223> Exemplary element X variant
<220>
   <221> misc_feature
   <222> (21)..(25)
   <223> At least one residue mutated or absent
<400> 632
   gtacttcata cattcgggtg gcttctgga 29
<210> 633
   <211> 44
   <212> DNA
   <213> Artificial sequence
<220>
   <223> PPE-1-derived regulatory element scheme
<220>
   <221> misc_feature
   <222> (36)..(40)
   <223> At least one residue mutated or absent
<220>
   <221> misc_feature
   <222> (36)..(40)
   <223> Mutated fragment (or variation of) can apear in multiple copies
<400> 633
   gtacttcata cttttcattc caatggggtg actttgcttc tgga 44
<210> 634
   <211> 35
   <212> DNA
   <213> Artificial sequence
<220>
   <223> PPE-1-derived regulatory element scheme
<400> 634
   gtacttcata cttttcattc caatggggtg acttt 35
<210> 635
   <211> 25
   <212> DNA
   <213> Artificial sequence
<220>
   <223> PPE-1-derived regulatory element scheme
<400> 635
   cattccaatg gggtgacttt gcttc 25
<210> 636
   <211> 29
   <212> DNA
   <213> Artificial sequence
<220>
   <223> PPE-1-derived regulatory element scheme
<400> 636
   cattccaatg gggtgacttt gcttctgga 29
<210> 637
   <211> 20
   <212> DNA
   <213> Artificial sequence
<220>
   <223> PPE-1-derived regulatory element scheme
<400> 637
   cattccaatg gggtgacttt 20
<210> 638
   <211> 14
   <212> DNA
   <213> Artificial sequence
<220>
   <223> PPE-1-derived regulatory element scheme
<400> 638
   actttgcttc tgga 14
<210> 639
   <211> 10
   <212> DNA
   <213> Artificial sequence
<220>
   <223> PPE-1-derived regulatory element scheme
<400> 639
   actttgcttc 10
<210> 640
   <211> 9
   <212> DNA
   <213> Artificial sequence
<220>
   <223> PPE-1-derived regulatory element scheme
<220>
   <221> misc_feature
   <222> (6)..(9)
   <223> One or more residues can be mutated or absent
<400> 640
   gcttctgga 9
<210> 641
   <211> 14
   <212> DNA
   <213> Artificial sequence
<220>
   <223> PPE-1-derived regulatory element scheme
<220>
   <221> misc_feature
   <222> (6)..(10)
   <223> At least one residue mutated or absent
<220>
   <221> misc_feature
   <222> (11)..(14)
   <223> One or more residues can be mutated or absent
<400> 641
   actttgcttc tgga 14
<210> 642
   <211> 10
   <212> DNA
   <213> Artificial sequence
<220>
   <223> PPE-1-derived regulatory element scheme
<220>
   <221> misc_feature
   <222> (6)..(10)
   <223> At least one residue mutated or absent
<400> 642
   actttgcttc 10
<210> 643
   <211> 9
   <212> DNA
   <213> Artificial sequence
<220>
   <223> PPE-1-derived regulatory element scheme
<220>
   <221> misc_feature
   <222> (1)..(5)
   <223> At least one residue mutated or absent
<220>
   <221> misc_feature
   <222> (6)..(9)
   <223> One or more residues can be mutated or absent
<400> 643
   gcttctgga 9
<210> 644
   <211> 15
   <212> DNA
   <213> Artificial sequence
<220>
   <223> PPE-1-derived regulatory element scheme
<220>
   <221> misc_feature
   <222> (11)..(15)
   <223> At least one residue mutated or absent
<220>
   <221> misc_feature
   <222> (11)..(15)
   <223> Mutated fragment (or variation of) can apear in multiple copies
<400> 644
   cattccaatg gcttc 15